# EUROPEAN PATENT SPECIFICATION

(11) **EP 2 785 382 B1**
(45) Date of publication and mention of the grant of the patent: **02.01.2019**
(21) Application number: 12854231.3
(22) Date of filing: 29.11.2012
(51) Int. Cl.: A61K 39/395, A61K 39/00, C07K 16/24, C07K 16/28, C07K 16/30, A61K 47/68

(54) **MULTIVALENT ANTIBODY COMPLEXES TARGETING IGF-1R SHOW POTENT TOXICITY AGAINST SOLID TUMORS**
MULTIVALENTE ANTIKÖRPERKOMPLEXE GEGEN IGF-1R MIT STARKER TOXIZITÄT GEGEN SOLIDE TUMOREN
COMPLEXES D'ANTICORPS MULTIVALENTS CIBLANT L'IGF-1R PRÉSENTANT UNE PUISSANTE TOXICITÉ CONTRE LES TUMEURS SOLIDES

(30) Priority: 02.12.2011 US 201161566273 P; 27.03.2012 US 201261616051 P; 30.05.2012 US 201213483761
(43) Date of publication of application: 08.10.2014
(73) Proprietor: IBC Pharmaceuticals, Inc., Morris Plains, NJ 07950 (US)
(72) Inventor: CHANG, Chien-Hsing, Morris Plains, New Jersey 07950 (US); GOLDENBERG, David M., Morris Plains, New Jersey 07950 (US)
(74) Representative: Bohmann, Armin K.
(86) International application number: PCT/US2012/067005
(87) International publication number: WO 2013/082254

(56) References cited:
- WO-A1-2011/090492
- US-A1- 2009 060 862
- US-A1- 2009 175 819
- US-A1- 2010 226 884
- US-A1- 2011 123 436
- US-A1- 2011 123 436
- A. NAING ET AL: "Phase I Trial of Cixutumumab Combined with Temsirolimus in Patients with Advanced Cancer", CLINICAL CANCER RESEARCH, vol. 17, no. 18, 15 September 2011 (2011-09-15), pages 6052-6060, XP055061603, ISSN: 1078-0432, DOI: 10.1158/1078-0432.CCR-10-2979
- E. ANDERS KOLB ET AL: "R1507, a fully human monoclonal antibody targeting IGF-1R, is effective alone and in combination with rapamycin in inhibiting growth of osteosarcoma xenografts", PEDIATRIC BLOOD & CANCER, 1 January 2010 (2010-01-01), pages n/a-n/a, XP055196541, ISSN: 1545-5009, DOI: 10.1002/pbc.22479
- R. T. KURMASHEVA ET AL: "The Insulin-like Growth Factor-1 Receptor-Targeting Antibody, CP-751,871, Suppresses Tumor-Derived VEGF and Synergizes with Rapamycin in Models of Childhood Sarcoma", CANCER RESEARCH, vol. 69, no. 19, 29 September 2009 (2009-09-29), pages 7662-7671, XP055185259, ISSN: 0008-5472, DOI: 10.1158/0008-5472.CAN-09-1693
- SRIRAM SATHYANARAYANAN ET AL: "Combination treatment with the anti-IGF1R antibody MK-0646 and the mTOR inhibitor deforolimus leads to more effective PI3K pathway targeting and anti-tumor activity", AMERICAN ASSOCIATION FOR CANCER RESEARCH. PROCEEDINGS OF THE ANNUAL MEETING; 100TH ANNUAL MEETING OF THE AMERICAN-ASSOCIATION-FOR-CANCER-RESEARCH; DENVER, CA, USA; APRIL 18 -22, 2009, AMERICAN ASSOCIATION FOR CANCER RESEARCH, US, vol. 50, 1 April 2009 (2009-04-01), page 679, XP002687423, ISSN: 0197-016X
- X WAN ET AL: "Rapamycin induces feedback activation of Akt signaling through an IGF-1R-dependent mechanism", ONCOGENE, vol. 26, no. 13, 25 September 2006 (2006-09-25), pages 1932-1940, XP055196548, ISSN: 0950-9232, DOI: 10.1038/sj.onc.1209990
- COULTER DON W; BLATT JULIE; D'ERCOLE A JOSEPH; MOATS-STAATS BILLIE M: "IGF-I receptor inhibition combined with rapamycin or temsirolimus inhibits neuroblastoma cell growth", ANTICANCER RESEARCH - INTERNATIONAL JOURNAL OF CANCER RESEARCH AND TREATMENT, vol. 28, no. 3A, 1 May 2008 (2008-05-01), pages 1509-1516, XP055196566, GR ISSN: 0250-7005
- THAKUR M L ET AL: "IMPROVED ANTIBODY TARGETING WITH INTERFERON-ALPHA-2B CONJUGATE", JOURNAL OF IMMUNOTHERAPY, LIPPINCOTT WILLIAMS & WILKINS, HAGERSTOWN, MD, US, vol. 20, no. 3, 1 January 1997 (1997-01-01), pages 194-201, XP002945738, ISSN: 1524-9557
- QUEK ET AL.: 'Combination mTOR and IGF-1 inhibition: Phase I trial of everolimus and figitumumab in patients with advanced sarcomas and other solid tumors.' CLIN CANCER RES. vol. 17, no. 4, 22 December 2010, pages 871 - 879, XP055070644

### SEQUENCE LISTING

<110> IBC PHARMACEUTICALS, INC.
<120> MULTIVALENT ANTIBODY COMPLEXES TARGETING IGF-1R SHOW POTENT TOXICITY AGAINST SOLID TUMORS
<130> IBC134WO1
<140>
   <141>
<150> 13/483,761
   <151> 2012-05-30
<150> 61/616,051
   <151> 2012-03-27
<150> 61/566,273
   <151> 2011-12-02
<160> 123
<170> PatentIn version 3.5
<210> 1
   <211> 44
   <212> PRT
   <213> Artificial Sequence
<220>
   <223> Description of Artificial Sequence: Synthetic polypeptide
<400> 1
   Ser His Ile Gln Ile Pro Pro Gly Leu Thr Glu Leu Leu Gln Gly Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 2
   <211> 45
   <212> PRT
   <213> Artificial Sequence
<220>
   <223> Description of Artificial Sequence: Synthetic polypeptide
<400> 2
   Cys Gly His Ile Gln Ile Pro Pro Gly Leu Thr Glu Leu Leu Gln Gly
   1               5                   10                  15
   Tyr Thr Val Glu Val Leu Arg Gln Gln Pro Pro Asp Leu Val Glu Phe
               20                  25                  30
   Ala Val Glu Tyr Phe Thr Arg Leu Arg Glu Ala Arg Ala
           35                  40                  45
<210> 3
   <211> 17
   <212> PRT
   <213> Artificial Sequence
<220>
   <223> Description of Artificial Sequence: Synthetic peptide
<400> 3
   Gln Ile Glu Tyr Leu Ala Lys Gln Ile Val Asp Asn Ala Ile Gln Gln
   1               5                   10                  15
   Ala
<210> 4
   <211> 21
   <212> PRT
   <213> Artificial Sequence
<220>
   <223> Description of Artificial Sequence: Synthetic peptide
<400> 4
   Cys Gly Gln Ile Glu Tyr Leu Ala Lys Gln Ile Val Asp Asn Ala Ile
   1               5                   10                  15
   Gln Gln Ala Gly Cys
               20
<210> 5
   <211> 50
   <212> PRT
   <213> Artificial sequence
<220>
   <223> Description of Artificial Sequence: Synthetic polypeptide
<400> 5
   Ser Leu Arg Glu Cys Glu Leu Tyr Val Gln Lys His Asn Ile Gln Ala
   1               5                   10                  15
   Leu Leu Lys Asp Ser Ile Val Gln Leu Cys Thr Ala Arg Pro Glu Arg
               20                  25                  30
   Pro Met Ala Phe Leu Arg Glu Tyr Phe Glu Arg Leu Glu Lys Glu Glu
           35                  40                  45
   Ala Lys
       50
<210> 6
   <211> 55
   <212> PRT
   <213> Artificial Sequence
<220>
   <223> Description of Artificial Sequence: Synthetic polypeptide
<400> 6
   Met Ser Cys Gly Gly Ser Leu Arg Glu Cys Glu Leu Tyr Val Gln Lys
   1               5                   10                  15
   His Asn Ile Gln Ala Leu Leu Lys Asp Ser Ile Val Gln Leu Cys Thr
               20                  25                  30
   Ala Arg Pro Glu Arg Pro Met Ala Phe Leu Arg Glu Tyr Phe Glu Arg
           35                  40                  45
   Leu Glu Lys Glu Glu Ala Lys
       50                  55
<210> 7
   <211> 23
   <212> PRT
   <213> Artificial Sequence
<220>
   <223> Description of Artificial Sequence: Synthetic peptide
<400> 7
   Cys Gly Phe Glu Glu Leu Ala Trp Lys Ile Ala Lys Met Ile Trp Ser
   1               5                   10                  15
   Asp Val Phe Gln Gln Gly Cys
               20
<210> 8
   <211> 51
   <212> PRT
   <213> Homo sapiens
<400> 8
   Ser Leu Arg Glu Cys Glu Leu Tyr Val Gln Lys His Asn Ile Gln Ala
   1               5                   10                  15
   Leu Leu Lys Asp Val Ser Ile Val Gln Leu Cys Thr Ala Arg Pro Glu
               20                  25                  30
   Arg Pro Met Ala Phe Leu Arg Glu Tyr Phe Glu Lys Leu Glu Lys Glu
           35                  40                  45
   Glu Ala Lys
       50
<210> 9
   <211> 54
   <212> PRT
   <213> Homo sapiens
<400> 9
   Ser Leu Lys Gly Cys Glu Leu Tyr Val Gln Leu His Gly Ile Gln Gln
   1               5                   10                  15
   Val Leu Lys Asp Cys Ile Val His Leu Cys Ile Ser Lys Pro Glu Arg
               20                  25                  30
   Pro Met Lys Phe Leu Arg Glu His Phe Glu Lys Leu Glu Lys Glu Glu
           35                  40                  45
   Asn Arg Gln Ile Leu Ala
       50
<210> 10
   <211> 44
   <212> PRT
   <213> Homo sapiens
<400> 10
   Ser His Ile Gln Ile Pro Pro Gly Leu Thr Glu Leu Leu Gln Gly Tyr
   1               5                   10                  15
   Thr Val Glu Val Gly Gln Gln Pro Pro Asp Leu Val Asp Phe Ala Val
               20                  25                  30
   Glu Tyr Phe Thr Arg Leu Arg Glu Ala Arg Arg Gln
           35                  40
<210> 11
   <211> 44
   <212> PRT
   <213> Homo sapiens
<400> 11
   Ser Ile Glu Ile Pro Ala Gly Leu Thr Glu Leu Leu Gln Gly Phe Thr
   1               5                   10                  15
   Val Glu Val Leu Arg His Gln Pro Ala Asp Leu Leu Glu Phe Ala Leu
               20                  25                  30
   Gln His Phe Thr Arg Leu Gln Gln Glu Asn Glu Arg
   35 40
<210> 12
   <211> 44
   <212> PRT
   <213> Artificial Sequence
<220>
   <223> Description of Artificial Sequence: Synthetic polypeptide
<400> 12
   Thr His Ile Gln Ile Pro Pro Gly Leu Thr Glu Leu Leu Gln Gly Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 13
   <211> 44
   <212> PRT
   <213> Artificial Sequence
<220>
   <223> Description of Artificial Sequence: Synthetic polypeptide
<400> 13
   Ser Lys Ile Gln Ile Pro Pro Gly Leu Thr Glu Leu Leu Gln Gly Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 14
   <211> 44
   <212> PRT
   <213> Artificial Sequence
<220>
   <223> Description of Artificial Sequence: Synthetic polypeptide
<400> 14
   Ser Arg Ile Gln Ile Pro Pro Gly Leu Thr Glu Leu Leu Gln Gly Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 15
   <211> 44
   <212> PRT
   <213> Artificial Sequence
<220>
   <223> Description of Artificial Sequence: Synthetic polypeptide
<400> 15
   Ser His Ile Asn Ile Pro Pro Gly Leu Thr Glu Leu Leu Gln Gly Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 16
   <211> 44
   <212> PRT
   <213> Artificial Sequence
<220>
   <223> Description of Artificial Sequence: Synthetic polypeptide
<400> 16
   Ser His Ile Gln Ile Pro Pro Ala Leu Thr Glu Leu Leu Gln Gly Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 17
   <211> 44
   <212> PRT
   <213> Artificial Sequence
<220>
   <223> Description of Artificial Sequence: Synthetic polypeptide
<400> 17
   Ser His Ile Gln Ile Pro Pro Gly Leu Ser Glu Leu Leu Gln Gly Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 18
   <211> 44
   <212> PRT
   <213> Artificial Sequence
<220>
   <223> Description of Artificial Sequence: Synthetic polypeptide
<400> 18
   Ser His Ile Gln Ile Pro Pro Gly Leu Thr Asp Leu Leu Gln Gly Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 19
   <211> 44
   <212> PRT
   <213> Artificial Sequence
<220>
   <223> Description of Artificial Sequence: Synthetic polypeptide
<400> 19
   Ser His Ile Gln Ile Pro Pro Gly Leu Thr Glu Leu Leu Asn Gly Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 20
   <211> 44
   <212> PRT
   <213> Artificial Sequence
<220>
   <223> Description of Artificial Sequence: Synthetic polypeptide
<400> 20
   Ser His Ile Gln Ile Pro Pro Gly Leu Thr Glu Leu Leu Gln Ala Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 21
   <211> 44
   <212> PRT
   <213> Artificial sequence
<220>
   <223> Description of Artificial Sequence: Synthetic polypeptide
<400> 21
   Ser His Ile Gln Ile Pro Pro Gly Leu Thr Glu Leu Leu Gln Gly Tyr
   1               5                   10                  15
   Ser Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 22
   <211> 44
   <212> PRT
   <213> Artificial Sequence
<220>
   <223> Description of Artificial Sequence: Synthetic polypeptide
<400> 22
   Ser His Ile Gln Ile Pro Pro Gly Leu Thr Glu Leu Leu Gln Gly Tyr
   1               5                   10                  15
   Thr Val Asp Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 23
   <211> 44
   <212> PRT
   <213> Artificial Sequence
<220>
   <223> Description of Artificial Sequence: Synthetic polypeptide
<400> 23
   Ser His Ile Gln Ile Pro Pro Gly Leu Thr Glu Leu Leu Gln Gly Tyr
   1               5                   10                  15
   Thr Val Glu Val Leu Lys Gln Gln Pro Pro Asp Leu Val Glu Phe Ala
               20                  25                  30
   Val Glu Tyr Phe Thr Arg Leu Arg Glu Ala Arg Ala
           35                  40
<210> 24
   <211> 44
   <212> PRT
   <213> Artificial Sequence
<220>
   <223> Description of Artificial Sequence: Synthetic polypeptide
<400> 24
   Ser His Ile Gln Ile Pro Pro Gly Leu Thr Glu Leu Leu Gln Gly Tyr
   1               5                   10                  15
   Thr Val Glu Val Leu Arg Asn Gln Pro Pro Asp Leu Val Glu Phe Ala
               20                  25                  30
   Val Glu Tyr Phe Thr Arg Leu Arg Glu Ala Arg Ala
           35                  40
<210> 25
   <211> 44
   <212> PRT
   <213> Artificial Sequence
<220>
   <223> Description of Artificial Sequence: Synthetic polypeptide
<400> 25
   Ser His Ile Gln Ile Pro Pro Gly Leu Thr Glu Leu Leu Gln Gly Tyr
   1               5                   10                  15
   Thr Val Glu Val Leu Arg Gln Asn Pro Pro Asp Leu Val Glu Phe Ala
               20                  25                  30
   Val Glu Tyr Phe Thr Arg Leu Arg Glu Ala Arg Ala
           35                  40
<210> 26
   <211> 44
   <212> PRT
   <213> Artificial sequence
<220>
   <223> Description of Artificial Sequence: Synthetic polypeptide
<400> 26
   Ser His Ile Gln Ile Pro Pro Gly Leu Thr Glu Leu Leu Gln Gly Tyr
   1               5                   10                  15
   Thr Val Glu Val Leu Arg Gln Gln Pro Pro Glu Leu Val Glu Phe Ala
               20                  25                  30
   Val Glu Tyr Phe Thr Arg Leu Arg Glu Ala Arg Ala
           35                  40
<210> 27
   <211> 44
   <212> PRT
   <213> Artificial Sequence
<220>
   <223> Description of Artificial Sequence: Synthetic polypeptide
<400> 27
   Ser His Ile Gln Ile Pro Pro Gly Leu Thr Glu Leu Leu Gln Gly Tyr
   1               5                   10                  15
   Thr Val Glu Val Leu Arg Gln Gln Pro Pro Asp Leu Val Asp Phe Ala
               20                  25                  30
   Val Glu Tyr Phe Thr Arg Leu Arg Glu Ala Arg Ala
           35                  40
<210> 28
   <211> 44
   <212> PRT
   <213> Artificial Sequence
<220>
   <223> Description of Artificial Sequence: Synthetic polypeptide
<400> 28
   Ser His Ile Gln Ile Pro Pro Gly Leu Thr Glu Leu Leu Gln Gly Tyr
   1               5                   10                  15
   Thr Val Glu Val Leu Arg Gln Gln Pro Pro Asp Leu Val Glu Phe Leu
               20                  25                  30
   Val Glu Tyr Phe Thr Arg Leu Arg Glu Ala Arg Ala
           35                  40
<210> 29
   <211> 44
   <212> PRT
   <213> Artificial Sequence
<220>
   <223> Description of Artificial Sequence: Synthetic polypeptide
<400> 29
   Ser His Ile Gln Ile Pro Pro Gly Leu Thr Glu Leu Leu Gln Gly Tyr
   1               5                   10                  15
   Thr Val Glu Val Leu Arg Gln Gln Pro Pro Asp Leu Val Glu Phe Ile
               20                  25                  30
   Val Glu Tyr Phe Thr Arg Leu Arg Glu Ala Arg Ala
           35                  40
<210> 30
   <211> 44
   <212> PRT
   <213> Artificial Sequence
<220>
   <223> Description of Artificial Sequence: Synthetic polypeptide
<400> 30
   Ser His Ile Gln Ile Pro Pro Gly Leu Thr Glu Leu Leu Gln Gly Tyr
   1               5                   10                  15
   Thr Val Glu Val Leu Arg Gln Gln Pro Pro Asp Leu Val Glu Phe Val
               20                  25                  30
   Val Glu Tyr Phe Thr Arg Leu Arg Glu Ala Arg Ala
           35                  40
<210> 31
   <211> 44
   <212> PRT
   <213> Artificial Sequence
<220>
   <223> Description of Artificial Sequence: Synthetic polypeptide
<400> 31
   Ser His Ile Gln Ile Pro Pro Gly Leu Thr Glu Leu Leu Gln Gly Tyr
   1               5                   10                  15
   Thr Val Glu Val Leu Arg Gln Gln Pro Pro Asp Leu Val Glu Phe Ala
               20                  25                  30
   Val Asp Tyr Phe Thr Arg Leu Arg Glu Ala Arg Ala
           35                  40
<210> 32
   <211> 17
   <212> PRT
   <213> Artificial Sequence
<220>
   <223> Description of Artificial Sequence: Synthetic peptide
<400> 32
   Asn Ile Glu Tyr Leu Ala Lys Gln Ile Val Asp Asn Ala Ile Gln Gln
   1               5                   10                  15
   Ala
<210> 33
   <211> 17
   <212> PRT
   <213> Artificial Sequence
<220>
   <223> Description of Artificial Sequence: Synthetic peptide
<400> 33
   Gln Leu Glu Tyr Leu Ala Lys Gln Ile Val Asp Asn Ala Ile Gln Gln
   1               5                   10                  15
   Ala
<210> 34
   <211> 17
   <212> PRT
   <213> Artificial Sequence
<220>
   <223> Description of Artificial Sequence: synthetic peptide
<400> 34
   Gln Val Glu Tyr Leu Ala Lys Gln Ile Val Asp Asn Ala Ile Gln Gln
   1 5 10 15
   Ala
<210> 35
   <211> 17
   <212> PRT
   <213> Artificial Sequence
<220>
   <223> Description of Artificial Sequence: Synthetic peptide
<400> 35
   Gln Ile Asp Tyr Leu Ala Lys Gln Ile Val Asp Asn Ala Ile Gln Gln
   1 5 10 15
   Ala
<210> 36
   <211> 17
   <212> PRT
   <213> Artificial Sequence
<220>
   <223> Description of Artificial Sequence: Synthetic peptide
<400> 36
   Gln Ile Glu Phe Leu Ala Lys Gln Ile Val Asp Asn Ala Ile Gln Gln
   1 5 10 15
   Ala
<210> 37
   <211> 17
   <212> PRT
   <213> Artificial Sequence
<220>
   <223> Description of Artificial Sequence: Synthetic peptide
<400> 37
   Gln Ile Glu Thr Leu Ala Lys Gln Ile Val Asp Asn Ala Ile Gln Gln
   1 5 10 15
   Ala
<210> 38
   <211> 17
   <212> PRT
   <213> Artificial Sequence
<220>
   <223> Description of Artificial Sequence: Synthetic peptide
<400> 38
   Gln Ile Glu Ser Leu Ala Lys Gln Ile Val Asp Asn Ala Ile Gln Gln
   1               5                   10                  15
   Ala
<210> 39
   <211> 17
   <212> PRT
   <213> Artificial sequence
<220>
   <223> Description of Artificial Sequence: synthetic peptide
<400> 39
   Gln Ile Glu Tyr Ile Ala Lys Gln Ile Val Asp Asn Ala Ile Gln Gln
   1               5                   10                  15
   Ala
<210> 40
   <211> 17
   <212> PRT
   <213> Artificial Sequence
<220>
   <223> Description of Artificial Sequence: Synthetic peptide
<400> 40
   Gln Ile Glu Tyr Val Ala Lys Gln Ile Val Asp Asn Ala Ile Gln Gln
   1               5                   10                  15
   Ala
<210> 41
   <211> 17
   <212> PRT
   <213> Artificial Sequence
<220>
   <223> Description of Artificial Sequence: Synthetic peptide
<400> 41
   Gln Ile Glu Tyr Leu Ala Arg Gln Ile Val Asp Asn Ala Ile Gln Gln
   1               5                   10                  15
   Ala
<210> 42
   <211> 17
   <212> PRT
   <213> Artificial Sequence
<220>
   <223> Description of Artificial Sequence: Synthetic peptide
<400> 42
   Gln Ile Glu Tyr Leu Ala Lys Asn Ile Val Asp Asn Ala Ile Gln Gln
   1               5                   10                  15
   Ala
<210> 43
   <211> 17
   <212> PRT
   <213> Artificial Sequence
<220>
   <223> Description of Artificial Sequence: Synthetic peptide
<400> 43
   Gln Ile Glu Tyr Leu Ala Lys Gln Ile Val Glu Asn Ala Ile Gln Gln
   1               5                   10                  15
   Ala
<210> 44
   <211> 17
   <212> PRT
   <213> Artificial Sequence
<220>
   <223> Description of Artificial Sequence: Synthetic peptide
<400> 44
   Gln Ile Glu Tyr Leu Ala Lys Gln Ile Val Asp Gln Ala Ile Gln Gln
   1               5                   10                  15
   Ala
<210> 45
   <211> 17
   <212> PRT
   <213> Artificial Sequence
<220>
   <223> Description of Artificial Sequence: Synthetic peptide
<400> 45
   Gln Ile Glu Tyr Leu Ala Lys Gln Ile Val Asp Asn Ala Ile Asn Gln
   1 5 10 15
   Ala
<210> 46
   <211> 17
   <212> PRT
   <213> Artificial Sequence
<220>
   <223> Description of Artificial Sequence: Synthetic peptide
<400> 46
   Gln Ile Glu Tyr Leu Ala Lys Gln Ile Val Asp Asn Ala Ile Gln Asn
   1 5 10 15
   Ala
<210> 47
   <211> 17
   <212> PRT
   <213> Artificial sequence
<220>
   <223> Description of Artificial Sequence: Synthetic peptide
<400> 47
   Gln Ile Glu Tyr Leu Ala Lys Gln Ile Val Asp Asn Ala Ile Gln Gln
   1 5 10 15
   Leu
<210> 48
   <211> 17
   <212> PRT
   <213> Artificial sequence
<220>
   <223> Description of Artificial Sequence: Synthetic peptide
<400> 48
   Gln Ile Glu Tyr Leu Ala Lys Gln Ile Val Asp Asn Ala Ile Gln Gln
   1 5 10 15
   Ile
<210> 49
   <211> 17
   <212> PRT
   <213> Artificial Sequence
<220>
   <223> Description of Artificial Sequence: Synthetic peptide
<400> 49
   Gln Ile Glu Tyr Leu Ala Lys Gln Ile Val Asp Asn Ala Ile Gln Gln
   1               5                   10                  15
   Val
<210> 50
   <211> 17
   <212> PRT
   <213> Artificial Sequence
<220>
   <223> Description of Artificial Sequence: Synthetic peptide
<400> 50
   Gln Ile Glu Tyr Val Ala Lys Gln Ile Val Asp Tyr Ala Ile His Gln
   1               5                   10                  15
   Ala
<210> 51
   <211> 17
   <212> PRT
   <213> Artificial sequence
<220>
   <223> Description of Artificial Sequence: Synthetic peptide
<400> 51
   Gln Ile Glu Tyr Lys Ala Lys Gln Ile Val Asp His Ala Ile His Gln
   1               5                   10                  15
   Ala
<210> 52
   <211> 17
   <212> PRT
   <213> Artificial Sequence
<220>
   <223> Description of Artificial Sequence: Synthetic peptide
<400> 52
   Gln Ile Glu Tyr His Ala Lys Gln Ile Val Asp His Ala Ile His Gln
   1               5                   10                  15
   Ala
<210> 53
   <211> 17
   <212> PRT
   <213> Artificial Sequence
<220>
   <223> Description of Artificial Sequence: Synthetic peptide
<400> 53
   Gln Ile Glu Tyr Val Ala Lys Gln Ile Val Asp His Ala Ile His Gln
   1               5                   10                  15
   Ala
<210> 54
   <211> 18
   <212> PRT
   <213> Artificial Sequence
<220>
   <223> Description of Artificial Sequence: Synthetic peptide
<400> 54
   Pro Leu Glu Tyr Gln Ala Gly Leu Leu Val Gln Asn Ala Ile Gln Gln
   1               5                   10                  15
   Ala Ile
<210> 55
   <211> 18
   <212> PRT
   <213> Artificial Sequence
<220>
   <223> Description of Artificial Sequence: Synthetic peptide
<400> 55
   Leu Leu Ile Glu Thr Ala Ser Ser Leu Val Lys Asn Ala Ile Gln Leu
   1               5                   10                  15
   Ser Ile
<210> 56
   <211> 18
   <212> PRT
   <213> Artificial sequence
<220>
   <223> Description of Artificial Sequence: Synthetic peptide
<400> 56
   Leu Ile Glu Glu Ala Ala Ser Arg Ile Val Asp Ala Val Ile Glu Gln
   1               5                   10                  15
   Val Lys
<210> 57
   <211> 18
   <212> PRT
   <213> Artificial Sequence
<220>
   <223> Description of Artificial Sequence: synthetic peptide
<400> 57
   Ala Leu Tyr Gln Phe Ala Asp Arg Phe Ser Glu Leu Val Ile Ser Glu
   1               5                   10                  15
   Ala Leu
<210> 58
   <211> 17
   <212> PRT
   <213> Artificial Sequence
<220>
   <223> Description of Artificial Sequence: Synthetic peptide
<400> 58
   Leu Glu Gln Val Ala Asn Gln Leu Ala Asp Gln Ile Ile Lys Glu Ala
   1               5                   10                  15
   Thr
<210> 59
   <211> 17
   <212> PRT
   <213> Artificial Sequence
<220>
   <223> Description of Artificial Sequence: Synthetic peptide
<400> 59
   Phe Glu Glu Leu Ala Trp Lys Ile Ala Lys Met Ile Trp Ser Asp Val
   1               5                   10                  15
   Phe
<210> 60
   <211> 18
   <212> PRT
   <213> Artificial sequence
<220>
   <223> Description of Artificial Sequence: Synthetic peptide
<400> 60
   Glu Leu Val Arg Leu Ser Lys Arg Leu Val Glu Asn Ala Val Leu Lys
   1               5                   10                  15
   Ala Val
<210> 61
   <211> 18
   <212> PRT
   <213> Artificial Sequence
<220>
   <223> Description of Artificial Sequence: synthetic peptide
<400> 61
   Thr Ala Glu Glu Val Ser Ala Arg Ile Val Gln Val Val Thr Ala Glu
   1               5                   10                  15
   Ala Val
<210> 62
   <211> 18
   <212> PRT
   <213> Artificial Sequence
<220>
   <223> Description of Artificial Sequence: Synthetic peptide
<400> 62
   Gln Ile Lys Gln Ala Ala Phe Gln Leu Ile Ser Gln Val Ile Leu Glu
   1               5                   10                  15
   Ala Thr
<210> 63
   <211> 16
   <212> PRT
   <213> Artificial Sequence
<220>
   <223> Description of Artificial Sequence: Synthetic peptide
<400> 63
   Leu Ala Trp Lys Ile Ala Lys Met Ile Val Ser Asp Val Met Gln Gln
   1               5                   10                  15
<210> 64
   <211> 24
   <212> PRT
   <213> Artificial Sequence
<220>
   <223> Description of Artificial Sequence: Synthetic peptide
<400> 64
   Asp Leu Ile Glu Glu Ala Ala Ser Arg Ile Val Asp Ala Val Ile Glu
   1               5                   10                  15
   Gln Val Lys Ala Ala Gly Ala Tyr
               20
<210> 65
   <211> 18
   <212> PRT
   <213> Artificial Sequence
<220>
   <223> Description of Artificial Sequence: Synthetic peptide
<400> 65
   Leu Glu Gln Tyr Ala Asn Gln Leu Ala Asp Gln Ile Ile Lys Glu Ala
   1               5                   10                  15
   Thr Glu
<210> 66
   <211> 20
   <212> PRT
   <213> Artificial Sequence
<220>
   <223> Description of Artificial Sequence: Synthetic peptide
<400> 66
   Phe Glu Glu Leu Ala Trp Lys Ile Ala Lys Met Ile Trp Ser Asp Val
   1               5                   10                  15
   Phe Gln Gln Cys
               20
<210> 67
   <211> 17
   <212> PRT
   <213> Artificial Sequence
<220>
   <223> Description of Artificial Sequence: Synthetic peptide
<400> 67
   Gln Ile Glu Tyr Leu Ala Lys Gln Ile Pro Asp Asn Ala Ile Gln Gln
   1               5                   10                  15
   Ala
<210> 68
   <211> 25
   <212> PRT
   <213> Artificial sequence
<220>
   <223> Description of Artificial Sequence: Synthetic peptide
<400> 68
   Lys Gly Ala Asp Leu Ile Glu Glu Ala Ala Ser Arg Ile Val Asp Ala
   1               5                   10                  15
   Val Ile Glu Gln Val Lys Ala Ala Gly
               20                  25
<210> 69
   <211> 25
   <212> PRT
   <213> Artificial Sequence
<220>
   <223> Description of Artificial Sequence: Synthetic peptide
<400> 69
   Lys Gly Ala Asp Leu Ile Glu Glu Ala Ala Ser Arg Ile Pro Asp Ala
   1               5                   10                  15
   Pro Ile Glu Gln Val Lys Ala Ala Gly
               20                  25
<210> 70
   <211> 25
   <212> PRT
   <213> Artificial Sequence
<220>
   <223> Description of Artificial Sequence: Synthetic peptide
<400> 70
   Pro Glu Asp Ala Glu Leu Val Arg Leu Ser Lys Arg Leu Val Glu Asn
   1 5 10 15
   Ala Val Leu Lys Ala Val Gln Gln Tyr
   20 25
<210> 71
   <211> 25
   <212> PRT
   <213> Artificial Sequence
<220>
   <223> Description of Artificial Sequence: Synthetic peptide
<400> 71
   Pro Glu Asp Ala Glu Leu Val Arg Thr Ser Lys Arg Leu Val Glu Asn
   1 5 10 15
   Ala Val Leu Lys Ala Val Gln Gln Tyr
   20 25
<210> 72
   <211> 25
   <212> PRT
   <213> Artificial Sequence
<220>
   <223> Description of Artificial Sequence: Synthetic peptide
<400> 72
   Pro Glu Asp Ala Glu Leu Val Arg Leu Ser Lys Arg Asp Val Glu Asn
   1 5 10 15
   Ala Val Leu Lys Ala Val Gln Gln Tyr
   20 25
<210> 73
   <211> 25
   <212> PRT
   <213> Artificial sequence
<220>
   <223> Description of Artificial Sequence: Synthetic peptide
<400> 73
   Pro Glu Asp Ala Glu Leu Val Arg Leu Ser Lys Arg Leu Pro Glu Asn
   1 5 10 15
   Ala Val Leu Lys Ala Val Gln Gln Tyr
   20 25
<210> 74
   <211> 25
   <212> PRT
   <213> Artificial Sequence
<220>
   <223> Description of Artificial Sequence: Synthetic peptide
<400> 74
   Pro Glu Asp Ala Glu Leu Val Arg Leu Ser Lys Arg Leu Pro Glu Asn
   1               5                   10                  15
   Ala Pro Leu Lys Ala Val Gln Gln Tyr
               20                  25
<210> 75
   <211> 25
   <212> PRT
   <213> Artificial Sequence
<220>
   <223> Description of Artificial Sequence: Synthetic peptide
<400> 75
   Pro Glu Asp Ala Glu Leu Val Arg Leu Ser Lys Arg Leu Val Glu Asn
   1               5                   10                  15
   Ala Val Glu Lys Ala Val Gln Gln Tyr
               20                  25
<210> 76
   <211> 25
   <212> PRT
   <213> Artificial Sequence
<220>
   <223> Description of Artificial Sequence: Synthetic peptide
<400> 76
   Glu Glu Gly Leu Asp Arg Asn Glu Glu Ile Lys Arg Ala Ala Phe Gln
   1               5                   10                  15
   Ile Ile Ser Gln Val Ile Ser Glu Ala
               20                  25
<210> 77
   <211> 25
   <212> PRT
   <213> Artificial Sequence
<220>
   <223> Description of Artificial Sequence: Synthetic peptide
<400> 77
   Leu Val Asp Asp Pro Leu Glu Tyr Gln Ala Gly Leu Leu Val Gln Asn
   1 5 10 15
   Ala Ile Gln Gln Ala Ile Ala Glu Gln
   20 25
<210> 78
   <211> 25
   <212> PRT
   <213> Artificial Sequence
<220>
   <223> Description of Artificial Sequence: Synthetic peptide
<400> 78
   Gln Tyr Glu Thr Leu Leu Ile Glu Thr Ala Ser Ser Leu Val Lys Asn
   1 5 10 15
   Ala Ile Gln Leu Ser Ile Glu Gln Leu
   20 25
<210> 79
   <211> 25
   <212> PRT
   <213> Artificial Sequence
<220>
   <223> Description of Artificial Sequence: Synthetic peptide
<400> 79
   Leu Glu Lys Gln Tyr Gln Glu Gln Leu Glu Glu Glu Val Ala Lys Val
   1 5 10 15
   Ile Val Ser Met Ser Ile Ala Phe Ala
   20 25
<210> 80
   <211> 25
   <212> PRT
   <213> Artificial Sequence
<220>
   <223> Description of Artificial Sequence: Synthetic peptide
<400> 80
   Asn Thr Asp Glu Ala Gln Glu Glu Leu Ala Trp Lys Ile Ala Lys Met
   1               5                   10                  15
   Ile Val Ser Asp Ile Met Gln Gln Ala
               20                  25
<210> 81
   <211> 25
   <212> PRT
   <213> Artificial Sequence
<220>
   <223> Description of Artificial Sequence: Synthetic peptide
<400> 81
   Val Asn Leu Asp Lys Lys Ala Val Leu Ala Glu Lys Ile Val Ala Glu
   1               5                   10                  15
   Ala Ile Glu Lys Ala Glu Arg Glu Leu
               20                  25
<210> 82
   <211> 25
   <212> PRT
   <213> Artificial Sequence
<220>
   <223> Description of Artificial sequence: Synthetic peptide
<400> 82
   Asn Gly Ile Leu Glu Leu Glu Thr Lys Ser Ser Lys Leu Val Gln Asn
   1               5                   10                  15
   Ile Ile Gln Thr Ala Val Asp Gln Phe
               20                  25
<210> 83
   <211> 25
   <212> PRT
   <213> Artificial Sequence
<220>
   <223> Description of Artificial Sequence: Synthetic peptide
<400> 83
   Thr Gln Asp Lys Asn Tyr Glu Asp Glu Leu Thr Gln Val Ala Leu Ala
   1               5                   10                  15
   Leu Val Glu Asp Val Ile Asn Tyr Ala
               20                  25
<210> 84
   <211> 25
   <212> PRT
   <213> Artificial sequence
<220>
   <223> Description of Artificial Sequence: Synthetic peptide
<400> 84
   Glu Thr Ser Ala Lys Asp Asn Ile Asn Ile Glu Glu Ala Ala Arg Phe
   1 5 10 15
   Leu Val Glu Lys Ile Leu Val Asn His
   20 25
<210> 85
   <211> 5
   <212> PRT
   <213> Artificial Sequence
<220>
   <223> Description of Artificial Sequence: Synthetic peptide
<400> 85
   Asp Tyr Tyr Met Tyr
   1 5
<210> 86
   <211> 17
   <212> PRT
   <213> Artificial Sequence
<220>
   <223> Description of Artificial Sequence: Synthetic peptide
<400> 86
   Tyr Ile Thr Asn Tyr Gly Gly Ser Thr Tyr Tyr Pro Asp Thr Val Lys
   1 5 10 15
   Gly
<210> 87
   <211> 12
   <212> PRT
   <213> Artificial Sequence
<220>
   <223> Description of Artificial Sequence: Synthetic peptide
<400> 87
   Gln Ser Asn Tyr Asp Tyr Asp Gly Trp Phe Ala Tyr
   1               5                   10
<210> 88
   <211> 11
   <212> PRT
   <213> Artificial Sequence
<220>
   <223> Description of Artificial Sequence: Synthetic peptide
<400> 88
   Lys Ala Ser Gln Glu Val Gly Thr Ala Val Ala
   1               5                   10
<210> 89
   <211> 7
   <212> PRT
   <213> Artificial Sequence
<220>
   <223> Description of Artificial Sequence: Synthetic peptide
<400> 89
   Trp Ala Ser Thr Arg His Thr
   1               5
<210> 90
   <211> 9
   <212> PRT
   <213> Artificial Sequence
<220>
   <223> Description of Artificial Sequence: Synthetic peptide
<400> 90
   Gln Gln Tyr Ser Asn Tyr Pro Leu Thr
   1               5
<210> 91
   <211> 44
   <212> PRT
   <213> Artificial Sequence
<220>
   <223> Description of Artificial Sequence: Synthetic polypeptide
<220>
   <221> MOD_RES
   <222> (1)..(1)
   <223> ser or Thr
<220>
   <221> MOD_RES
   <222> (2)..(2)
   <223> His, Lys or Arg
<220>
   <221> MOD_RES
   <222> (4)..(4)
   <223> Gln or Asn
<220>
   <221> MOD_RES
   <222> (8)..(8)
   <223> Gly or Ala
<220>
   <221> MOD_RES
   <222> (10)..(10)
   <223> Thr or Ser
<220>
   <221> MOD_RES
   <222> (11)..(11)
   <223> Glu or Asp
<220>
   <221> MOD_RES
   <222> (14)..(14)
   <223> Gln or Asn
<220>
   <221> MOD_RES
   <222> (15)..(15)
   <223> Gly or Ala
<220>
   <221> MOD_RES
   <222> (17)..(17)
   <223> Thr or Ser
<220>
   <221> MOD_RES
   <222> (19)..(19)
   <223> Glu or Asp
<220>
   <221> MOD_RES
   <222> (22)..(22)
   <223> Arg or Lys
<220>
   <221> MOD_RES
   <222> (23)..(24)
   <223> Gln or Asn
<220>
   <221> MOD_RES
   <222> (27)..(27)
   <223> Asp or Glu
<220>
   <221> MOD_RES
   <222> (30)..(30)
   <223> Glu or Asp
<220>
   <221> MOD_RES
   <222> (32)..(32)
   <223> Ala, Leu, Ile or Val
<220>
   <221> MOD_RES
   <222> (34)..(34)
   <223> Glu or Asp
<220>
   <221> MOD_RES
   <222> (37)..(37)
   <223> Thr or Ser
<220>
   <221> MOD_RES
   <222> (38)..(38)
   <223> Arg or Lys
<220>
   <221> MOD_RES
   <222> (40)..(40)
   <223> Arg or Lys
<220>
   <221> MOD_RES
   <222> (41)..(41)
   <223> Glu or Asp
<220>
   <221> MOD_RES
   <222> (42)..(42)
   <223> Ala, Leu, Ile or val
<220>
   <221> MOD_RES
   <222> (43)..(43)
   <223> Arg or Lys
<220>
   <221> MOD_RES
   <222> (44)..(44)
   <223> Ala, Leu, Ile or Val
<400> 91
   Xaa Xaa Ile Xaa Ile Pro Pro Xaa Leu Xaa Xaa Leu Leu Xaa Xaa Tyr
   1               5                   10                  15
   Xaa Val Xaa Val Leu Xaa Xaa Xaa Pro Pro Xaa Leu Val Xaa Phe Xaa
               20                  25                  30
   Val Xaa Tyr Phe Xaa Xaa Leu Xaa Xaa Xaa Xaa Xaa
           35                  40
<210> 92
   <211> 17
   <212> PRT
   <213> Artificial Sequence
<220>
   <223> Description of Artificial sequence: Synthetic peptide
<220>
   <221> MOD_RES
   <222> (1)..(1)
   <223> Gln or Asn
<220>
   <221> MOD_RES
   <222> (2)..(2)
   <223> Ile, Leu or Val
<220>
   <221> MOD_RES
   <222> (3)..(3)
   <223> Glu or Asp
<220>
   <221> MOD_RES
   <222> (4)..(4)
   <223> Tyr, Phe, Thr or Ser
<220>
   <221> MOD_RES
   <222> (5)..(5)
   <223> Leu, Ile or val
<220>
   <221> MOD_RES
   <222> (7)..(7)
   <223> Lys or Arg
<220>
   <221> MOD_RES
   <222> (8)..(8)
   <223> Gln or Asn
<220>
   <221> MOD_RES
   <222> (11)..(11)
   <223> Asp or Glu
<220>
   <221> MOD_RES
   <222> (12)..(12)
   <223> Asn or Gln
<220>
   <221> MOD_RES
   <222> (15)..(16)
   <223> Gln or Asn
<220>
   <221> MOD_RES
   <222> (17)..(17)
   <223> Ala, Leu, Ile or Val
<400> 92
   Xaa Xaa Xaa Xaa Xaa Ala Xaa Xaa Ile Val Xaa Xaa Ala Ile Xaa Xaa
   1               5                   10                  15
   Xaa
<210> 93
   <211> 44
   <212> PRT
   <213> Artificial Sequence
<220>
   <223> Description of Artificial Sequence: Synthetic polypeptide
<220>
   <221> MOD_RES
   <222> (1)..(1)
   <223> Ser or Thr
<220>
   <221> MOD_RES
   <222> (4)..(4)
   <223> Gln or Asn
<220>
   <221> MOD_RES
   <222> (10)..(10)
   <223> Thr or Ser
<220>
   <221> MOD_RES
   <222> (18)..(18)
   <223> Val, Ile, Leu or Ala
<220>
   <221> MOD_RES
   <222> (23)..(23)
   <223> Gln or Asn
<220>
   <221> MOD_RES
   <222> (33)..(33)
   <223> Val, Ile, Leu or Ala
<220>
   <221> MOD_RES
   <222> (34)..(34)
   <223> Glu or Asp
<220>
   <221> MOD_RES
   <222> (37)..(37)
   <223> Thr or Ser
<220>
   <221> MOD_RES
   <222> (38)..(38)
   <223> Arg or Lys
<220>
   <221> MOD_RES
   <222> (40)..(40)
   <223> Arg or Lys
<220>
   <221> MOD_RES
   <222> (42)..(42)
   <223> Ala, Leu, Ile or Val
<220>
   <221> MOD_RES
   <222> (44)..(44)
   <223> Ala, Leu, Ile or Val
<400> 93
   Xaa His Ile Xaa Ile Pro Pro Gly Leu Xaa Glu Leu Leu Gln Gly Tyr
   1               5                   10                  15
   Thr Xaa Glu Val Leu Arg Xaa Gln Pro Pro Asp Leu Val Glu Phe Ala
               20                  25                  30
   Xaa Xaa Tyr Phe Xaa Xaa Leu Xaa Glu Xaa Arg Xaa
           35                  40
<210> 94
   <211> 121
   <212> PRT
   <213> Artificial sequence
<220>
   <223> Description of Artificial Sequence: Synthetic polypeptide
<400> 94
   Gln Val Gln Leu Gln Glu Ser Gly Gly Gly Val Val Gln Pro Gly Arg
   1 5 10 15
   Ser Leu Arg Leu Ser Cys Ser Ala Ser Gly Phe Thr Phe Ser Asp Tyr
   20 25 30
   Tyr Met Tyr Trp Val Arg Gln Ala Pro Gly Lys Gly Leu Glu Trp Val
   35 40 45
   Ala Tyr Ile Thr Asn Tyr Gly Gly Ser Thr Tyr Tyr Pro Asp Thr Val
   50 55 60
   Lys Gly Arg Phe Thr Ile Ser Arg Asp Asn Ala Lys Asn Thr Leu Phe
   65 70 75 80
   Leu Gln Met Asp Ser Leu Arg Pro Glu Asp Thr Gly Val Tyr Phe Cys
   85 90 95
   Ala Arg Gln Ser Asn Tyr Asp Tyr Asp Gly Trp Phe Ala Tyr Trp Gly
   100 105 110
   Gln Gly Thr Pro Val Thr Val Ser Ser
   115 120
<210> 95
   <211> 108
   <212> PRT
   <213> Artificial Sequence
<220>
   <223> Description of Artificial Sequence: Synthetic polypeptide
<400> 95
   Asp Ile Gln Leu Thr Gln Ser Pro Ser Ser Leu Ser Ala Ser Val Gly
   1 5 10 15
   Asp Arg Val Thr Ile Thr Cys Lys Ala Ser Gln Glu Val Gly Thr Ala
   20 25 30
   Val Ala Trp Tyr Gln Gln Lys Pro Gly Lys Ala Pro Lys Leu Leu Ile
   35 40 45
   Tyr Trp Ala Ser Thr Arg His Thr Gly Val Pro Ser Arg Phe Ser Gly
   50 55 60
   Ser Gly Ser Gly Thr Asp Phe Thr Phe Thr Ile Ser Ser Leu Gln Pro
   65 70 75 80
   Glu Asp Ile Ala Thr Tyr Phe Cys Gln Gln Tyr Ser Asn Tyr Pro Leu
   85 90 95
   Thr Phe Gly Gln Gly Thr Lys Val Glu Ile Lys Arg
   100 105
<210> 96
   <211> 47
   <212> DNA
   <213> Artificial Sequence
<220>
   <223> Description of Artificial Sequence: Synthetic primer
<400> 96
   ccgcggtcac atggcaccac ctctcttgca gcttccacca agggccc 47
<210> 97
   <211> 33
   <212> DNA
   <213> Artificial sequence
<220>
   <223> Description of Artificial Sequence: Synthetic primer
<400> 97
   ccggccgtcg cactcattta cccagagaca ggg 33
<210> 98
   <211> 52
   <212> DNA
   <213> Artificial Sequence
<220>
   <223> Description of Artificial Sequence: synthetic oligonucleotide
<400> 98
   gagtccaaat atggtccccc atgcccaccg tgcccaggta agccaaccca gg 52
<210> 99
   <211> 56
   <212> DNA
   <213> Artificial Sequence
<220>
   <223> Description of Artificial Sequence: Synthetic oligonucleotide
<400> 99
   cctgggttgg cttacctggg cacggtgggc atgggggacc atatttggac tctgca 56
<210> 100
   <211> 20
   <212> DNA
   <213> Artificial Sequence
<220>
   <223> Description of Artificial sequence: Synthetic primer
<400> 100
   gaacctcgcg gacagttaag 20
<210> 101
   <211> 53
   <212> DNA
   <213> Artificial Sequence
<220>
   <223> Description of Artificial Sequence: Synthetic primer
<400> 101
   ggatcctccg ccgccgcagc tcttaggttt cttgtccacc ttggtgttgc tgg 53
<210> 102
   <211> 55
   <212> PRT
   <213> Artificial Sequence
<220>
   <223> Description of Artificial Sequence: Synthetic polypeptide
<400> 102
   Gly Ser Gly Gly Gly Gly Ser Gly Gly Gly Gly Ser His Ile Gln Ile
   1 5 10 15
   Pro Pro Gly Leu Thr Glu Leu Leu Gln Gly Tyr Thr Val Glu Val Leu
   20 25 30
   Arg Gln Gln Pro Pro Asp Leu Val Glu Phe Ala Val Glu Tyr Phe Thr
   35 40 45
   Arg Leu Arg Glu Ala Arg Ala
   50 55
<210> 103
   <211> 92
   <212> DNA
   <213> Artificial sequence
<220>
   <223> Description of Artificial Sequence: Synthetic oligonucleotide
<400> 103
   gtggcgggtc tggcggaggt ggcagccaca tccagatccc gccggggctc acggagctgc 60
   tgcagggcta cacggtggag gtgctgcgac ag 92
<210> 104
   <211> 92
   <212> DNA
   <213> Artificial sequence
<220>
   <223> Description of Artificial Sequence: Synthetic oligonucleotide
<400> 104
   gcgcgagctt ctctcaggcg ggtgaagtac tccactgcga attcgacgag gtcaggcggc 60
   tgctgtcgca gcacctccac cgtgtagccc tg 92
<210> 105
   <211> 30
   <212> DNA
   <213> Artificial Sequence
<220>
   <223> Description of Artificial Sequence: Synthetic primer
<400> 105
   ggatccggag gtggcgggtc tggcggaggt 30
<210> 106
   <211> 30
   <212> DNA
   <213> Artificial Sequence
<220>
   <223> Description of Artificial Sequence: Synthetic primer
<400> 106
   cggccgtcaa gcgcgagctt ctctcaggcg 30
<210> 107
   <211> 29
   <212> PRT
   <213> Artificial Sequence
<220>
   <223> Description of Artificial Sequence: synthetic peptide
<400> 107
   Gly Ser Gly Gly Gly Gly Ser Gly Gly Gly Gly Ser Gln Ile Glu Tyr
   1               5                   10                  15
   Leu Ala Lys Gln Ile Val Asp Asn Ala Ile Gln Gln Ala
               20                  25
<210> 108
   <211> 96
   <212> DNA
   <213> Artificial sequence
<220>
   <223> Description of Artificial sequence: Synthetic oligonucleotide
<400> 108
   ggatccggag gtggcgggtc tggcggaggt ggcagccaga tcgagtacct ggccaagcag 60
   atcgtggaca acgccatcca gcaggcctga cggccg 96
<210> 109
   <211> 96
   <212> DNA
   <213> Artificial sequence
<220>
   <223> Description of Artificial Sequence: Synthetic oligonucleotide
<400> 109
   cggccgtcag gcctgctgga tggcgttgtc cacgatctgc ttggccaggt actcgatctg 60
   gctgccacct ccgccagacc cgccacctcc ggatcc 96
<210> 110
   <211> 30
   <212> DNA
   <213> Artificial Sequence
<220>
   <223> Description of Artificial Sequence: synthetic primer
<400> 110
   ggatccggag gtggcgggtc tggcggaggt 30
<210> 111
   <211> 22
   <212> DNA
   <213> Artificial sequence
<220>
   <223> Description of Artificial Sequence: Synthetic primer
<400> 111
   cggccgtcag gcctgctgga tg 22
<210> 112
   <211> 10
   <212> PRT
   <213> Artificial Sequence
<220>
   <223> Description of Artificial Sequence: Synthetic peptide
<400> 112
   Gly Gly Gly Gly Ser Gly Gly Gly Cys Gly
   1               5                   10
<210> 113
   <211> 73
   <212> DNA
   <213> Artificial sequence
<220>
   <223> Description of Artificial Sequence: Synthetic oligonucleotide
<400> 113
   gatccggagg tggcgggtct ggcggaggtt gcggccacat ccagatcccg ccggggctca 60
   cggagctgct gca 73
<210> 114
   <211> 65
   <212> DNA
   <213> Artificial Sequence
<220>
   <223> Description of Artificial sequence: Synthetic oligonucleotide
<400> 114
   gcagctccgt gagccccggc gggatctgga tgtggccgca acctccgcca gacccgccac 60
   ctccg 65
<210> 115
   <211> 93
   <212> DNA
   <213> Artificial Sequence
<220>
   <223> Description of Artificial Sequence: Synthetic oligonucleotide
<400> 115
   gatccggagg tggcgggtct ggcggatgtg gccagatcga gtacctggcc aagcagatcg 60
   tggacaacgc catccagcag gccggctgct gaa 93
<210> 116
   <211> 89
   <212> DNA
   <213> Artificial sequence
<220>
   <223> Description of Artificial Sequence: Synthetic oligonucleotide
<400> 116
   ttcagcagcc ggcctgctgg atggcgttgt ccacgatctg cttggccagg tactcgatct 60
   ggccacatcc gccagacccg ccacctccg 89
<210> 117
   <211> 9
   <212> PRT
   <213> Artificial Sequence
<220>
   <223> Description of Artificial Sequence: Synthetic peptide
<400> 117
   Gly Ser Gly Gly Gly Gly Ser Gly Gly
   1               5
<210> 118
   <211> 24
   <212> DNA
   <213> Artificial Sequence
<220>
   <223> Description of Artificial Sequence: Synthetic primer
<400> 118
   agatctggcg cacctgaact cctg 24
<210> 119
   <211> 33
   <212> DNA
   <213> Artificial Sequence
<220>
   <223> Description of Artificial Sequence: synthetic primer
<400> 119
   gaattcggat cctttacccg gagacaggga gag 33
<210> 120
   <211> 330
   <212> PRT
   <213> Artificial Sequence
<220>
   <223> Description of Artificial Sequence: synthetic polypeptide
<400> 120
   Ala Ser Thr Lys Gly Pro Ser Val Phe Pro Leu Ala Pro Ser Ser Lys
   1 5 10 15
   Ser Thr Ser Gly Gly Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Gln Thr
   65 70 75 80
   Tyr Ile Cys Asn Val Asn His Lys Pro Ser Asn Thr Lys Val Asp Lys
   85 90 95
   Lys Ala Glu Pro Lys Ser Cys Asp Lys Thr His Thr Cys Pro Pro Cys
   100 105 110
   Pro Ala Pro Glu Leu Leu Gly Gly Pro Ser Val Phe Leu Phe Pro Pro
   115 120 125
   Lys Pro Lys Asp Thr Leu Met Ile Ser Arg Thr Pro Glu Val Thr Cys
   130 135 140
   Val Val Val Asp Val Ser His Glu Asp Pro Glu Val Lys Phe Asn Trp
   145 150 155 160
   Tyr Val Asp Gly Val Glu Val His Asn Ala Lys Thr Lys Pro Arg Glu
   165 170 175
   Glu Gln Tyr Asn Ser Thr Tyr Arg Val Val Ser Val Leu Thr Val Leu
   180 185 190
   His Gln Asp Trp Leu Asn Gly Lys Glu Tyr Lys Cys Lys Val Ser Asn
   195 200 205
   Lys Ala Leu Pro Ala Pro Ile Glu Lys Thr Ile Ser Lys Ala Lys Gly
   210 215 220
   Gln Pro Arg Glu Pro Gln Val Tyr Thr Leu Pro Pro Ser Arg Asp Glu
   225 230 235 240
   Leu Thr Lys Asn Gln Val Ser Leu Thr Cys Leu Val Lys Gly Phe Tyr
   245 250 255
   Pro Ser Asp Ile Ala Val Glu Trp Glu Ser Asn Gly Gln Pro Glu Asn
   260 265 270
   Asn Tyr Lys Thr Thr Pro Pro Val Leu Asp Ser Asp Gly Ser Phe Phe
   275 280 285
   Leu Tyr Ser Lys Leu Thr Val Asp Lys Ser Arg Trp Gln Gln Gly Asn
   290 295 300
   Val Phe Ser Cys Ser Val Met His Glu Ala Leu His Asn His Tyr Thr
   305 310 315 320
   Gln Lys Ser Leu Ser Leu Ser Pro Gly Lys
   325 330
<210> 121
   <211> 330
   <212> PRT
   <213> Artificial Sequence
<220>
   <223> Description of Artificial Sequence: Synthetic polypeptide
<400> 121
   Ala Ser Thr Lys Gly Pro Ser Val Phe Pro Leu Ala Pro Ser Ser Lys
   1 5 10 15
   Ser Thr Ser Gly Gly Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Gln Thr
   65 70 75 80
   Tyr Ile Cys Asn Val Asn His Lys Pro Ser Asn Thr Lys Val Asp Lys
   85 90 95
   Arg Val Glu Pro Lys Ser Cys Asp Lys Thr His Thr Cys Pro Pro Cys
   100 105 110
   Pro Ala Pro Glu Leu Leu Gly Gly Pro Ser Val Phe Leu Phe Pro Pro
   115 120 125
   Lys Pro Lys Asp Thr Leu Met Ile Ser Arg Thr Pro Glu Val Thr Cys
   130 135 140
   Val Val Val Asp Val Ser His Glu Asp Pro Glu Val Lys Phe Asn Trp
   145 150 155 160
   Tyr Val Asp Gly Val Glu Val His Asn Ala Lys Thr Lys Pro Arg Glu
   165 170 175
   Glu Gln Tyr Asn Ser Thr Tyr Arg Val Val Ser Val Leu Thr Val Leu
   180 185 190
   His Gln Asp Trp Leu Asn Gly Lys Glu Tyr Lys Cys Lys Val Ser Asn
   195 200 205
   Lys Ala Leu Pro Ala Pro Ile Glu Lys Thr Ile Ser Lys Ala Lys Gly
   210 215 220
   Gln Pro Arg Glu Pro Gln Val Tyr Thr Leu Pro Pro Ser Arg Glu Glu
   225 230 235 240
   Met Thr Lys Asn Gln Val Ser Leu Thr Cys Leu Val Lys Gly Phe Tyr
   245 250 255
   Pro Ser Asp Ile Ala Val Glu Trp Glu Ser Asn Gly Gln Pro Glu Asn
   260 265 270
   Asn Tyr Lys Thr Thr Pro Pro Val Leu Asp Ser Asp Gly Ser Phe Phe
   275 280 285
   Leu Tyr Ser Lys Leu Thr Val Asp Lys Ser Arg Trp Gln Gln Gly Asn
   290 295 300
   Val Phe Ser Cys Ser Val Met His Glu Ala Leu His Asn His Tyr Thr
   305 310 315 320
   Gln Lys Ser Leu Ser Leu Ser Pro Gly Lys
   325 330
<210> 122
   <211> 5
   <212> PRT
   <213> Artificial Sequence
<220>
   <223> Description of Artificial Sequence: Synthetic peptide
<400> 122
   Gly Gly Gly Gly Ser
   1 5
<210> 123
   <211> 10
   <212> PRT
   <213> Artificial Sequence
<220>
   <223> Description of Artificial Sequence: Synthetic peptide
<400> 123
   Gly Gly Gly Gly Ser Gly Gly Gly Gly Ser
   1 5 10

## Description

### FIELD

The present invention is related to a complex comprising an anti-IGF-1R IgG antibody and two or more copies of an effector, for use in the treatment of cancer, and an mTOR inhibitor for use in the treatment of cancer.

### BACKGROUND

The insulin-like growth factor type I receptor (IGF-1R) is a member of the large class of tyrosine kinase receptors, which regulate a variety of intracellular pathways. IGF-1R binds IGF-1, a polypeptide hormone structurally similar to insulin (Laron, Mol Pathol. 2001, 54:311-16). The IGF-1 receptor is homologous to the insulin receptor (IR), sharing about 70% overall sequence homology with IR (Riedemann and Macaulay, Endocrine-Related Cancer, 2006, 13:S33-43). Not surprisingly, inhibitors developed against IGF-1R tend to show cross-reactivity with the insulin receptor, accounting for at least part of the toxicity profiles of such compounds (Miller and Yee, 2005, Cancer Res. 65:10123-27; Riedemann and Macaulay, 2006).

The IGF system plays an important role in regulating cell proliferation, differentiation, apoptosis and transformation (Jones et al, Endocrinology Rev. 1995. 16:3-34). The IGF system comprises two receptors, insulin like growth factor receptor 1 (IGF-1R; CD221) and insulin like growth factor receptor 2 (IGF-2R; CD222); two ligands, insulin like growth factor 1 (IGF-1) and IGF-2; and several IGF binding proteins (IGFBP-1 to IGFBP-6). In addition, a large group of IGFBP proteases (e.g., caspases, metalloproteinases, prostate-specific antigen) hydrolyze IGF bound IGFBP to release free IGFs, which then interact with IGF-1R and IGF-2R.

IGF-1R comprises two extracellular α subunits (130-135 kD) and two membrane spanning β-subunits (95 kD) that contain the cytoplasmic tyrosine kinase domain. IGF-1R, like the insulin receptor (IR), differs from other receptor tyrosine kinase family members by having a covalent dimeric (α2β2) structure. IGF-1R contains 84% sequence identity to IR in the kinase domain, while the membrane and C-terminal regions share 61% and 44% sequence identity, respectively (Ulrich et al., EMBO J., 1986, 5:2503-12; Blakesley et al., Cytokine Growth Factor Rev., 1996. 7:153-56).

IGF-1 and IGF-2 are activating ligands of IGF-1R. Binding of IGF-1 and IGF-2 to the α-chain induces conformational changes that result in autophosphorylation of each β-chain at specific tyrosine residues, converting the receptor from the unphosphorylated inactive state to the phosphorylated active state. The activation of three tyrosine residues in the activation loop (Tyr residues at 1131, 1135 and 1136) of the kinase domain leads to an increase in catalytic activity that triggers docking and phosphorylation of substrates such as IRS-1 and Shc adaptor proteins. Activation of these substrates leads to phosphorylation of additional proteins involved in the signaling cascade of survival (PI3K, AKT, TOR, S6) and/or proliferation (mitogen-activated protein kinase, p42/p44) (Pollak et al., Nature Reviews Cancer. 2004. 4:505-516; Baserga et al., Biochim Biophys Acta. 1997. 1332:F105-F126; Baserga et al, Int. J. Cancer. 2003. 107:873-77).

IGF-1R has anti-apoptotic effects in both normal and cancer cells (Resnicoff et al., 1995, Cancer Res. 55:2463-69; Kang et al., Am J Physiol Renal Physiol., 2003, 285:F1013-24; Riedemann and Macaulay, 2006). IGF-1R activation has been reported to be significant in the development of resistance to a variety of cytotoxic agents, such as chemotherapeutic agents, radionuclides and EGFR inhibitors (Jones et al., Endocr Relat Cancer 2004, 11:793-814; Warshamana-Greene et al., 2005, Clin. Cancer Res. 11:1563-71; Riedemann and Macaulay, 2006; Lloret et al., 2007, Gynecol. Oncol. 106:8-11). IGF-1R is overexpressed in a wide range of tumor lines, such as melanoma, neuroblastoma, colon cancer, prostate cancer, renal cancer, breast cancer and pancreatic cancer (Ellis et al., 1998, Breast Cancer Treat. 52:175-84; van Golen et al., 2000, Cell Death Differ. 7:654-65; Zhang et al., 2001, Breast Cancer Res. 2:170-75; Jones et al., 2004; Riedemann and Macaulay, 2006). A functional IGF-1R is required for transformation and promotes cancer cell growth, survival and metastasis (Riedemann and Macaulay, 2006).

Attempts have been made to develop IGF-1R inhibitors for use as anti-cancer agents, such as tyrphostins, pyrrolo[2,3-d]-pyrimidine derivatives, nordihydroguaiaretic acid analogs, diaryureas, AG538, AG1024, NVP-AEW541, NVP-ADW742, BMS-5326924, BMS-554417, OSI-906, INSM-18, luteolin, simvastatin, silibinin, black tea polyphenols, picropodophyllin, anti-IGF-1R antibodies and siRNA inhibitors (Arteaga et al., 1998, J Clin Invest. 84:1418-23; Warshamana-Greene et al., 2005; Klein and Fischer, 2002, Carcinogenesis 23:217-21; Blum et al., 2000, Biochemistry 39:15705-12; Garcia-Echeverria et al., 2004, Cancer Cell 5:231-39; Garber, 2005, JNCI 97:790-92; Bell et al., 2005, Biochemistry 44:930-40; Wu et al., 2005, Clin Cancer Res 11:3065-74; Wang et al., 2005, Mol Cancer Ther 4:1214-21; Singh and Agarwal, 2006, Mol Carinog. 45:436-42; Gable et al., 2006, Mol Cancer Ther 5:1079-86; Niu et al., Cell Biol Int., 2007, 31:156-64; Blecha et al., 2007, Biorg Med Chem Lett. 17:4026-29; Qian et al., 2007, Acta Biochim Biophys Sin, 39:137-47; Fang et al., 2007, Carcinogenesis 28:713-23; Cohen et al., 2005, Clin Cancer Res 11:2063-73; Sekine et al., Biochem Biophys Res Commun., 2008, 25:356-61; Haluska et al., 2008, J Clin Oncol. 26:May 20 suppl; abstr 14510; U.S. Patent Application Publ. No. 2006-233810). Typically, these agents have tended to cross-react to a greater or lesser extent with both IGF-1R and IR and/or to act as IGF-1R agonists. The use of such agents for cancer therapy has been limited by their toxicity (Riedemann and Macaulay, 2006). A need exists in the field for more effective forms of anti-IGF-1R antibodies and complexes thereof.

US 2011/0123436 A1 is related to compositions and methods of use of targeted delivery complexes for delivery of siRNA to a disease-associated cell, tissue or pathogen.

Thakur ML et al. (Journal of Immunotherapy, vol. 20, no. 3, 1 January 1997, pp. 194-201) report on improved antibody targeting with interferon-alpha-2B conjugate.

US 2009/060862 A1 is related to methods and compositions for forming PEGylated complexes of defined stoichiometry and structure.

WO 2011/090492 A1 is related to a class of monospecific and bispecific humanized antibodies that target insulin-like growth factor type I receptor (IGF-1R).

Wan X et al. (Oncogene, vol. 26, no. 13, 25 September 2006, pp. 1932-1940) report that rapamycin induces feedback activation of Akt signaling through an IGF-1R-dependent mechanism.

Coulter DW et al. (Anticancer Research 28: 1509-1516 (2008)) report that IGF-1 receptor inhibition combined with rapamycin or temsirolimus inhibits neuroblastoma cell growth.

Kurmasheva RT et al. (Cancer Research, vol. 69, no. 19, 29 September 2009, pp. 7662-7671) report that insulin-like growth factor-1 receptor-targeting antibody CP-751,871 suppresses tumor-derived VEGF and synergizes with rapamycin in models of childhood sarcoma.

Anders Kolb A et al. (Pediatric Blood & Cancer, 2010, 55: 67-75) report that R1507 which is a fully human monoclonal antibody targeting IGF-1R, is effective alone and in combination with rapamycin in inhibiting growth of osteosarcoma xenografts.

Sathyanarayanan S et al. (2009 AACR Annual Meeting, April 18-22, 2009, abstract 2809) report that combination treatment with the anti-IGF-1R antibody MK-0646 and the mTOR inhibitor deforolimus leads to more effective PI3K pathway targeting and anti-tumor activity.

Naing A et al. (Clinical Cancer Research, vol. 17, no. 18, 15 September 2011, pp. 6052-6060) report on a phase I trial of cixutumumab combined with temsirolimus in patients with advanced cancer.

Quek R et al. (Clin. Cancer Res., vol. 17, no. 4, 22 December 2010, pp. 871-879) report on a phase I trial of everolimus and figitumumab in patients with advanced sarcomas and other solid tumors.

### SUMMARY

The problem underlying the present invention is solved by the subject matter of the attached independent claims. Preferred embodiments may be taken from the attached dependent claims.

More specifically, the problem underlying the present invention is solved in a first aspect by a complex comprising
(i) an anti-IGF-1R IgG antibody attached to one or more anchor domain (AD) moieties from an AKAP protein, and
(ii) two or more copies of an effector attached to a dimerization and docking domain (DDD) moiety from a human protein kinase A (PKA) regulatory subunit RIα, RIβ, RIIa or RIIβ, wherein the effector is a second antibody fragment, a second antibody or a cytokine,
   wherein two copies of the DDD moiety form a dimer that binds to the AD moiety to form the complex,
   for use in a method of treating a cancer that expresses IGF-1R, wherein the method comprises:
   a) administering the complex to an individual with the cancer; and
   b) administering an mTOR inhibitor to the individual, wherein the mTOR inhibitor
   is selected from the group consisting of temsirolimus, rapamycin, ridaforolimus and everolimus;
   wherein the combination of anti-IGF-1R antibody or antigen-binding fragment and mTOR inhibitor is more effective than either alone.

More specifically, the problem underlying the present invention is solved in a second aspect by an mTOR inhibitor selected from the group consisting of temsirolimus, rapamycin, ridaforolimus and everolimus, for use in a method of treating a cancer that expresses IGF-1R, wherein the method comprises:
a) administering to an individual with the cancer a complex comprising
   (i) an anti-IGF-1R IgG antibody attached to one or more anchor domain (AD) moieties from an AKAP protein, and
   (ii) two or more copies of an effector attached to a dimerization and docking domain (DDD) moiety from a human protein kinase A (PKA) regulatory subunit RIα, RIβ, RIIα or RIIβ, wherein the effector is a second antibody fragment, a second antibody or a cytokine,
      wherein two copies of the DDD moiety form a dimer that binds to the AD moiety to form the complex; and
b) administering the mTOR inhibitor to the individual;
wherein the combination of anti-IGF-1R antibody or antigen-binding fragment and mTOR inhibitor is more effective than either alone.

In an embodiment of both the first and the second aspect, the second antibody fragment is an anti-IGF-1R antibody fragment, an anti-TROP2 antibody fragment or an anti-CEACAM6 antibody fragment and wherein the second antibody is an anti-IGF-1R antibody, an anti-TROP2 antibody or an anti-CEACAM6 antibody.

In an embodiment of both the first and the second aspect, the second antibody fragment is an anti-IGF-1R antibody fragment and the second antibody is an anti-IGF-1R antibody.

In an embodiment of both the first and the second aspect, the second antibody fragment binds to a tumor-associated antigen selected from the group consisting of carbonic anhydrase IX, CCCL19, CCCL21 , CSAp, CD1, CD1a, CD2, CD3, CD4, CD5, CD8, CD11A, CD14, CD15, CD16, CD18, CD19, CD20, IGF-1R, CD21, CD22, CD23, CD25, CD29, CD30, CD32b, CD33, CD37, CD38, CD40, CD40L, CD45, CD46, CD52, CD54, CD55, CD59, CD64, CD66a-e, CD67, CD70, CD74, CD79a, CD80, CD83, CD95, CD126, CD133, CD138, CD147, CD154, CEACAM5, CEACAM6, B7, ED-B fibronectin, Factor H, FHL-1, Flt-3, folate receptor, GROB, HMGB-1, hypoxia inducible factor (HIF), HM1.24, insulin-like growth factor-1 (ILGF-1), IFN-γ, IFN-α, IFN-β, IL-2, IL-4R, IL-6R, IL-13R, IL-15R, IL-17R, IL-18R, IL-6, IL-8, IL-12, IL-15, IL-17, IL-18, IL-25, IP-10, MAGE, mCRP, MCP-1, MIP-1A, MIP-1B, MIF, MUC1, MUC2, MUC3, MUC4, MUC5ac, PAM4 antigen, NCA-95, NCA-90, PSMA, EGP-1, EGP-2, AFP, la, HM1.24, HLA-DR, tenascin, Le(y), RANTES, T101, TAC, Tn antigen, Thomson-Friedenreich antigens, tumor necrosis antigens, TNF-α, TRAIL receptor (R1 and R2), VEGFR, EGFR, P1GF, complement factors C3, C3a, C3b, C5a, C5, and an oncogene product.

In an embodiment of both the first and the second aspect, the cytokine is interferon-α2b.

In an embodiment of both the first and the second aspect, the cytokine is selected from the group consisting of human growth hormone, N-methionyl human growth hormone, parathyroid hormone, thyroxine, insulin, proinsulin, relaxin, prorelaxin, follicle stimulating hormone (FSH), thyroid stimulating hormone (TSH), luteinizing hormone (LH), hepatic growth factor, prostaglandin, fibroblast growth factor, prolactin, placental lactogen, tumor necrosis factor-α, tumor necrosis factor-β, mullerian-inhibiting substance, mouse gonadotropin-associated peptide, inhibin, activin, vascular endothelial growth factor, integrin, thrombopoietin (TPO), NGF-β, platelet-growth factor, TGF-α, TGF- β, insulin-like growth factor-I, insulin-like growth factor-II, erythropoietin (EPO), interferon-α, interferon-β, interferon-γ, macrophage-CSF (M-CSF), interleukin-1 (IL-1), IL-la , IL-2, IL-3, IL-4, IL-5, IL-6, IL-7, IL-8, IL-9, IL-10, IL-11, IL-12, IL-13, IL-14, IL-15, IL-16, IL-17, IL-18, IL-21, IL-25, LIF, FLT-3, angiostatin, thrombospondin, endostatin, tumor necrosis factor and lymphotoxin.

In an embodiment of both the first and the second aspect, the anti-IGF- 1R antibody or fragment thereof binds to the first half of the cysteine-rich domain of IGF-1R, between amino acid residues 151 and 222 of human IGF-1R.

In an embodiment of both the first and the second aspect, the anti-IGF-1R antibody or fragment thereof comprises the heavy chain CDR sequences CDR1 (DYYMY, SEQ ID NO:85), CDR2 (YITNYGGSTYYPDTVKG, SEQ ID NO:86) and CDR3 (QSNYDYDGWFAY, SEQ ID NO:87) and the light chain CDR sequences CDR1 (KASQEVGTAVA, SEQ ID NO:88), CDR2 (WASTRHT, SEQ ID NO:89) and CDR3 (QQYSNYPLT, SEQ ID NO:90).

In an embodiment of both the first and the second aspect, the cancer is selected from the group consisting of Wilms' tumor, Ewing sarcoma, a neuroendocrine tumor, a glioblastoma, a neuroblastoma, a melanoma, skin cancer, breast cancer, colon cancer, rectal cancer, prostate cancer, liver cancer, renal cancer, pancreatic cancer, lung cancer, biliary cancer, cervical cancer, endometrial cancer, esophageal cancer, gastric cancer, head and neck cancer, medullary thyroid carcinoma, ovarian cancer, glioma, lymphoma, leukemia, myeloma, acute lymphoblastic leukemia, acute myelogenous leukemia, chronic lymphocytic leukemia, chronic myelogenous leukemia, Hodgkin's lymphoma, non-Hodgkin's lymphoma, and urinary bladder cancer.

In an embodiment of both the first and the second aspect, the mTOR inhibitor is temsirolimus.

In an embodiment of both the first and the second aspect, the anti-IGF-1R antibody is a naked antibody or is conjugated to at least one therapeutic agent.

In an embodiment of both the first and the second aspect, said therapeutic agent is selected from the group consisting of a radionuclide, an immunomodulator, an anti-angiogenic agent, a cytokine, a chemokine, a growth factor, a hormone, a drug, a prodrug, an enzyme, an oligonucleotide, a pro-apoptotic agent, an interference RNA, a photoactive therapeutic agent, a cytotoxic agent, a chemotherapeutic agent and a toxin.

In an embodiment of both the first and the second aspect, the drug is selected from the group consisting of 5-fluorouracil, aplidin, azaribine, anastrozole, anthracyclines, bendamustine, bleomycin, bortezomib, bryostatin-1, busulfan, calicheamycin, camptothecin, carboplatin, 10-hydroxycamptothecin, carmustine, celebrex, chlorambucil, cisplatin (CDDP), Cox-2 inhibitors, irinotecan (CPT-11), SN-38, carboplatin, cladribine, camptothecans, cyclophosphamide, cytarabine, dacarbazine, docetaxel, dactinomycin, daunorubicin, doxorubicin, 2-pyrrolinodoxorubicine (2P-DOX), cyano-morpholino doxorubicin, doxorubicin glucuronide, epirubicin glucuronide, estramustine, epipodophyllotoxin, estrogen receptor binding agents, etoposide (VP16), etoposide glucuronide, etoposide phosphate, floxuridine (FUdR), 3',5'-0-dioleoyl-FudR (FUdR-dO), fludarabine, flutamide, farnesyl-protein transferase inhibitors, gemcitabine, hydroxyurea, idarubicin, ifosfamide, L-asparaginase, lenolidamide, leucovorin, lomustine, mechlorethamine, melphalan, mercaptopurine, 6-mercaptopurine, methotrexate, mitoxantrone, mithramycin, mitomycin, mitotane, navelbine, nitrosourea, plicomycin, procarbazine, paclitaxel, pentostatin, PSI-341, raloxifene, semustine, streptozocin, tamoxifen, taxol, temazolomide (an aqueous form of DTIC), transplatinum, thalidomide, thioguanine, thiotepa, teniposide, topotecan, uracil mustard, vinorelbine, vinblastine, vincristine and vinca alkaloids.

In an embodiment of both the first and the second aspect, the radioisotope is selected from the group consisting of ¹¹¹In_{,} ¹⁷⁷Lu, ²¹²Bi, ²¹³Bi, ²¹¹At, ⁶²Cu, ⁶⁷Cu, ⁹⁰Y, ¹²⁵I, ¹³¹I, ³²P, ³³P, ⁴⁷Sc, ¹¹¹Ag, ⁶⁷Ga, ¹⁴²Pr, ¹⁵³Sm, ¹⁶¹Tb, ¹⁶⁶Dy, ¹⁶⁶Ho, ¹⁸⁶Re, ¹⁸⁸Re, ¹⁸⁹Re, ²¹²Pb, ²²³Ra, ²²⁵Ac, ⁵⁹Fe, ⁷⁵Se, ⁷⁷As, ⁸⁹Sr, ⁹⁹Mo, ¹⁰⁵Rh, ¹⁰⁹Pd, ¹⁴³Pr, ¹⁴⁹Pm, ¹⁶⁹Er, ¹⁹⁴Ir, ¹⁹⁸Au, ¹⁹⁹Au, and ²¹¹Pb.

Preferably, the anti-IGF-1R antibodies bind to IGF-1R but not to IR. More preferably, the anti-IGF-1R antibodies are not agonists of IGF-1R. Most preferably, the anti-IGF-1R antibodies bind to an epitope of IGF-1R comprising the first half of the cysteine-rich domain of IGF-1R, between amino acid residues 151 and 222 of the human IGF-1R sequence. (See, e.g., Adams et al., Cell Mol Life Sci 57:1050-93,2000; NCBI Accession No. AAB22215).

In certain embodiments, the anti-IGF-1R antibody is a murine, chimeric, humanized or human antibody or antigen-binding fragment thereof comprising the heavy chain CDR sequences CDR1 (DYYMY, SEQ ID NO:85), CDR2 (YITNYGGSTYYPDTVKG, SEQ ID NO:86) and CDR3 (QSNYDYDGWFAY, SEQ ID NO:87) and the light chain CDR sequences CDR1 (KASQEVGTAVA, SEQ ID NO:88), CDR2 (WASTRHT, SEQ ID NO:89) and CDR3 (QQYSNYPLT, SEQ ID NO:90). In alternative embodiments, the anti-IGF-1R antibody is a chimeric, humanized or human antibody that binds to the same epitope of IGF-1R and/or that blocks binding to IGF-1R of a murine R1 antibody comprising the heavy chain CDR sequences CDR1 (DYYMY, SEQ ID NO:85), CDR2 (YITNYGGSTYYPDTVKG, SEQ ID NO:86) and CDR3 (QSNYDYDGWFAY, SEQ ID NO:87) and the light chain CDR sequences CDR1 (KASQEVGTAVA, SEQ ID NO:88), CDR2 (WASTRHT, SEQ ID NO:89) and CDR3 (QQYSNYPLT, SEQ ID NO:90). The anti-IGF-1R antibody may be a naked antibody or may be an immunoconjugate attached to at least one therapeutic agent and/or at least one diagnostic agent.

Although the second anti-TAA antibody or fragment thereof may be an anti-TROP2 or anti-CEACAM6 antibody or fragment, in alternative embodiments the second antibody or fragment may bind to any of a number of known tumor-associated antigens, such as carbonic anhydrase IX, CCCL19, CCCL21, CSAp, CD1, CD1a, CD2, CD3, CD4, CD5, CD8, CD11A, CD14, CD15, CD16, CD18, CD19, IGF-1R, CD20, CD21, CD22, CD23, CD25, CD29, CD30, CD32b, CD33, CD37, CD38, CD40, CD40L, CD45, CD46, CD52, CD54, CD55, CD59, CD64, CD66a-e, CD67, CD70, CD74, CD79a, CD80, CD83, CD95, CD126, CD133, CD138, CD147, CD154, AFP, PSMA, CEACAM5, CEACAM-6, B7, ED-B of fibronectin, Factor H, FHL-1, Flt-3, folate receptor, GROB, HMGB-1, hypoxia inducible factor (HIF), HM1.24, insulin-like growth factor-1 (ILGF-1), IFN-γ, IFN-α, IFN-β, IL-2, IL-4R, IL-6R, IL-13R, IL-15R, IL-17R, IL-18R, IL-6, IL-8, IL-12, IL-15, IL-17, IL-18, IL-25, IP-10, MAGE, mCRP, MCP-1, MIP-1A, MIP-1B, MIF, MUC1, MUC2, MUC3, MUC4, MUC5ac, PAM4 antigen, NCA-95, NCA-90, la, HM1.24, EGP-1, EGP-2, HLA-DR, tenascin, Le(y), RANTES, T101, TAC, Tn antigen, Thomson-Friedenreich antigens, tumor necrosis antigens, TNF-α, TRAIL receptor (R1 and R2), VEGFR, EGFR, PlGF, complement factors C3, C3a, C3b, C5a, C5.

The second anti-TAA antibody may be selected from any of a wide variety of anti-cancer antibodies known in the art, including but not limited to hPAM4 (U.S. Patent No. 7,282,567), hA20 (U.S. Patent No. 7,251,164), hA19 (U.S. Patent No. 7,109,304), hIMMU31 (U.S. Patent No. 7,300,655), hLL1 (U.S. Patent No. 7,312,318,), hLL2 (U.S. Patent No. 7,074,403), hMu-9 (U.S. Patent No. 7,387,773), hL243 (U.S. Patent No. 7,612,180), hMN-14 (U.S. Patent No. 6,676,924), hMN-15 (U.S. Patent No. 7,541,440), hR1 (U.S. Provisional Patent Application 61/145,896), hRS7 (U.S. Patent No. 7,238,785), hMN-3 (U.S. Patent No. 7,541,440), AB-PG1-XG1-026 (U.S. Patent Application 11/983,372, deposited as ATCC PTA-4405 and PTA-4406) and D2/B (WO 2009/130575). In certain embodiments, a second, different anti-IGF-1R antibody may be used, such as any of the anti-IGF-1R antibodies in clinical development (see, e.g., Ryan and Goss, The Oncologist, 2008, 13:16-24).

In various embodiments, the complexes are DOCK-AND-LOCK™ (DNL™) complexes. The technology to make DNL™ complexes has been described in U.S. Patent Nos. 7,521,056; 7,527,787; 7,534,866; 7,550,143; 7,666,400; 7,906,118; 8,003,111 and 8,034,352. The technique relies upon the binding interaction between a dimerization and docking domain (DDD) moiety of human protein kinase A (PKA) regulatory subunit RIα, RIβ, RIIα or RIIβ and an anchor domain (AD) moiety of an A-kinase anchoring protein (AKAP). The PKA DDD moieties spontaneously form dimers that bind to an AD moiety to join the complex together. The AD and DDD moiety may be attached to an effector, such as an antibody, antibody fragment, cytokine, toxin, enzyme, hormone or other protein or peptide, for example in the form of a fusion protein. Alternatively, the AD and DDD moieties may be attached to effectors by other covalent linkage, such as by chemical cross-linking. The technique is not limiting and any effector moiety that may be attached to an AD or DDD moiety may be incorporated into a DNL™ complex.

The anti-IGF-1R containing complex may be administered alone, or in combination with one or more therapeutic agents. The agents may be attached to the complex or may be administered separately. As discussed below, therapeutic agents may include, but are not limited to, radionuclides, immunomodulators, anti-angiogenic agents, cytokines, chemokines, growth factors, hormones, drugs, prodrugs, enzymes, oligonucleotides, siRNAs, pro-apoptotic agents, photoactive therapeutic agents, cytotoxic agents, chemotherapeutic agents, toxins, other antibodies or antigen binding fragments thereof. In preferred embodiments the therapeutic agent may be an EGFR inhibitor (e.g., erlotinib or anti-EGFR antibody, such as erbitux), an IGF-1R inhibitor such as tryphostins (e.g., AG1024, AG538), pyrrolo[2,3-d]-pyrimidine derivatives (e.g., NVP-AEW541) or an mTOR inhibitor such as temsirolimus, rapamycin, ridaforolimus or everolimus.

Any cancer or diseased cell that expresses IGF-1R may be treated and/or diagnosed with the anti-IGF-1R antibodies, including but not limited to Wilms' tumor, Ewing sarcoma, neuroendocrine tumors, glioblastomas, neuroblastoma, melanoma, skin, breast, colon, rectum, prostate, liver, renal, pancreatic and/or lung cancer, as well as lymphomas, leukemias, and myelomas. Other forms of cancer that may be treated include but are not limited to acute lymphoblastic leukemia, acute myelogenous leukemia, biliary cancer, cervical cancer, chronic lymphocytic leukemia, chronic myelogenous leukemia, endometrial cancer, esophageal cancer, gastric cancer, head and neck cancer, Hodgkin's lymphoma, medullary thyroid carcinoma, non-Hodgkin's lymphoma, ovarian cancer, glioma and urinary bladder cancer.

### BRIEF DESCRIPTION OF THE DRAWINGS

The following drawings form part of the present specification and are included to further demonstrate certain embodiments of the present invention. The embodiments may be better understood by reference to one or more of these drawings in combination with the detailed description of specific embodiments presented herein.

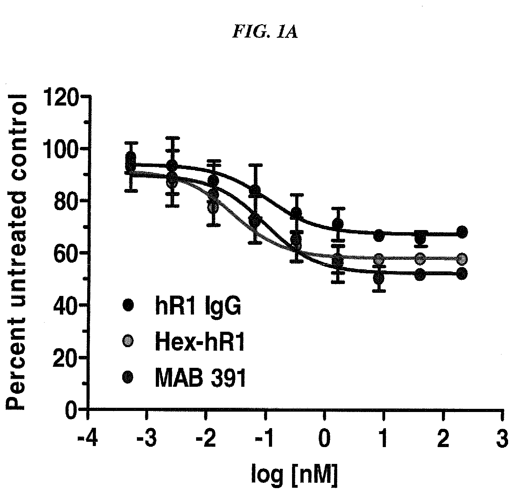

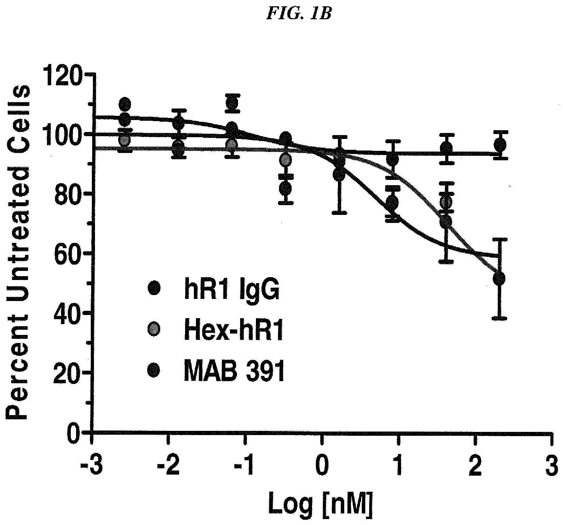

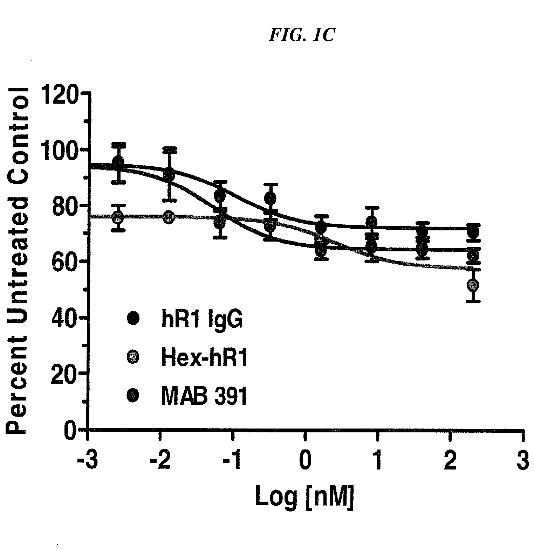

**FIG. 1****. Anti-IGF-1R mediated growth inhibition under serum-free conditions.** Cells were grown in serum-free media containing holo-transferrin (10 mg/mL) for 24 h before the addition of the antibodies. After a 1-h incubation with the antibodies, IGF-1 (100 ng/mL) was added to all the wells. Plates were incubated for a further 96 h before MTS reagent was added and cell growth inhibition determined using Prism Graph Pad software. The cell lines examined were (**A**) Caki-2 cells, (**B**) ACHN cells and (**C**) 786-0 cells.

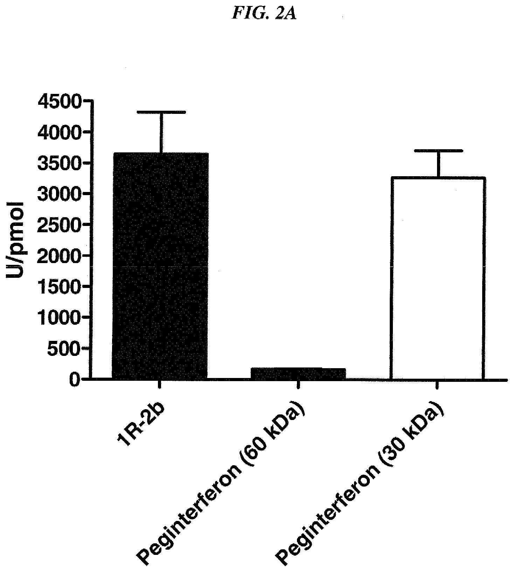

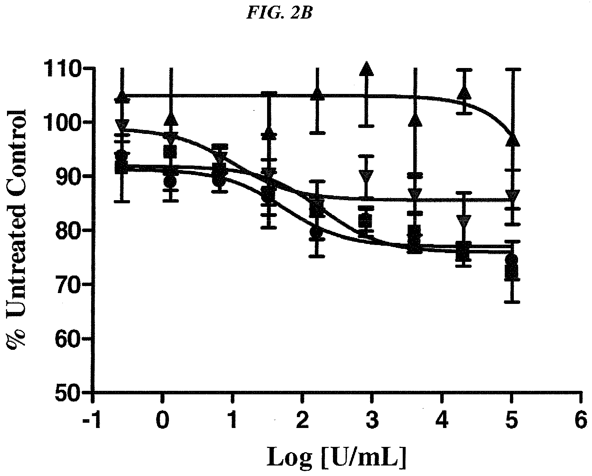

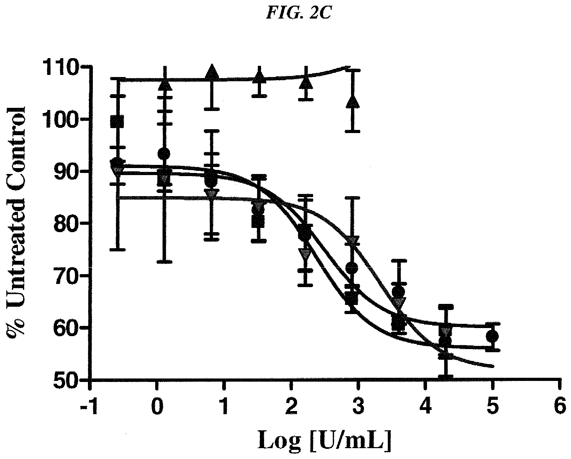

**FIG.2****. Characterization** of **1R-2b in RCC.** (**A**) Based on the luciferase reporter gene assay (iLite kit), **1R-2b** yielded a specific activity of 15x10⁶ U/mg or 3750 U/pmole versus 180 and 3255 U/pmole for two different pegylated-IFN molecules. In growth inhibition assays of **(B)** 786-O and (C) ACHN, **1R-2b** had EC50 values of 49 and 62 pM, respectively.

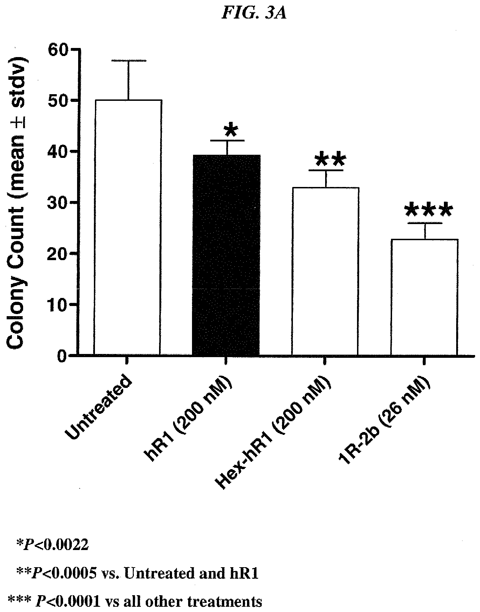

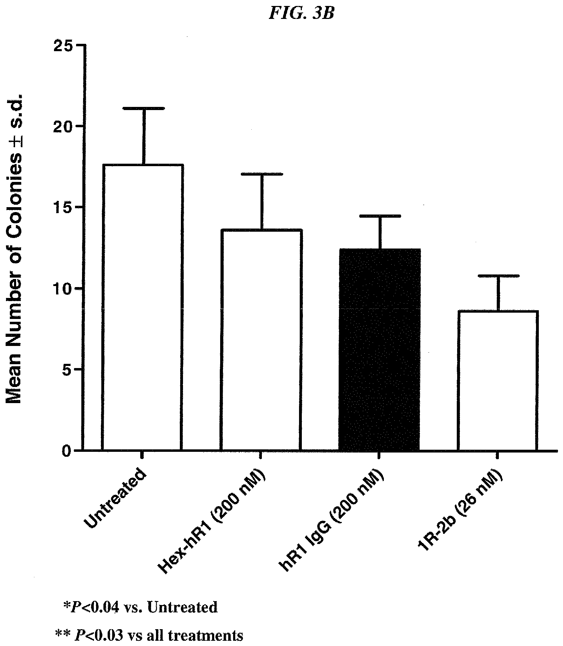

**FIG.3****. Growth inhibition under anchorage-independent conditions.** A 1% base agar was mixed 1:1 with 2x growth media (10% FBS final concentration) and added to wells of a 24-well plate. Either **(A)** ACHN cells or **(B)** 786-0 cells in 2x growth media were mixed 1:1 with 0.7% agarose and added (1250 cells per well) to the base agar. Cells were fed by weekly replacement of growth media on the top of the agarose layer. Treated wells contained the test articles in the agarose/cell layer at the beginning and in subsequent feedings. Once colonies were clearly visible by microscopy in untreated control wells, the medium was removed and the colonies stained with crystal violet. Colonies were counted under a microscope and the average number was determined from five different fields of view within the well.

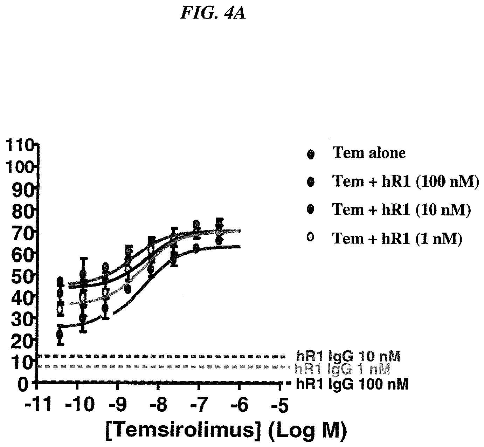

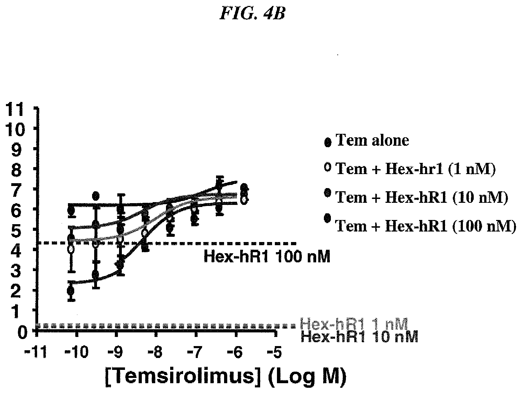

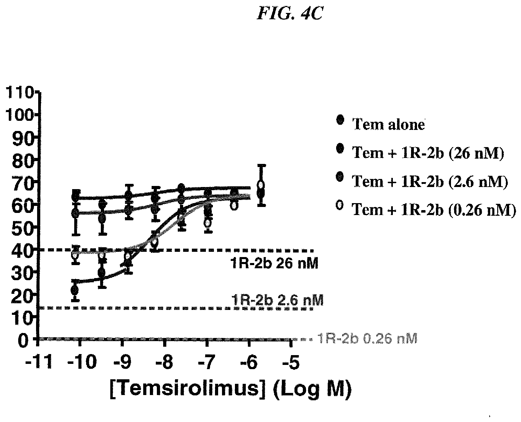

**FIG. 4****. Synergy between anti-IGF-1R treatment and an mTOR inhibitor.** ACHN cells were harvested, washed in PBS several times to remove FBS, and plated in 96-wells plates overnight in SFM. On the following day, various doses (1 mM to 0.06 nM) of the mTOR inhibitor temsirolimus was added to the plates with and without hR1 or Hex-hR1 (100, 10, and 1 nM constant amounts) or 1R-2b (26, 2.6, or 0.26 nM; NOTE: 26 nM 1R-2b ∼100,000 Units/mL of IFN). IGF-1 was added at 100 ng/mL. Plates were incubated for 96-h before MTS substrate was added to all the wells and the plates read at 492nm. Data was graphed as Percent Growth Inhibition vs. [temsirolimus]. IC50-values for temsirolimus were determined for each condition and Combinatorial Index (CI) was calculated based on changes in these values when co-incubated with hRl, Hex-hR1, or 1R-2b (CI<1 for synergy). **(A)** Combination of temsirolimus with hR1 (CI = 0.64). The IC₅₀ values for temsirolimus concentration needed to mediate 50% inhibition of cell growth were 7.76 nM for Tem alone (R² 0.94); 1.45 nM with 100 nM hR1 (R² 0.88); 0.56 nM with 10 nM hR1 (R² 0.84); and 2.86 nM with 1 nM hR1 (R² 0.93). (**B**) Combination of temsirolimus with Hex-hR1 (CI = 0.43). The IC₅₀ values were 7.76 nM for Tem alone (R² 0.94); 3.15 nM with 1 nM Hex-hR1 (R² 0.63); 0.06 nM with 10 nM Hex-hR1 (R² 0.66); and <0.06 nM with 100 nM HexhR1 (R² 0.63). (**C**) Combination of temsirolimus with 1R-2b (CI = 0.02). The IC₅₀ values were 7.76 nM for Tem alone (R² 0.94); <0.06 nM with 26 nM 1R-2b (R² 0.32); <0.06 nM with 2.6 nM 1R-2b (R² 0.34); and 12.7 nM with 0.26 nM 1R-2b (R² 0.81).

### DETAILED DESCRIPTION

### Definitions

Unless otherwise specified, "a" or "an" means "one or more".

As used herein, the term "about" means plus or minus ten percent (10%) of a value. For example, "about 100" would refer to any number between 90 and 110.

A "therapeutic agent" is an atom, molecule, or compound that is useful in the treatment of a disease. Examples of therapeutic agents include antibodies, antibody fragments, peptides, drugs, toxins, enzymes, nucleases, hormones, immunomodulators, antisense oligonucleotides, small interfering RNA (siRNA), chelators, boron compounds, photoactive agents, oligonucleotides (e.g., RNAi or siRNA) and radioisotopes.

A "diagnostic agent" is an atom, molecule, or compound that is useful in diagnosing a disease. Useful diagnostic agents include, but are not limited to, radioisotopes, dyes (such as with the biotin-streptavidin complex), contrast agents, fluorescent compounds or molecules, and enhancing agents (e.g., paramagnetic ions) for magnetic resonance imaging (MRI).

An "antibody" as used herein refers to a full-length (i.e., naturally occurring or formed by normal immunoglobulin gene fragment recombinatorial processes) immunoglobulin molecule (*e.g*., an IgG antibody) or an immunologically active (i.e., specifically binding) portion of an immunoglobulin molecule, like an antibody fragment. An "antibody" includes monoclonal, polyclonal, bispecific, multispecific, murine, chimeric, humanized and human antibodies.

A "naked antibody" is an antibody or antigen binding fragment thereof that is not attached to a therapeutic or diagnostic agent. The Fc portion of an intact naked antibody can provide effector functions, such as complement fixation and ADCC (see, *e.g*., Markrides, Pharmacol Rev 50:59-87, 1998). Other mechanisms by which naked antibodies induce cell death may include apoptosis. (Vaswani and Hamilton, Ann Allergy Asthma Immunol 81: 105-119, 1998.)

An "antibody fragment" is a portion of an intact antibody such as F(ab')₂, F(ab)₂, Fab', Fab, Fv, sFv, scFv, dAb and the like. Regardless of structure, an antibody fragment binds with the same antigen that is recognized by the full-length antibody. For example, antibody fragments include isolated fragments consisting of the variable regions, such as the "Fv" fragments consisting of the variable regions of the heavy and light chains or recombinant single chain polypeptide molecules in which light and heavy variable regions are connected by a peptide linker ("scFv proteins"). "Single-chain antibodies", often abbreviated as "scFv" consist of a polypeptide chain that comprises both a V_{H} and a V_{L} domain which interact to form an antigen- binding site. The V_{H} and V_{L} domains are usually linked by a peptide of 1 to 25 amino acid residues. Antibody fragments also include diabodies, triabodies and single domain antibodies (dAb).

A "chimeric antibody" is a recombinant protein that contains the variable domains including the complementarity determining regions (CDRs) of an antibody derived from one species, preferably a rodent antibody, while the constant domains of the antibody molecule are derived from those of a human antibody. For veterinary applications, the constant domains of the chimeric antibody may be derived from that of other species, such as a cat or dog.

A "humanized antibody" is a recombinant protein in which the CDRs from an antibody from one species; e.g., a rodent antibody, are transferred from the heavy and light variable chains of the rodent antibody into human heavy and light variable domains. Additional FR amino acid substitutions from the parent, e.g. murine, antibody may be made. The constant domains of the antibody molecule are derived from those of a human antibody.

A "human antibody" is an antibody obtained from transgenic mice that have been genetically engineered to produce human antibodies in response to antigenic challenge. In this technique, elements of the human heavy and light chain locus are introduced into strains of mice derived from embryonic stem cell lines that contain targeted disruptions of the endogenous heavy chain and light chain loci. The transgenic mice can synthesize human antibodies specific for human antigens, and the mice can be used to produce human antibody-secreting hybridomas. Methods for obtaining human antibodies from transgenic mice are described by Green et al., Nature Genet. 7:13 (1994), Lonberg et al., Nature 368:856 (1994), and Taylor et al., Int. Immun. 6:579 (1994). A fully human antibody also can be constructed by genetic or chromosomal transfection methods, as well as phage display technology, all of which are known in the art. (See, e.g., McCafferty et al., Nature 348:552-553 (1990) for the production of human antibodies and fragments thereof in vitro, from immunoglobulin variable domain gene repertoires from unimmunized donors). Human antibodies may also be generated by *in vitro* activated B cells. (See, U.S. Pat. Nos. 5,567,610 and 5,229,275).

### Antibodies and Antibody Fragments

Techniques for preparing monoclonal antibodies against virtually any target antigen are well known in the art. *See,* for example, Kohler and Milstein, Nature 256: 495 (1975), and Coligan et al. (eds.), CURRENT PROTOCOLS IN IMMUNOLOGY, VOL. 1, pages 2.5.1-2.6.7 (John Wiley & Sons 1991). Briefly, monoclonal antibodies can be obtained by injecting mice with a composition comprising an antigen, removing the spleen to obtain B-lymphocytes, fusing the B-lymphocytes with myeloma cells to produce hybridomas, cloning the hybridomas, selecting positive clones which produce antibodies to the antigen, culturing the clones that produce antibodies to the antigen, and isolating the antibodies from the hybridoma cultures.

Antibodies can be isolated and purified from hybridoma cultures by a variety of well-established techniques. Such isolation techniques include affinity chromatography with Protein-A Sepharose, size-exclusion chromatography, and ion-exchange chromatography. See, for example, Coligan at pages 2.7.1-2.7.12 and pages 2.9.1-2.9.3. Also, see Baines et al., "Purification of Immunoglobulin G (IgG)," in METHODS IN MOLECULAR BIOLOGY, VOL. 10, pages 79-104 (The Humana Press, Inc. 1992).

After the initial raising of antibodies to the immunogen, the antibodies can be sequenced and subsequently prepared by recombinant techniques. Humanization and chimerization of murine antibodies and antibody fragments are well known to those skilled in the art. The use of antibody components derived from humanized, chimeric or human antibodies obviates potential problems associated with the immunogenicity of murine constant regions.

### Chimeric Antibodies

A chimeric antibody is a recombinant protein in which the variable regions of a human antibody have been replaced by the variable regions of, for example, a mouse antibody, including the complementarity-determining regions (CDRs) of the mouse antibody. Chimeric antibodies exhibit decreased immunogenicity and increased stability when administered to a subject. General techniques for cloning murine immunoglobulin variable domains are disclosed, for example, in Orlandi et al., Proc. Nat'l Acad. Sci. USA 86: 3833 (1989). Techniques for constructing chimeric antibodies are well known to those of skill in the art. As an example, Leung et al., Hybridoma 13:469 (1994), produced an LL2 chimera by combining DNA sequences encoding the V_{κ} and V_{H} domains of murine LL2, an anti-CD22 monoclonal antibody, with respective human κ and IgG₁ constant region domains.

### Humanized Antibodies

Techniques for producing humanized antibodies are well known in the art (see, e.g., Jones et al., Nature 321: 522 (1986), Riechmann et al., Nature 332: 323 (1988), Verhoeyen et al., Science 239: 1534 (1988), Carter et al., Proc. Nat'l Acad. Sci. USA 89: 4285 (1992), Sandhu, Crit. Rev. Biotech. 12: 437 (1992), and Singer et al., J. Immun. 150: 2844 (1993)). A chimeric or murine monoclonal antibody may be humanized by transferring the mouse CDRs from the heavy and light variable chains of the mouse immunoglobulin into the corresponding variable domains of a human antibody. The mouse framework regions (FR) in the chimeric monoclonal antibody are also replaced with human FR sequences. As simply transferring mouse CDRs into human FRs often results in a reduction or even loss of antibody affinity, additional modification might be required in order to restore the original affinity of the murine antibody. This can be accomplished by the replacement of one or more human residues in the FR regions with their murine counterparts to obtain an antibody that possesses good binding affinity to its epitope. See, for example, Tempest et al., Biotechnology 9:266 (1991) and Verhoeyen et al., Science 239: 1534 (1988). Generally, those human FR amino acid residues that differ from their murine counterparts and are located close to or touching one or more CDR amino acid residues would be candidates for substitution.

### Human Antibodies

Methods for producing fully human antibodies using either combinatorial approaches or transgenic animals transformed with human immunoglobulin loci are known in the art (*e.g.,* Mancini et al., 2004, New Microbiol. 27:315-28; Conrad and Scheller, 2005, Comb. Chem. High Throughput Screen. 8:117-26; Brekke and Loset, 2003, Curr. Opin. Phamacol. 3:544-50). A fully human antibody also can be constructed by genetic or chromosomal transfection methods, as well as phage display technology, all of which are known in the art. See for example, McCafferty et al., Nature 348:552-553 (1990). Such fully human antibodies are expected to exhibit even fewer side effects than chimeric or humanized antibodies and to function *in vivo* as essentially endogenous human antibodies. In certain embodiments, the invention as defined in the claims may utilize human antibodies produced by such techniques.

In one alternative, the phage display technique may be used to generate human antibodies (*e.g.,* Dantas-Barbosa et al., 2005, Genet. Mol. Res. 4:126-40). Human antibodies may be generated from normal humans or from humans that exhibit a particular disease state, such as cancer (Dantas-Barbosa et al., 2005). The advantage to constructing human antibodies from a diseased individual is that the circulating antibody repertoire may be biased towards antibodies against disease-associated antigens.

In one non-limiting example of this methodology, Dantas-Barbosa et al. (2005) constructed a phage display library of human Fab antibody fragments from osteosarcoma patients. Generally, total RNA was obtained from circulating blood lymphocytes (*Id.*). Recombinant Fab were cloned from the µ, γ and κ chain antibody repertoires and inserted into a phage display library (*Id*.). RNAs were converted to cDNAs and used to make Fab cDNA libraries using specific primers against the heavy and light chain immunoglobulin sequences (Marks et al., 1991, J. Mol. Biol. 222:581-97). Library construction was performed according to Andris-Widhopf et al. (2000, In: Phage Display Laboratory Manual, Barbas et al. (eds), 1st edition, Cold Spring Harbor Laboratory Press, Cold Spring Harbor, NY pp. 9.1 to 9.22). The final Fab fragments were digested with restriction endonucleases and inserted into the bacteriophage genome to make the phage display library. Such libraries may be screened by standard phage display methods, as known in the art (see, e.g., Pasqualini and Ruoslahti, 1996, Nature 380:364-366; Pasqualini, 1999, The Quart. J. Nucl. Med. 43:159-162).

Phage display can be performed in a variety of formats, for their review, see e.g. Johnson and Chiswell, Current Opinion in Structural Biology 3:5564-571 (1993). Human antibodies may also be generated by *in vitro* activated B cells. See U.S. Patent Nos. 5,567,610 and 5,229,275. The skilled artisan will realize that these techniques are exemplary and any known method for making and screening human antibodies or antibody fragments may be utilized.

In another alternative, transgenic animals that have been genetically engineered to produce human antibodies may be used to generate antibodies against essentially any immunogenic target, using standard immunization protocols. Methods for obtaining human antibodies from transgenic mice are disclosed by Green et al., Nature Genet. 7:13 (1994), Lonberg et al., Nature 368:856 (1994), and Taylor et al., Int. Immun. 6:579 (1994). A non-limiting example of such a system is the XenoMouse® (*e.g.,* Green et al., 1999, J. Immunol. Methods 231:11-23) from Abgenix (Fremont, CA). In the XenoMouse® and similar animals, the mouse antibody genes have been inactivated and replaced by functional human antibody genes, while the remainder of the mouse immune system remains intact.

The XenoMouse® was transformed with germline-configured YACs (yeast artificial chromosomes) that contained portions of the human IgH and Igkappa loci, including the majority of the variable region sequences, along accessory genes and regulatory sequences. The human variable region repertoire may be used to generate antibody producing B cells, which may be processed into hybridomas by known techniques. A XenoMouse® immunized with a target antigen will produce human antibodies by the normal immune response, which may be harvested and/or produced by standard techniques discussed above. A variety of strains of XenoMouse® are available, each of which is capable of producing a different class of antibody. Transgenically produced human antibodies have been shown to have therapeutic potential, while retaining the pharmacokinetic properties of normal human antibodies (Green et al., 1999). The skilled artisan will realize that the claimed compositions and methods are not limited to use of the XenoMouse® system but may utilize any transgenic animal that has been genetically engineered to produce human antibodies.

### Antibody Fragments

Antibody fragments which recognize specific epitopes can be generated by known techniques. Antibody fragments are antigen binding portions of an antibody, such as F(ab')₂. Fab', F(ab)₂, Fab, Fv, sFv and the like. F(ab')₂ fragments can be produced by pepsin digestion of the antibody molecule and Fab' fragments can be generated by reducing disulfide bridges of the F(ab')₂ fragments. Alternatively, Fab' expression libraries can be constructed (Huse et al., 1989, Science, 246:1274-1281) to allow rapid and easy identification of monoclonal Fab' fragments with the desired specificity. F(ab)₂ fragments may be generated by papain digestion of an antibody.

A single chain Fv molecule (scFv) comprises a VL domain and a VH domain. The VL and VH domains associate to form a target binding site. These two domains are further covalently linked by a peptide linker (L). Methods for making scFv molecules and designing suitable peptide linkers are described in US Patent No. 4,704,692, US Patent No. 4,946,778, R. Raag and M. Whitlow, "Single Chain Fvs." FASEB Vol 9:73-80 (1995) and R.E. Bird and B.W. Walker, "Single Chain Antibody Variable Regions," TIBTECH, Vol 9: 132-137 (1991).

Techniques for producing single domain antibodies (DABs) are also known in the art, as disclosed for example in Cossins et al. (2006, Prot Express Purif 51:253-259).

An antibody fragment can be prepared by proteolytic hydrolysis of the full length antibody or by expression in *E. coli* or another host of the DNA coding for the fragment. An antibody fragment can be obtained by pepsin or papain digestion of full length antibodies by conventional methods. These methods are described, for example, by Goldenberg, U.S. Patent Nos. 4,036,945 and 4,331,647 and references contained therein. Also, see Nisonoff et al., Arch Biochem. Biophys. 89: 230 (1960); Porter, Biochem. J. 73: 119 (1959), Edelman et al., in METHODS IN ENZYMOLOGY VOL. 1, page 422 (Academic Press 1967), and Coligan at pages 2.8.1-2.8.10 and 2.10.-2.10.4.

### Known Antibodies

Antibodies of use may be commercially obtained from a wide variety of known sources. For example, a variety of antibody secreting hybridoma lines are available from the American Type Culture Collection (ATCC, Manassas, VA). A large number of antibodies against various disease targets, including but not limited to tumor-associated antigens, have been deposited at the ATCC and/or have published variable region sequences and are available for use in the claimed methods and compositions. See, e.g., U.S. Patent Nos. 7,312,318; 7,282,567; 7,151,164; 7,074,403; 7,060,802; 7,056,509; 7,049,060; 7,045,132; 7,041,803; 7,041,802; 7,041,293; 7,038,018; 7,037,498; 7,012,133; 7,001,598; 6,998,468; 6,994,976; 6,994,852; 6,989,241; 6,974,863; 6,965,018; 6,964,854; 6,962,981; 6,962,813; 6,956,107; 6,951,924; 6,949,244; 6,946,129; 6,943,020; 6,939,547; 6,921,645; 6,921,645; 6,921,533; 6,919,433; 6,919,078; 6,916,475; 6,905,681; 6,899,879; 6,893,625; 6,887,468; 6,887,466; 6,884,594; 6,881,405; 6,878,812; 6,875,580; 6,872,568; 6,867,006; 6,864,062; 6,861,511; 6,861,227; 6,861,226; 6,838,282; 6,835,549; 6,835,370; 6,824,780; 6,824,778; 6,812,206; 6,793,924; 6,783,758; 6,770,450; 6,767,711; 6,764,688; 6,764,681; 6,764,679; 6,743,898; 6,733,981; 6,730,307; 6,720,155; 6,716,966; 6,709,653; 6,693,176; 6,692,908; 6,689,607; 6,689,362; 6,689,355; 6,682,737; 6,682,736; 6,682,734; 6,673,344; 6,653,104; 6,652,852; 6,635,482; 6,630,144; 6,610,833; 6,610,294; 6,605,441; 6,605,279; 6,596,852; 6,592,868; 6,576,745; 6,572;856; 6,566,076; 6,562,618; 6,545,130; 6,544,749; 6,534,058; 6,528,625; 6,528,269; 6,521,227; 6,518,404; 6,511,665; 6,491,915; 6,488,930; 6,482,598; 6,482,408; 6,479,247; 6,468,531; 6,468,529; 6,465,173; 6,461,823; 6,458,356; 6,455,044; 6,455,040; 6,451,310; 6,444,206; 6,441,143; 6,432,404; 6,432,402; 6,419,928; 6,413,726; 6,406,694; 6,403,770; 6,403,091; 6,395,276; 6,395,274; 6,387,350; 6,383,759; 6,383,484; 6,376,654; 6,372,215; 6,359,126; 6,355,481; 6,355,444; 6,355,245; 6,355,244; 6,346,246; 6,344,198; 6,340,571; 6,340,459; 6,331,175; 6,306,393; 6,254,868; 6,187,287; 6,183,744; 6,129,914; 6,120,767; 6,096,289; 6,077,499; 5,922,302; 5,874,540; 5,814,440; 5,798,229; 5,789,554; 5,776,456; 5,736,119; 5,716,595; 5,677,136; 5,587,459; 5,443,953, 5,525,338. These are exemplary only and a wide variety of other antibodies and their hybridomas are known in the art. The skilled artisan will realize that antibody sequences or antibody-secreting hybridomas against almost any disease-associated antigen may be obtained by a simple search of the ATCC, NCBI and/or USPTO databases for antibodies against a selected disease-associated target of interest. The antigen binding domains of the cloned antibodies may be amplified, excised, ligated into an expression vector, transfected into an adapted host cell and used for protein production, using standard techniques well known in the art (see, e.g., U.S. Patent Nos. 7,531,327; 7,537,930; 7,608,425 and 7,785,880). Such known antibodies may be used in combination with one or more anti-IGF-1R antibodies, either as part of a complex, or administered separately or together with an anti-IGF- 1R antibody.

Particular antibodies that may be of use for therapy of cancer within the scope of the invention as defined in the claims include, but are not limited to, LL1 (anti-CD74), LL2 and RFB4 (anti-CD22), RS7 (anti-epithelial glycoprotein-1 (EGP-1)), PAM4 and KC4 (both anti-mucin), MN-14 (anti-carcinoembryonic antigen (CEA, also known as CD66e), Mu-9 (anti-colon-specific antigen-p), Immu 31 (an anti-alpha-fetoprotein), TAG-72 (e.g., CC49), Tn, J591 or HuJ591 (anti-PSMA (prostate-specific membrane antigen)), AB-PG1-XG1-026 (anti-PSMA dimer), D2/B (anti-PSMA), G250 (anti-carbonic anhydrase IX), hL243 (anti-HLA-DR), alemtuzumab (anti-CD52), bevacizumab (anti-VEGF), cetuxiamab (anti-EGFR), gemtuzumab (anti-CD33), ibritumomab tiuxetan (anti-CD20); panitumumab (anti-EGFR); rituximab (anti-CD20); tositumomab (anti-CD20); GA101 (anti-CD20); and trastuzumab (anti-ErbB2). Such antibodies are known in the art (e.g., U.S. Patent Nos. 5,686,072; 5,874,540; 6,107,090; 6,183,744; 6,306,393; 6,653,104; 6,730.300; 6,899,864; 6,926,893; 6,962,702; 7,074,403; 7,230,084; 7,238,785; 7,238,786; 7,256,004; 7,282,567; 7,300,655; 7,312,318; 7,585,491; 7,612,180; 7,642,239; and U.S. Patent Application Publ. No. 20040202666 (now abandoned); 20050271671; and 20060193865.) Specific known antibodies of use include hPAM4 (U.S. Patent No. 7,282,567), hA20 (U.S. Patent No. 7,251,164), hA19 (U.S. Patent No. 7,109,304), hIMMU31 (U.S. Patent No. 7,300,655), hLL1 (U.S. Patent No. 7,312,318,), hLL2 (U.S. Patent No. 7,074,403), hMu-9 (U.S. Patent No. 7,387,773), hL243 (U.S. Patent No. 7,612,180), hMN-14 (U.S. Patent No. 6,676,924), hMN-15 (U.S. Patent No. 7,541,440), hR1 (U.S. Patent Application 12/772,645), hRS7 (U.S. Patent No. 7,238,785), hMN-3 (U.S. Patent No. 7,541,440), AB-PG1-XG1-026 (U.S. Patent Application 11/983,372, deposited as ATCC PTA-4405 and PTA-4406) and D2/B (WO 2009/130575).

Anti-TNF-α antibodies are known in the art and may be of use to treat immune diseases, such as autoimmune disease, immune dysfunction (e.g., graft-versus-host disease, organ transplant rejection) or diabetes. Known antibodies against TNF-α include the human antibody CDP571 (Ofei et al., 2011, Diabetes 45:881-85); murine antibodies MTNFAI, M2TNFAI, M3TNFAI, M3TNFABI, M302B and M303 (Thermo Scientific, Rockford, IL); infliximab (Centocor, Malvern, PA); certolizumab pegol (UCB, Brussels, Belgium); and adalimumab (Abbott, Abbott Park, IL). These and many other known anti-TNF-α antibodies may be used in the invention as defined in the claims. Other antibodies of use for therapy of immune dysregulatory or autoimmune disease include, but are not limited to, anti-B-cell antibodies such as veltuzumab, epratuzumab, milatuzumab or hL243; tocilizumab (anti-IL-6 receptor); basiliximab (anti-CD25); daclizumab (anti-CD25); efalizumab (anti-CD11a); muromonab-CD3 (anti-CD3 receptor); anti-CD40L (UCB, Brussels, Belgium); natalizumab (anti-α4 integrin) and omalizumab (anti-IgE). While anti-IGF-1R antibodies have primarily been addressed to cancer therapy to date, there are indications that IGF-1R may also be involved in immune system function and autoimmune diseases (see, e.g., Smith, 2010, Pharm Rev 62:199-236).

Type-1 and Type-2 diabetes may be treated using known antibodies against B-cell antigens, such as CD22 (epratuzumab), CD74 (milatuzumab), CD19 (hA19), CD20 (veltuzumab) or HLA-DR (hL243) (*see, e.g*., Winer et al., 2011, Nature Med 17:610-18). Anti-CD3 antibodies also have been proposed for therapy of type 1 diabetes (Cernea et al., 2010, Diabetes Metab Rev 26:602-05).

Macrophage migration inhibitory factor (MIF) is an important regulator of innate and adaptive immunity and apoptosis. It has been reported that CD74 is the endogenous receptor for MIF (Leng et al., 2003, J Exp Med 197:1467-76). The therapeutic effect of antagonistic anti-CD74 antibodies on MIF-mediated intracellular pathways may be of use for treatment of a broad range of disease states, such as cancers of the bladder, prostate, breast, lung, colon and chronic lymphocytic leukemia (e.g., Meyer-Siegler et al., 2004, BMC Cancer 12:34; Shachar & Haran, 2011, Leuk Lymphoma 52:1446-54); autoimmune diseases such as rheumatoid arthritis and systemic lupus erythematosus (Morand & Leech, 2005, Front Biosci 10:12-22; Shachar & Haran, 2011, Leuk Lymphoma 52:1446-54); kidney diseases such as renal allograft rejection (Lan, 2008, Nephron Exp Nephrol. 109:e79-83); and numerous inflammatory diseases (Meyer-Siegler et al., 2009, Mediators Inflamm epub March 22, 2009; Takahashi et al., 2009, Respir Res 10:33; Milatuzumab (hLL1) is an exemplary anti-CD74 antibody of therapeutic use for treatment of MIF-mediated diseases.

Bapineuzumab is in clinical trials for Alzheimer's disease therapy. Other antibodies proposed for therapy of Alzheimer's disease include Alz 50 (Ksiezak-Reding et al., 1987, J Biol Chem 263:7943-47), gantenerumab, and solanezumab. Infliximab, an anti-TNF-α antibody, has been reported to reduce amyloid plaques and improve cognition.

Antibodies to fibrin (e.g., scFv(59D8); T2Gls; MH1) are known and in clinical trials as imaging agents for disclosing said clots and pulmonary emboli, while anti-granulocyte antibodies, such as MN-3, MN-15, anti-NCA95, and anti-CD15 antibodies, can target myocardial infarcts and myocardial ischemia. (See, e.g., U.S. Patent Nos. 5,487,892; 5,632,968; 6,294,173; 7,541,440) Anti-macrophage, anti-low-density lipoprotein (LDL), anti-MIF, and anti-CD74 (e.g., hLL1) antibodies can be used to target atherosclerotic plaques. Abciximab (anti-glycoprotein IIb/IIIa) has been approved for adjuvant use for prevention of restenosis in percutaneous coronary interventions and the treatment of unstable angina (Waldmann et al., 2000, Hematol 1:394-408). Anti-CD3 antibodies have been reported to reduce development and progression of atherosclerosis (Steffens et al., 2006, Circulation 114:1977-84). Antibodies against oxidized LDL induced a regression of established atherosclerosis in a mouse model (Ginsberg, 2007, J Am Coll Cardiol 52:2319-21). Anti-ICAM-1 antibody was shown to reduce ischemic cell damage after cerebral artery occlusion in rats (Zhang et al., 1994, Neurology 44:1747-51). Commercially available monoclonal antibodies to leukocyte antigens are represented by: OKT anti-T-cell monoclonal antibodies (available from Ortho Pharmaceutical Company) which bind to normal T-lymphocytes; the monoclonal antibodies produced by the hybridomas having the ATCC accession numbers HB44, HB55, HB12, HB78 and HB2; G7E11, W8E7, NKP15 and GO22 (Becton Dickinson); NEN9.4 (New England Nuclear); and FMC11 (Sera Labs). A description of antibodies against fibrin and platelet antigens is contained in Knight, Semin. Nucl. Med., 20:52-67 (1990).

### Antibody Allotypes

Immunogenicity of therapeutic antibodies is associated with increased risk of infusion reactions and decreased duration of therapeutic response (Baert et al., 2003, N Engl J Med 348:602-08). The extent to which therapeutic antibodies induce an immune response in the host may be determined in part by the allotype of the antibody (Stickler et al., 2011, Genes and Immunity 12:213-21). Antibody allotype is related to amino acid sequence variations at specific locations in the constant region sequences of the antibody. The allotypes of IgG antibodies containing a heavy chain γ-type constant region are designated as Gm allotypes (1976, J Immunol 117:1056-59).

For the common IgG1 human antibodies, the most prevalent allotype is G1m1 (Stickler et al., 2011, Genes and Immunity 12:213-21). However, the G1m3 allotype also occurs frequently in Caucasians (*Id.*)*.* It has been reported that G1m1 antibodies contain allotypic sequences that tend to induce an immune response when administered to non-Glml (nG1m1) recipients, such as G1m3 patients (*Id*.). Non-Glml allotype antibodies are not as immunogenic when administered to G1m1 patients (*Id.*)*.*

The human G1m1 allotype comprises the amino acids aspartic acid at Kabat position 356 and leucine at Kabat position 358 in the CH3 sequence of the heavy chain IgG1. The nG1m1 allotype comprises the amino acids glutamic acid at Kabat position 356 and methionine at Kabat position 358. Both G1ml and nG1ml allotypes comprise a glutamic acid residue at Kabat position 357 and the allotypes are sometimes referred to as DEL and EEM allotypes. A non-limiting example of the heavy chain constant region sequences for G1m1 and nG1m1 allotype antibodies is shown for the exemplary antibodies rituximab (SEQ ID NO:120) and veltuzumab (SEQ ID NO:121).

### Rituimab heavy chain variable region sequence (SEQ ID NO:120)

### Veltuzumab heavy chain variable region (SEQ ID NO:121)

Jefferis and Lefranc (2009, mAbs 1:1-7) reviewed sequence variations characteristic of IgG allotypes and their effect on immunogenicity. They reported that the G1m3 allotype is characterized by an arginine residue at Kabat position 214, compared to a lysine residue at Kabat 214 in the G1m17 allotype. The nG1m1,2 allotype was characterized by glutamic acid at Kabat position 356, methionine at Kabat position 358 and alanine at Kabat position 431. The G1m1,2 allotype was characterized by aspartic acid at Kabat position 356, leucine at Kabat position 358 and glycine at Kabat position 431. In addition to heavy chain constant region sequence variants, Jefferis and Lefranc (2009) reported allotypic variants in the kappa light chain constant region, with the Km1 allotype characterized by valine at Kabat position 153 and leucine at Kabat position 191, the Km1,2 allotype by alanine at Kabat position 153 and leucine at Kabat position 191, and the Km3 allotypoe characterized by alanine at Kabat position 153 and valine at Kabat position 191.

With regard to therapeutic antibodies, veltuzumab and rituximab are, respectively, humanized and chimeric IgG1 antibodies against CD20, of use for therapy of a wide variety of hematological malignancies and/or autoimmune diseases. **Table 1** compares the allotype sequences of rituximab vs. veltuzumab. As shown in **Table 1,** rituximab (G1m17,1) is a DEL allotype IgG1, with an additional sequence variation at Kabat position 214 (heavy chain CH1) of lysine in rituximab vs. arginine in veltuzumab. It has been reported that veltuzumab is less immunogenic in subjects than rituximab (*see, e.g.,* Morchhauser et al., 2009, J Clin Oncol 27:3346-53; Goldenberg et al., 2009, Blood 113:1062-70; Robak & Robak, 2011, BioDrugs 25:13-25), an effect that has been attributed to the difference between humanized and chimeric antibodies. However, the difference in allotypes between the EEM and DEL allotypes likely also accounts for the lower immunogenicity of veltuzumab.

**Table 1. Allotypes of Rituximab vs. Veltuzumab**

| | | *Heavy chain position and associated allotypes* | | | | | |
|---|---|---|---|---|---|---|---|
| | *Complete allotype* | *214 (allotype)* | | *356*/*358 (allotype)* | | *431 (allotype)* | |
| *Rituximab* | *G1m17,1* | *K* | *17* | *D*/*L* | *1* | *A* | - |
| *Veltuzumab* | *G1m3* | *R* | *3* | *E*/*M* | - | *A* | - |

In order to reduce the immunogenicity of therapeutic antibodies in individuals of nG1m1 genotype, it is desirable to select the allotype of the antibody to correspond to the G1m3 allotype, characterized by arginine at Kabat 214, and the nG1m1,2 null-allotype, characterized by glutamic acid at Kabat position 356, methionine at Kabat position 358 and alanine at Kabat position 431. Surprisingly, it was found that repeated subcutaneous administration of G1m3 antibodies over a long period of time did not result in a significant immune response. In alternative embodiments, the human IgG4 heavy chain in common with the G1m3 allotype has arginine at Kabat 214, glutamic acid at Kabat 356, methionine at Kabat 359 and alanine at Kabat 431. Since immunogenicity appears to relate at least in part to the residues at those locations, use of the human IgG4 heavy chain constant region sequence for therapeutic antibodies is also a preferred embodiment Combinations of G1m3 IgG1 antibodies with IgG4 antibodies may also be of use for therapeutic administration.

### Immunoconjugates

In certain embodiments of the invention as defined in the claims, the antibodies or fragments thereof may be conjugated to one or more therapeutic or diagnostic agents. The therapeutic agents do not need to be the same but can be different, e.g. a drug and a radioisotope. For example, ¹³¹I can be incorporated into a tyrosine of an antibody or fusion protein and a drug attached to an epsilon amino group of a lysine residue. Therapeutic and diagnostic agents also can be attached, for example to reduced SH groups and/or to carbohydrate side chains. Many methods for making covalent or non-covalent conjugates of therapeutic or diagnostic agents with antibodies or fusion proteins are known in the art and any such known method may be utilized.

A therapeutic or diagnostic agent can be attached at the hinge region of a reduced antibody component via disulfide bond formation. Alternatively, such agents can be attached using a heterobifunctional cross-linker, such as *N*-succinyl 3-(2-pyridyldithio)propionate (SPDP). Yu et al., Int. J. Cancer 56: 244 (1994). General techniques for such conjugation are well-known in the art. See, for example, Wong, CHEMISTRY OF PROTEIN CONJUGATION AND CROSS-LINKING (CRC Press 1991); Upeslacis et al., "Modification of Antibodies by Chemical Methods," in MONOCLONAL ANTIBODIES: PRINCIPLES AND APPLICATIONS, Birch et al. (eds.), pages 187-230 (Wiley-Liss, Inc. 1995); Price, "Production and Characterization of Synthetic Peptide-Derived Antibodies," in MONOCLONAL ANTIBODIES: PRODUCTION, ENGINEERING AND CLINICAL APPLICATION, Ritter et al. (eds.), pages 60-84 (Cambridge University Press 1995). Alternatively, the therapeutic or diagnostic agent can be conjugated via a carbohydrate moiety in the Fc region of the antibody. The carbohydrate group can be used to increase the loading of the same agent that is bound to a thiol group, or the carbohydrate moiety can be used to bind a different therapeutic or diagnostic agent.

Methods for conjugating peptides to antibody components via an antibody carbohydrate moiety are well-known to those of skill in the art. See, for example, Shih et al., Int. J. Cancer 41: 832 (1988); Shih et al., Int. J. Cancer 46: 1101 (1990); and Shih et al., U.S. Patent No. 5,057,313. The general method involves reacting an antibody component having an oxidized carbohydrate portion with a carrier polymer that has at least one free amine function. This reaction results in an initial Schiff base (imine) linkage, which can be stabilized by reduction to a secondary amine to form the final conjugate.

The Fc region may be absent if the antibody used as the antibody component of the immunoconjugate is an antibody fragment. However, it is possible to introduce a carbohydrate moiety into the light chain variable region of a full length antibody or antibody fragment. See, for example, Leung et al., J. Immunol. 154: 5919 (1995); Hansen et al., U.S. Patent No. 5,443,953 (1995), Leung et al., U.S. patent No. 6,254,868. The engineered carbohydrate moiety is used to attach the therapeutic or diagnostic agent.

An alternative method for attaching toxins or other functional groups to antibodies or complexes thereof involves use of click chemistry reactions. The click chemistry approach was originally conceived as a method to rapidly generate complex substances by joining small subunits together in a modular fashion. (See, e.g., Kolb et al., 2004, Angew Chem Int Ed 40:3004-31; Evans, 2007, Aust J Chem 60:384-95.) Various forms of click chemistry reaction are known in the art, such as the Huisgen 1,3-dipolar cycloaddition copper catalyzed reaction (Tornoe et al., 2002, J Organic Chem 67:3057-64), which is often referred to as the "click reaction." Other alternatives include cycloaddition reactions such as the Diels-Alder, nucleophilic substitution reactions (especially to small strained rings like epoxy and aziridine compounds), carbonyl chemistry formation of urea compounds and reactions involving carbon-carbon double bonds, such as alkynes in thiol-yne reactions.

The azide alkyne Huisgen cycloaddition reaction uses a copper catalyst in the presence of a reducing agent to catalyze the reaction of a terminal alkyne group attached to a first molecule. In the presence of a second molecule comprising an azide moiety, the azide reacts with the activated alkyne to form a 1,4-disubstituted 1,2,3-triazole. The copper catalyzed reaction occurs at room temperature and is sufficiently specific that purification of the reaction product is often not required. (Rostovstev et al., 2002, Angew Chem Int Ed 41:2596; Tornoe et al., 2002, J Org Chem 67:3057.) The azide and alkyne functional groups are largely inert towards biomolecules in aqueous medium, allowing the reaction to occur in complex solutions. The triazole formed is chemically stable and is not subject to enzymatic cleavage, making the click chemistry product highly stable in biological systems. Although the copper catalyst is toxic to living cells, the copper-based click chemistry reaction may be used *in vitro* for immunoconjugate formation.

A copper-free click reaction has been proposed for covalent modification of biomolecules. (See, e.g., Agard et al., 2004, J Am Chem Soc 126:15046-47.) The copper-free reaction uses ring strain in place of the copper catalyst to promote a [3 + 2] azide-alkyne cycloaddition reaction (*Id*.) For example, cyclooctyne is a 8-carbon ring structure comprising an internal alkyne bond. The closed ring structure induces a substantial bond angle deformation of the acetylene, which is highly reactive with azide groups to form a triazole. Thus, cyclooctyne derivatives may be used for copper-free click reactions (*Id*.)

Another type of copper-free click reaction was reported by Ning et al. (2010, Angew Chem Int Ed 49:3065-68), involving strain-promoted alkyne-nitrone cycloaddition. To address the slow rate of the original cyclooctyne reaction, electron-withdrawing groups are attached adjacent to the triple bond (*Id*.) Examples of such substituted cyclooctynes include difluorinated cyclooctynes, 4-dibenzocyclooctynol and azacyclooctyne *(Id.)* An alternative copper-free reaction involved strain-promoted akyne-nitrone cycloaddition to give N-alkylated isoxazolines (*Id*.) The reaction was reported to have exceptionally fast reaction kinetics and was used in a one-pot three-step protocol for site-specific modification of peptides and proteins *(Id.)* Nitrones were prepared by the condensation of appropriate aldehydes with *N*-methylhydroxylamine and the cycloaddition reaction took place in a mixture of acetonitrile and water *(Id.)*

### Bispecific and Multispecific Antibodies

Certain embodiments of the invention as defined in the claims may involve bispecific or even multispecific complexes comprising an anti-IGF-1R antibody or antibody fragment. Numerous methods to produce bispecific or multispecific antibodies are known, as disclosed, for example, in U.S. Patent No. 7,405,320. Bispecific antibodies can be produced by the quadroma method, which involves the fusion of two different hybridomas, each producing a monoclonal antibody recognizing a different antigenic site (Milstein and Cuello, Nature, 1983; 305:537-540).

Another method for producing bispecific antibodies uses heterobifunctional crosslinkers to chemically tether two different monoclonal antibodies (Staerz, et al. Nature. 1985; 314:628-631; Perez, et al. Nature. 1985; 316:354-356). Bispecific antibodies can also be produced by reduction of each of two parental monoclonal antibodies to the respective half molecules, which are then mixed and allowed to reoxidize to obtain the hybrid structure (Staerz and Bevan. Proc Natl Acad Sci U S A. 1986; 83:1453-1457). Another alternative involves chemically cross-linking two or three separately purified Fab' fragments using appropriate linkers. (See, e.g.,
European Patent Application 0453082).

Other methods include improving the efficiency of generating hybrid hybridomas by gene transfer of distinct selectable markers via retrovirus-derived shuttle vectors into respective parental hybridomas, which are fused subsequently (DeMonte, et al. Proc Natl Acad Sci U S A. 1990, 87:2941-2945); or transfection of a hybridoma cell line with expression plasmids containing the heavy and light chain genes of a different antibody.

Cognate V_{H} and V_{L} domains can be joined with a peptide linker of appropriate composition and length (usually consisting of more than 12 amino acid residues) to form a single-chain Fv (scFv) with binding activity. Methods of manufacturing scFvs are disclosed in U.S. Pat. No. 4,946,778 and U.S. Pat. No. 5,132,405. Reduction of the peptide linker length to less than 12 amino acid residues prevents pairing of V_{H} and V_{L} domains on the same chain and forces pairing of V_{H} and V_{L} domains with complementary domains on other chains, resulting in the formation of functional multimers. Polypeptide chains of V_{H} and V_{L} domains that are joined with linkers between 3 and 12 amino acid residues form predominantly dimers (termed diabodies). With linkers between 0 and 2 amino acid residues, trimers (termed triabody) and tetramers (termed tetrabody) are favored, but the exact patterns of oligomerization appear to depend on the composition as well as the orientation of V-domains (V_{H}-linker-V_{L} or V_{L}-linker-V_{H}), in addition to the linker length.

These techniques for producing multispecific or bispecific antibodies exhibit various difficulties in terms of low yield, necessity for purification, low stability or the labor-intensiveness of the technique. A more recent technique to produce DNL™ complexes, described in more detail below, has been utilized to produce combinations of virtually any desired antibodies, antibody fragments and other effector molecules. The technique allows the assembly of monospecific, bispecific or multispecific antibodies, either as naked antibody moieties or in combination with a wide range of other effector molecules such as immunomodulators, enzymes, chemotherapeutic agents, chemokines, cytokines, diagnostic agents, therapeutic agents, radionuclides, imaging agents, anti-angiogenic agents, growth factors, oligonucleotides, hormones, peptides, toxins, pro-apoptotic agents, or a combination thereof. Any of the techniques known in the art for making bispecific or multispecific antibodies may be utilized in the practice of the presently claimed methods.

### Affibodies and Fynomers

Certain alternative embodiments may utilize affibodies in place of antibodies. Affibodies are commercially available from Affibody AB (Solna, Sweden). Affibodies are small proteins that function as antibody mimetics and are of use in binding target molecules. Affibodies were developed by combinatorial engineering on an alpha helical protein scaffold (Nord et al., 1995, Protein Eng 8:601-8; Nord et al., 1997, Nat Biotechnol 15:772-77). The affibody design is based on a three helix bundle structure comprising the IgG binding domain of protein A (Nord et al., 1995; 1997). Affibodies with a wide range of binding affinities may be produced by randomization of thirteen amino acids involved in the Fc binding activity of the bacterial protein A (Nord et al., 1995; 1997). After randomization, the PCR amplified library was cloned into a phagemid vector for screening by phage display of the mutant proteins. The phage display library may be screened against any known antigen, using standard phage display screening techniques (e.g., Pasqualini and Ruoslahti, 1996, Nature 380:364-366; Pasqualini, 1999, Quart. J. Nucl. Med. 43:159-162), in order to identify one or more affibodies against the target antigen.

A ¹⁷⁷Lu-labeled affibody specific for HER2/neu has been demonstrated to target HER2-expressing xenografts *in vivo* (Tolmachev et al., 2007, Cancer Res 67:2773-82). Although renal toxicity due to accumulation of the low molecular weight radiolabeled compound was initially a problem, reversible binding to albumin reduced renal accumulation, enabling radionuclide-based therapy with labeled affibody (*Id.*)*.*

The feasibility of using radiolabeled affibodies for *in vivo* tumor imaging has been recently demonstrated (Tolmachev et al., 2011, Bioconjugate Chem 22:894-902). A maleimide-derivatized NOTA was conjugated to the anti-HER2 affibody and radiolabeled with ¹¹¹In (*Id.*)*.* Administration to mice bearing the HER2-expressing DU-145 xenograft, followed by gamma camera imaging, allowed visualization of the xenograft (*Id.*)*.*

Fynomers can also bind to target antigens with a similar affinity and specificity to antibodies. Fynomers are based on the human Fyn SH3 domain as a scaffold for assembly of binding molecules. The Fyn SH3 domain is a fully human, 63 amino acid protein that can be produced in bacteria with high yields. Fynomers may be linked together to yield a multispecific binding protein with affinities for two or more different antigen targets. Fynomers are commercially available from COVAGEN AG (Zurich, Switzerland).

The skilled artisan will realize that affibodies or fynomers may be used as targeting molecules in the practice of the claimed methods and compositions.

### Pre-Targeting

Bispecific or multispecific antibodies may be utilized in pre-targeting techniques. Pre-targeting is a multistep process originally developed to resolve the slow blood clearance of directly targeting antibodies, which contributes to undesirable toxicity to normal tissues such as bone marrow. With pre-targeting, a radionuclide or other therapeutic agent is attached to a small delivery molecule (targetable construct or targetable construct) that is cleared within minutes from the blood. A pre-targeting bispecific or multispecific antibody, which has binding sites for the targetable construct as well as a target antigen, is administered first, free antibody is allowed to clear from circulation and then the targetable construct is administered.

Pre-targeting methods are disclosed, for example, in Goodwin et al., U.S. Pat. No. 4,863,713; Goodwin et al., J. Nucl. Med. 29:226, 1988; Hnatowich et al., J. Nucl. Med. 28:1294, 1987; Oehr et al., J. Nucl. Med. 29:728, 1988; Klibanov et al., J. Nucl. Med. 29:1951, 1988; Sinitsyn et al., J. Nucl. Med. 30:66, 1989; Kalofonos et al., J. Nucl. Med. 31:1791, 1990; Schechter et al., Int. J. Cancer 48:167, 1991; Paganelli et al., Cancer Res. 51:5960, 1991; Paganelli et al., Nucl. Med. Commun. 12:211, 1991; U.S. Pat. No. 5,256,395; Stickney et al., Cancer Res. 51:6650, 1991; Yuan et al., Cancer Res. 51:3119,1991; U.S. Pat. Nos. 6,077,499; 7,011,812; 7,300,644; 7,074,405; 6,962,702; 7,387,772; 7,052,872; 7,138,103; 6,090,381; 6,472,511; 6,962,702; 6,962,702; 7,074,405; and U.S. Ser. No. 10/114,315 (now abandoned).

A pre-targeting method of treating or diagnosing a disease or disorder in a subject may be provided by: (1) administering to the subject a bispecific antibody or antibody fragment; (2) optionally administering to the subject a clearing composition, and allowing the composition to clear the antibody from circulation; and (3) administering to the subject the targetable construct, containing one or more chelated or chemically bound therapeutic or diagnostic agents. The technique may also be utilized for antibody dependent enzyme prodrug therapy (ADEPT) by administering an enzyme conjugated to a targetable construct, followed by a prodrug that is converted into active form by the enzyme.

### DOCK-AND-LOCK™ (DNL™)

According to the invention as defined in the claims, an anti-IGF-1R complex is formed as a DOCK-AND-LOCK™ (DNL™) complex (see, e.g., U.S. Patent Nos. 7,521,056; 7,527,787; 7,534,866; 7,550,143 and 7,666,400.) Generally, the technique takes advantage of the specific and high-affinity binding interactions that occur between a dimerization and docking domain (DDD) sequence of the regulatory (R) subunits of cAMP-dependent protein kinase (PKA) and an anchor domain (AD) sequence derived from any of a variety of AKAP proteins (Baillie et al., FEBS Letters. 2005; 579: 3264. Wong and Scott, Nat. Rev. Mol. Cell Biol. 2004; 5: 959). The DDD and AD peptides may be attached to any protein, peptide or other molecule. Because the DDD sequences spontaneously dimerize and bind to the AD sequence, the technique allows the formation of complexes between any selected molecules that may be attached to DDD or AD sequences.

Although the standard DNL™ complex comprises a trimer with two DDD-linked molecules attached to one AD-linked molecule, variations in complex structure allow the formation of dimers, trimers, tetramers, pentamers, hexamers and other multimers. In some embodiments, the DNL™ complex may further comprise two or more antibodies, antibody fragments or fusion proteins which bind to the same antigenic determinant or to two or more different antigens. The DNL™ complex may also comprise one or more other effectors, such as proteins, peptides, immunomodulators, cytokines, interleukins, interferons, binding proteins, peptide ligands, carrier proteins, toxins, ribonucleases such as onconase, inhibitory oligonucleotides such as siRNA, antigens or xenoantigens, polymers such as PEG, enzymes, therapeutic agents, hormones, cytotoxic agents, anti-angiogenic agents, pro-apoptotic agents or any other molecule or aggregate.

PKA, which plays a central role in one of the best studied signal transduction pathways triggered by the binding of the second messenger cAMP to the R subunits, was first isolated from rabbit skeletal muscle in 1968 (Walsh et al., J. Biol. Chem. 1968;243:3763). The structure of the holoenzyme consists of two catalytic subunits held in an inactive form by the R subunits (Taylor, J. Biol. Chem. 1989;264:8443). Isozymes of PKA are found with two types of R subunits (RI and RII), and each type has α and β isoforms (Scott, Pharmacol. Ther. 1991;50:123). Thus, the four isoforms of PKA regulatory subunits are RIα, RIβ, RIIα and RIIβ. The R subunits have been isolated only as stable dimers and the dimerization domain has been shown to consist of the first 44 amino-terminal residues of RIIα (Newlon et al., Nat. Struct. Biol. 1999; 6:222). As discussed below, similar portions of the amino acid sequences of other regulatory subunits are involved in dimerization and docking, each located near the N-terminal end of the regulatory subunit. Binding of cAMP to the R subunits leads to the release of active catalytic subunits for a broad spectrum of serine/threonine kinase activities, which are oriented toward selected substrates through the compartmentalization of PKA via its docking with AKAPs (Scott et al., J. Biol. Chem. 1990;265;21561)

Since the first AKAP, microtubule-associated protein-2, was characterized in 1984 (Lohmann et al., Proc. Natl. Acad. Sci USA. 1984; 81:6723), more than 50 AKAPs that localize to various sub-cellular sites, including plasma membrane, actin cytoskeleton, nucleus, mitochondria, and endoplasmic reticulum, have been identified with diverse structures in species ranging from yeast to humans (Wong and Scott, Nat. Rev. Mol. Cell Biol. 2004;5:959). The AD of AKAPs for PKA is an amphipathic helix of 14-18 residues (Carr et al., J. Biol. Chem. 1991;266:14188). The amino acid sequences of the AD are quite varied among individual AKAPs, with the binding affinities reported for RII dimers ranging from 2 to 90 nM (Alto et al., Proc. Natl. Acad. Sci. USA. 2003;100:4445). AKAPs will only bind to dimeric R subunits. For human RIIα, the AD binds to a hydrophobic surface formed by the 23 amino-terminal residues (Colledge and Scott, Trends Cell Biol. 1999; 6:216). Thus, the dimerization domain and AKAP binding domain of human RIIα are both located within the same N-terminal 44 amino acid sequence (Newlon et al., Nat. Struct. Biol. 1999;6:222; Newlon et al., EMBO J. 2001;20:1651), which is termed the DDD herein.

We have developed a platform technology to utilize the DDD of human PKA regulatory subunits and the AD of AKAP as an excellent pair of linker modules for docking any two entities, referred to hereafter as **A** and **B,** into a noncovalent complex, which could be further locked into a DNL™ complex through the introduction of cysteine residues into both the DDD and AD at strategic positions to facilitate the formation of disulfide bonds. The general methodology of the approach is as follows. Entity **A** is constructed by linking a DDD sequence to a precursor of **A**, resulting in a first component hereafter referred to as **a.** Because the DDD sequence would effect the spontaneous formation of a dimer, **A** would thus be composed of **a₂**. Entity **B** is constructed by linking an AD sequence to a precursor of **B,** resulting in a second component hereafter referred to as **b.** The dimeric motif of DDD contained in **a**₂ will create a docking site for binding to the AD sequence contained in **b,** thus facilitating a ready association of **a₂** and **b** to form a binary, trimeric complex composed of **a₂b.** This binding event is made irreversible with a subsequent reaction to covalently secure the two entities via disulfide bridges, which occurs very efficiently based on the principle of effective local concentration because the initial binding interactions should bring the reactive thiol groups placed onto both the DDD and AD into proximity (Chmura et al., Proc. Natl. Acad. Sci. USA. 2001;98:8480) to ligate site-specifically. Using various combinations of linkers, adaptor modules and precursors, a wide variety of DNL™ constructs of different stoichiometry may be produced and used (see, e.g., U.S. Nos. 7,550,143; 7,521,056; 7,534,866; 7,527,787 and 7,666,400.)

By attaching the DDD and AD away from the functional groups of the two precursors, such site-specific ligations are also expected to preserve the original activities of the two precursors. This approach is modular in nature and potentially can be applied to link, site-specifically and covalently, a wide range of substances, including peptides, proteins, antibodies, antibody fragments, and other effector moieties with a wide range of activities. Utilizing the fusion protein method of constructing AD and DDD conjugated effectors described in the Examples below, virtually any protein or peptide may be incorporated into a DNL™ construct. However, the technique is not limiting and other methods of conjugation may be utilized.

A variety of methods are known for making fusion proteins, including nucleic acid synthesis, hybridization and/or amplification to produce a synthetic double-stranded nucleic acid encoding a fusion protein of interest. Such double-stranded nucleic acids may be inserted into expression vectors for fusion protein production by standard molecular biology techniques (see, e.g. Sambrook et al., Molecular Cloning, A laboratory manual, 2nd Ed, 1989). In such preferred embodiments, the AD and/or DDD moiety may be attached to either the N-terminal or C-terminal end of an effector protein or peptide. However, the skilled artisan will realize that the site of attachment of an AD or DDD moiety to an effector moiety may vary, depending on the chemical nature of the effector moiety and the part(s) of the effector moiety involved in its physiological activity. Site-specific attachment of a variety of effector moieties may be performed using techniques known in the art, such as the use of bivalent cross-linking reagents and/or other chemical conjugation techniques.

### Structure-Function Relationships in AD and DDD Moieties

For different types of DNL™ constructs, different AD or DDD sequences may be utilized. Exemplary DDD and AD sequences are provided below.
*DDD1*
   SHIQIPPGLTELLQGYTVEVLRQQPPDLVEFAVEYFTRLREARA (SEQ ID NO:1)
*DDD2*
   CGHIQIPPGLTELLQGYTVEVLRQQPPDLVEFAVEYFTRLREARA (SEQ ID NO:2)
*AD1*
   QIEYLAKQIVDNAIQQA (SEQ ID NO:3)
*AD2*
   CGQIEYLAKQIVDNAIQQAGC (SEQ ID NO:4)

The skilled artisan will realize that DDD1 and DDD2 are based on the DDD sequence of the human RIIα isoform of protein kinase A. However, in alternative embodiments, the DDD and AD moieties may be based on the DDD sequence of the human RIa form of protein kinase A and a corresponding AKAP sequence, as exemplified in DDD3, DDD3C and AD3 below.
*DDD3*
   SLRECELYVQKHNIQALLKDSIVQLCTARPERPMAFLREYFERLEKEEAK (SEQ ID NO:5)
*DDD3C*
*AD3*
   CGFEELAWKIAKMIWSDVFQQGC (SEQ ID NO:7)

In other alternative embodiments, other sequence variants of AD and/or DDD moieties may be utilized in construction of the DNL™ complexes. For example, there are only four variants of human PKA DDD sequences, corresponding to the DDD moieties of PKA RIα, RIIα, RIβ and RIIβ. The RIIα DDD sequence is the basis of DDD1 and DDD2 disclosed above. The four human PKA DDD sequences are shown below. The DDD sequence represents residues 1-44 of RIIα, 1-44 of RIIβ, 12-61 of RIa and 13-66 of RIβ. (Note that the sequence of DDD1 is modified slightly from the human PKA RIIα DDD moiety.)
*PKA RI*α
   SLRECELYVQKHNIQALLKDVSIVQLCTARPERPMAFLREYFEKLEKEEAK (SEQ ID NO:8)
*PKA RI*β
   SLKGCELYVQLHGIQQVLKDCIVHLCISKPERPMKFLREHFEKLEKEENRQILA (SEQ ID NO:9)
*PKA RII*α
   SHIQIPPGLTELLQGYTVEVGQQPPDLVDFAVEYFTRLREARRQ (SEQ ID NO: 10)
*PKA RII*β
   SIEIPAGLTELLQGFTVEVLRHQPADLLEFALQHFTRLQQENER (SEQ ID NO:11)

The structure-function relationships of the AD and DDD domains have been the subject of investigation. (See, e.g., Burns-Hamuro et al., 2005, Protein Sci 14:2982-92; Carr et al., 2001, J Biol Chem 276:17332-38; Alto et al., 2003, Proc Natl Acad Sci USA 100:4445-50; Hundsrucker et al., 2006, Biochem J 396:297-306; Stokka et al., 2006, Biochem J 400:493-99; Gold et al., 2006, Mol Cell 24:383-95; Kinderman et al., 2006, Mol Cell 24:397-408.)

For example, Kinderman et al. (2006, Mol Cell 24:397-408) examined the crystal structure of the AD-DDD binding interaction and concluded that the human DDD sequence contained a number of conserved amino acid residues that were important in either dimer formation or AKAP binding, underlined in SEQ ID NO:1 below. (See Figure 1 of Kinderman et al., 2006.) The skilled artisan will realize that in designing sequence variants of the DDD sequence, one would desirably avoid changing any of the underlined residues, while conservative amino acid substitutions might be made for residues that are less critical for dimerization and AKAP binding.
SHIQIPPGLTELLQGYTVEVLRQQPPDLVEFAVEYFTRLREARA (SEQ ID NO:1)

As discussed in more detail below, conservative amino acid substitutions have been characterized for each of the twenty common L-amino acids. Thus, based on the data of Kinderman (2006) and conservative amino acid substitutions, potential alternative DDD sequences based on SEQ ID NO:1 are shown in **Table 2.** In devising **Table 2,** only highly conservative amino acid substitutions were considered. For example, charged residues were only substituted for residues of the same charge, residues with small side chains were substituted with residues of similar size, hydroxyl side chains were only substituted with other hydroxyls, etc. Because of the unique effect of proline on amino acid secondary structure, no other residues were substituted for proline. A limited number of such potential alternative DDD moiety sequences are shown in SEQ ID NO:12 to SEQ ID NO:31 below. The skilled artisan will realize that an almost unlimited number of alternative species within the genus of DDD moieties can be constructed by standard techniques, for example using a commercial peptide synthesizer or well known site-directed mutagenesis techniques. The effect of the amino acid substitutions on AD moiety binding may also be readily determined by standard binding assays, for example as disclosed in Alto et al. (2003, Proc Natl Acad Sci USA 100:4445-50).

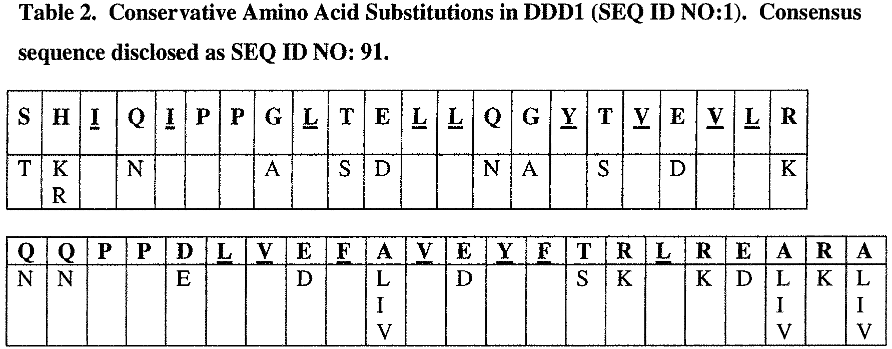

THIQIPPGLTELLQGYTVEVLRQQPPDLVEFAVEYFTRLREARA (SEQ ID NO:12)
SKIQIPPGLTELLQGYTVEVLRQQPPDLVEFAVEYFTRLREARA (SEQ ID NO:13)
SRIQIPPGLTELLQGYTVEVLRQQPPDLVEFAVEYFTRLREARA (SEQ ID NO:14)
SHINIPPGLTELLQGYTVEVLRQQPPDLVEFAVEYFTRLREARA (SEQ ID NO:15)
SHIQIPPALTELLQGYTVEVLRQQPPDLVEFAVEYFTRLREARA (SEQ ID NO:16)
SHIQIPPGLSELLQGYTVEVLRQQPPDLVEFAVEYFTRLREARA (SEQ ID NO:17)
SHIQIPPGLTDLLQGYTVEVLRQQPPDLVEFAVEYFTRLREARA (SEQ ID NO:18)
SHIQIPPGLTELLNGYTVEVLRQQPPDLVEFAVEYFTRLREARA (SEQ ID NO:19)
SHIQIPPGLTELLQAYTVEVLRQQPPDLVEFAVEYFTRLREARA (SEQ ID NO:20)
SHIQIPPGLTELLQGYSVEVLRQQPPDLVEFAVEYFTRLREARA (SEQ ID NO:21)
SHIQIPPGLTELLQGYTVDVLRQQPPDLVEFAVEYFTRLREARA (SEQ ID NO:22)
SHIQIPPGLTELLQGYTVEVLKQQPPDLVEFAVEYFTRLREARA (SEQ ID NO:23)
SHIQIPPGLTELLQGYTVEVLRNQPPDLVEFAVEYFTRLREARA (SEQ ID NO:24)
SHIQIPPGLTELLQGYTVEVLRQNPPDLVEFAVEYFTRLREARA (SEQ ID NO:25)
SHIQIPPGLTELLQGYTVEVLRQQPPELVEFAVEYFTRLREARA (SEQ ID NO:26)
SHIQIPPGLTELLQGYTVEVLRQQPPDLVDFAVEYFTRLREARA (SEQ ID NO:27)
SHIQIPPGLTELLQGYTVEVLRQQPPDLVEFLVEYFTRLREARA (SEQ ID NO:28)
SHIQIPPGLTELLQGYTVEVLRQQPPDLVEFIVEYFTRLREARA (SEQ ID NO:29)
SHIQIPPGLTELLQGYTVEVLRQQPPDLVEFVVEYFTRLREARA (SEQ ID NO:30)
SHIQIPPGLTELLQGYTVEVLRQQPPDLVEFAVDYFTRLREARA (SEQ ID NO:31)

Alto et al. (2003, Proc Natl Acad Sci USA 100:4445-50) performed a bioinformatic analysis of the AD sequence of various AKAP proteins to design an RII selective AD sequence called AKAP-IS (SEQ ID NO:3), with a binding constant for DDD of 0.4 nM. The AKAP-IS sequence was designed as a peptide antagonist of AKAP binding to PKA. Residues in the AKAP-IS sequence where substitutions tended to decrease binding to DDD are underlined in SEQ ID NO:3 below. The skilled artisan will realize that in designing sequence variants of the AD sequence, one would desirably avoid changing any of the underlined residues, while conservative amino acid substitutions might be made for residues that are less critical for DDD binding. **Table 3** shows potential conservative amino acid substitutions in the sequence of AKAP-IS (AD1, SEQ ID NO:3), similar to that shown for DDD1 (SEQ ID NO:1) in **Table 2** above.

A limited number of such potential alternative AD moiety sequences are shown in SEQ ID NO:32 to SEQ ID NO:49 below. Again, a very large number of species within the genus of possible AD moiety sequences could be made, tested and used by the skilled artisan, based on the data of Alto et al. (2003). It is noted that Figure 2 of Alto (2003) shows an even large number of potential amino acid substitutions that may be made, while retaining binding activity to DDD moieties, based on actual binding experiments.
***AKAP-IS***
QIEYLAKQIVDNAIQQA (SEQ ID NO:3)

**Table 3. Conservative Amino Acid Substitutions in AD1 (SEQ ID NO:3). Consensus sequence disclosed as SEQ ID NO: 92.**

| **Q** | **I** | **E** | **Y** | **L** | **A** | **K** | **Q** | **I** | **V** | **D** | **N** | **A** | **I** | **Q** | **Q** | **A** |
|---|---|---|---|---|---|---|---|---|---|---|---|---|---|---|---|---|
| N | LV | D | FTS | IV | | R | N | | | E | Q | | | N | N | LIV |

NIEYLAKQIVDNAIQQA (SEQ ID NO:32)
QLEYLAKQIVDNAIQQA (SEQ ID NO:33)
QVEYLAKQIVDNAIQQA (SEQ ID NO:34)
QIDYLAKQIVDNAIQQA (SEQ ID NO:35)
QIEFLAKQIVDNAIQQA (SEQ ID NO:36)
QIETLAKQIVDNAIQQA (SEQ ID NO:37)
QIESLAKQIVDNAIQQA (SEQ ID NO:38)
QIEYIAKQIVDNAIQQA (SEQ ID NO:39)
QIEYVAKQIVDNAIQQA (SEQ ID NO:40)
QIEYLARQIVDNAIQQA (SEQ ID NO:41)
QIEYLAKNIVDNAIQQA (SEQ ID NO:42)
QIEYLAKQIVENAIQQA (SEQ ID NO:43)
QIEYLAKQIVDQAIQQA (SEQ ID NO:44)
QIEYLAKQIVDNAINQA (SEQ ID NO:45)
QIEYLAKQIVDNAIQNA (SEQ ID NO:46)
QIEYLAKQIVDNAIQQL (SEQ ID NO:47)
QIEYLAKQIVDNAIQQI (SEQ ID NO:48)
QIEYLAKQIVDNAIQQV (SEQ ID NO:49)

Gold et al. (2006, Mol Cell 24:383-95) utilized crystallography and peptide screening to develop a SuperAKAP-IS sequence (SEQ ID NO:50), exhibiting a five order of magnitude higher selectivity for the RII isoform of PKA compared with the RI isoform. Underlined residues indicate the positions of amino acid substitutions, relative to the AKAP-IS sequence, which increased binding to the DDD moiety of RIIα. In this sequence, the N-terminal Q residue is numbered as residue number 4 and the C-terminal A residue is residue number 20. Residues where substitutions could be made to affect the affinity for RIIα were residues 8, 11, 15, 16, 18, 19 and 20 (Gold et al., 2006). It is contemplated that in certain alternative embodiments, the SuperAKAP-IS sequence may be substituted for the AKAP-IS AD moiety sequence to prepare DNL™ constructs. Other alternative sequences that might be substituted for the AKAP-IS AD sequence are shown in SEQ ID NO:51-53. Substitutions relative to the AKAP-IS sequence are underlined. It is anticipated that, as with the AD2 sequence shown in SEQ ID NO:4, the AD moiety may also include the additional N-terminal residues cysteine and glycine and C-terminal residues glycine and cysteine.
*SuperAKAP-IS*
   QIEYVAKQIVDYAIHQA (SEQ ID NO:50)
*Alternative AKAP sequences*
   QIEYKAKQIVDHAIHQA (SEQ ID NO:51)
   QIEYHAKQIVDHAIHQA (SEQ ID NO:52)
   QIEYVAKQIVDHAIHQA (SEQ ID NO:53)

Figure 2 of Gold et al. disclosed additional DDD-binding sequences from a variety of AKAP proteins, shown below.

### RII-Specific AKAPs

*AKAP-KL*
   PLEYQAGLLVQNAIQQAI (SEQ ID NO:54)
*AKAP79*
   LLIETASSLVKNAIQLSI (SEQ ID NO:55)
*AKAP-Lbc*
   LIEEAASRIVDAVIEQVK (SEQ ID NO:56)

### RI-Specific AKAPs

*AKAPce*
   ALYQFADRFSELVISEAL (SEQ ID NO:57)
*RIAD*
   LEQVANQLADQIIKEAT (SEQ ID NO:58)
*PV38*
   FEELAWKIAKMIWSDVF (SEQ ID NO:59)

### Dual-Specificity AKAPs

*AKAP7*
   ELVRLSKRLVENAVLKAV (SEQ ID NO:60)
*MAP2D*
   TAEEVSARIVQVVTAEAV (SEQ ID NO:61)
*DAKAP1*
   QIKQAAFQLISQVILEAT (SEQ ID NO:62)
*DAKAP2*
   LAWKIAKMIVSDVMQQ (SEQ ID NO:63)

Stokka et al. (2006, Biochem J 400:493-99) also developed peptide competitors of AKAP binding to PKA, shown in SEQ ID NO:64-66. The peptide antagonists were designated as Ht31 (SEQ ID NO:64), RIAD (SEQ ID NO:65) and PV-38 (SEQ ID NO:66). The Ht-31 peptide exhibited a greater affinity for the RII isoform of PKA, while the RIAD and PV-38 showed higher affinity for RI.
*Ht31*
   DLIEEAASRIVDAVIEQVKAAGAY (SEQ ID NO:64)
*RIAD*
   LEQYANQLADQIIKEATE (SEQ ID NO:65)
*PV-38*
   FEELAWKIAKMIWSDVFQQC (SEQ ID NO:66)

Hundsrucker et al. (2006, Biochem J 396:297-306) developed still other peptide competitors for AKAP binding to PKA, with a binding constant as low as 0.4 nM to the DDD of the RII form of PKA. The sequences of various AKAP antagonistic peptides are provided in Table 1 of Hundsrucker et al., reproduced in **Table 4** below. AKAPIS represents a synthetic RII subunit-binding peptide. All other peptides are derived from the RII-binding domains of the indicated AKAPs.

**Table 4. AKAP Peptide sequences**

| | Peptide Sequence |
|---|---|
| AKAPIS | QIEYLAKQIVDNAIQQA (SEQ ID NO:3) |
| AKAPIS-P | QIEYLAKQIPDNAIQQA (SEQ ID NO:67) |
| Ht31 | KGADLIEEAASRIVDAVIEQVKAAG (SEQ ID NO:68) |
| Ht31-P | KGADLIEEAASRIPDAPIEQVKAAG (SEQ ID NO:69) |
| AKAP7 -wt-pep | PEDAELVRLSKRLVENAVLKAVQQY (SEQ ID NO:70) |
| AKAP7 -L304T-pep | PEDAELVRTSKRLVENAVLKAVQQY (SEQ ID NO:71) |
| AKAP7 -L308D-pep | PEDAELVRLSKRDVENAVLKAVQQY (SEQ ID NO:72) |
| AKAP7 -P-pep | PEDAELVRLSKRLPENAVLKAVQQY (SEQ ID NO:73) |
| AKAP7 -PP-pep | PEDAELVRLSKRLPENAPLKAVQQY (SEQ ID NO:74) |
| AKAP7 -L314E-pep | PEDAELVRLSKRLVENAVEKAVQQY (SEQ ID NO:75) |
| AKAP1-pep | EEGLDRNEEIKRAAFQIISQVISEA (SEQ ID NO:76) |
| AKAP2-pep | LVDDPLEYQAGLLVQNAIQQAIAEQ (SEQ ID NO:77) |
| AKAP5-pep | QYETLLIETASSLVKNAIQLSIEQL (SEQ ID NO:78) |
| AKAP9-pep | LEKQYQEQLEEEVAKVIVSMSIAFA (SEQ ID NO:79) |
| AKAP10-pep | NTDEAQEELAWKIAKMIVSDIMQQA (SEQ ID NO:80) |
| AKAP11-pep | VNLDKKAVLAEKIVAEAIEKAEREL (SEQ ID NO:81) |
| AKAP12-pep | NGILELETKSSKLVQNIIQTAVDQF (SEQ ID NO:82) |
| AKAP14-pep | TQDKNYEDELTQVALALVEDVINYA (SEQ ID NO:83) |
| Rab32-pep | ETSAKDNINIEEAARFLVEKILVNH (SEQ ID NO:84) |

Residues that were highly conserved among the AD domains of different AKAP proteins are indicated below by underlining with reference to the AKAP IS sequence (SEQ ID NO:3). The residues are the same as observed by Alto et al. (2003), with the addition of the C-terminal alanine residue. (See FIG. 4 of Hundsrucker et al. (2006).) The sequences of peptide antagonists with particularly high affinities for the RII DDD sequence were those of AKAP-IS, AKAP7δ-wt-pep, AKAP7δ-L304T-pep and AKAP78-L308D-pep.
*AKAP-IS*
QIEYLAKQIVDNAIQQA (SEQ ID NO:3)

Carr et al. (2001, J Biol Chem 276:17332-38) examined the degree of sequence homology between different AKAP-binding DDD sequences from human and non-human proteins and identified residues in the DDD sequences that appeared to be the most highly conserved among different DDD moieties. These are indicated below by underlining with reference to the human PKA RIIα DDD sequence of SEQ ID NO:1. Residues that were particularly conserved are further indicated by italics. The residues overlap with, but are not identical to those suggested by Kinderman et al. (2006) to be important for binding to AKAP proteins. The skilled artisan will realize that in designing sequence variants of DDD, it would be most preferred to avoid changing the most conserved residues (italicized), and it would be preferred to also avoid changing the conserved residues (underlined), while conservative amino acid substitutions may be considered for residues that are neither underlined nor italicized..
SHIQ*IP*P*GL*T*ELL*Q*G*Y*T*V*EVLR*Q*QP*P*DL*VE*FA*VE*YF*TR*L*REA*R*A (SEQ ID NO:1)

A modified set of conservative amino acid substitutions for the DDD1 (SEQ ID NO:1) sequence, based on the data of Carr et al. (2001) is shown in **Table 5**. Even with this reduced set of substituted sequences, there are over 65,000 possible alternative DDD moiety sequences that may be produced, tested and used by the skilled artisan without undue experimentation. The skilled artisan could readily derive such alternative DDD amino acid sequences as disclosed above for **Table 2 and Table 3.**

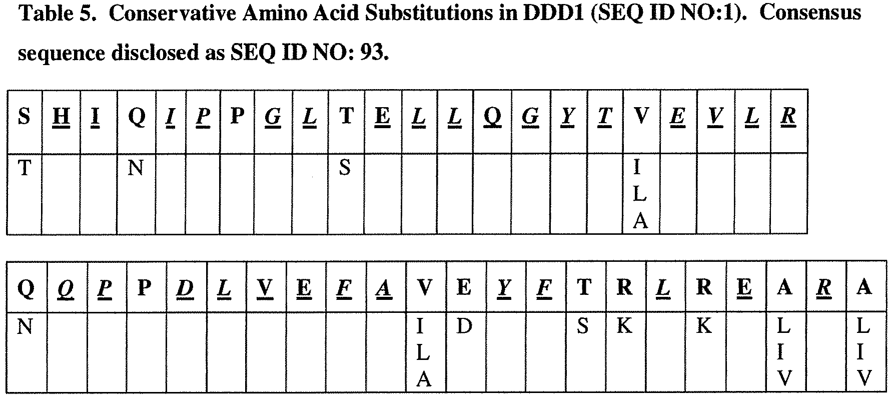

The skilled artisan will realize that these and other amino acid substitutions in the DDD or AD amino acid sequences may be utilized to produce alternative species within the genus of AD or DDD moieties, using techniques that are standard in the field and only routine experimentation.

### Amino Acid Substitutions

In alternative embodiments, the disclosed methods and compositions may involve production and use of proteins or peptides with one or more substituted amino acid residues. For example, the DDD and/or AD sequences used to make DNL™ constructs may be modified as discussed above.

The skilled artisan will be aware that, in general, amino acid substitutions typically involve the replacement of an amino acid with another amino acid of relatively similar properties (i.e., conservative amino acid substitutions). The properties of the various amino acids and effect of amino acid substitution on protein structure and function have been the subject of extensive study and knowledge in the art.

For example, the hydropathic index of amino acids may be considered (Kyte & Doolittle, 1982, J. Mol. Biol., 157:105-132). The relative hydropathic character of the amino acid contributes to the secondary structure of the resultant protein, which in turn defines the interaction of the protein with other molecules. Each amino acid has been assigned a hydropathic index on the basis of its hydrophobicity and charge characteristics (Kyte & Doolittle, 1982), these are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5). In making conservative substitutions, the use of amino acids whose hydropathic indices are within ± 2 is preferred, within ± 1 are more preferred, and within ± 0.5 are even more preferred.

Amino acid substitution may also take into account the hydrophilicity of the amino acid residue (e.g., U.S. Pat. No. 4,554,101). Hydrophilicity values have been assigned to amino acid residues: arginine (+3.0); lysine (+3.0); aspartate (+3.0); glutamate (+3.0); serine (+0.3); asparagine (+0.2); glutamine (+0.2); glycine (0); threonine (-0.4); proline (-0.5 .+-.1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). Replacement of amino acids with others of similar hydrophilicity is preferred.

Other considerations include the size of the amino acid side chain. For example, it would generally not be preferred to replace an amino acid with a compact side chain, such as glycine or serine, with an amino acid with a bulky side chain, e.g., tryptophan or tyrosine. The effect of various amino acid residues on protein secondary structure is also a consideration. Through empirical study, the effect of different amino acid residues on the tendency of protein domains to adopt an alpha-helical, beta-sheet or reverse turn secondary structure has been determined and is known in the art (see, e.g., Chou & Fasman, 1974, Biochemistry, 13:222-245; 1978, Ann. Rev. Biochem., 47: 251-276; 1979, Biophys. J., 26:367-384).

Based on such considerations and extensive empirical study, tables of conservative amino acid substitutions have been constructed and are known in the art. For example: arginine and lysine; glutamate and aspartate; serine and threonine; glutamine and asparagine; and valine, leucine and isoleucine. Alternatively: Ala (A) leu, ile, val; Arg (R) gln, asn, lys; Asn (N) his, asp, lys, arg, gln; Asp (D) asn, glu; Cys (C) ala, ser; Gln (Q) glu, asn; Glu (E) gln, asp; Gly (G) ala; His (H) asn, gln, lys, arg; Ile (I) val, met, ala, phe, leu; Leu (L) val, met, ala, phe, ile; Lys (K) gln, asn, arg; Met (M) phe, ile, leu; Phe (F) leu, val, ile, ala, tyr; Pro (P) ala; Ser (S), thr; Thr (T) ser; Trp (W) phe, tyr; Tyr (Y) trp, phe, thr, ser; Val (V) ile, leu, met, phe, ala.

Other considerations for amino acid substitutions include whether or not the residue is located in the interior of a protein or is solvent exposed. For interior residues, conservative substitutions would include: Asp and Asn; Ser and Thr; Ser and Ala; Thr and Ala; Ala and Gly; Ile and Val; Val and Leu; Leu and Ile; Leu and Met; Phe and Tyr; Tyr and Trp. (See, e.g., PROWL website at rockefeller.edu) For solvent exposed residues, conservative substitutions would include: Asp and Asn; Asp and Glu; Glu and Gln; Glu and Ala; Gly and Asn; Ala and Pro; Ala and Gly; Ala and Ser; Ala and Lys; Ser and Thr; Lys and Arg; Val and Leu; Leu and Ile; Ile and Val; Phe and Tyr. (Id.) Various matrices have been constructed to assist in selection of amino acid substitutions, such as the PAM250 scoring matrix, Dayhoff matrix, Grantham matrix, McLachlan matrix, Doolittle matrix, Henikoff matrix, Miyata matrix, Fitch matrix, Jones matrix, Rao matrix, Levin matrix and Risler matrix (*Idem.*)

In determining amino acid substitutions, one may also consider the existence of intermolecular or intramolecular bonds, such as formation of ionic bonds (salt bridges) between positively charged residues (e.g., His, Arg, Lys) and negatively charged residues (e.g., Asp, Glu) or disulfide bonds between nearby cysteine residues.

Methods of substituting any amino acid for any other amino acid in an encoded protein sequence are well known and a matter of routine experimentation for the skilled artisan, for example by the technique of site-directed mutagenesis or by synthesis and assembly of oligonucleotides encoding an amino acid substitution and splicing into an expression vector construct.

### Therapeutic Agents

In various embodiments of the invention as defined in the claims, therapeutic agents such as cytotoxic agents, anti-angiogenic agents, pro-apoptotic agents, antibiotics, hormones, hormone antagonists, chemokines, drugs, prodrugs, toxins, enzymes or other agents may be used, either conjugated to the subject anti-IGF-1R complexes or separately administered before, simultaneously with, or after the anti-IGF-1R complex. Drugs of use may possess a pharmaceutical property selected from the group consisting of antimitotic, antikinase, alkylating, antimetabolite, antibiotic, alkaloid, anti-angiogenic, pro-apoptotic agents and combinations thereof.

Exemplary drugs of use may include 5-fluorouracil, aplidin, azaribine, anastrozole, anthracyclines, bendamustine, bleomycin, bortezomib, bryostatin-1, busulfan, calicheamycin, camptothecin, carboplatin, 10-hydroxycamptothecin, carmustine, celebrex, chlorambucil, cisplatin (CDDP), Cox-2 inhibitors, irinotecan (CPT-11), SN-38, carboplatin, cladribine, camptothecans, cyclophosphamide, cytarabine, dacarbazine, docetaxel, dactinomycin, daunorubicin, doxorubicin, 2-pyrrolinodoxorubicine (2P-DOX), cyano-morpholino doxorubicin, doxorubicin glucuronide, epirubicin glucuronide, estramustine, epipodophyllotoxin, estrogen receptor binding agents, etoposide (VP16), etoposide glucuronide, etoposide phosphate, floxuridine (FUdR), 3',5'-O-dioleoyl-FudR (FUdR-dO), fludarabine, flutamide, farnesyl-protein transferase inhibitors, gemcitabine, hydroxyurea, idarubicin, ifosfamide, L-asparaginase, lenolidamide, leucovorin, lomustine, mechlorethamine, melphalan, mercaptopurine, 6-mercaptopurine, methotrexate, mitoxantrone, mithramycin, mitomycin, mitotane, navelbine, nitrosourea, plicomycin, procarbazine, paclitaxel, pentostatin, PSI-341, raloxifene, semustine, streptozocin, tamoxifen, taxol, temazolomide (an aqueous form of DTIC), transplatinum, thalidomide, thioguanine, thiotepa, teniposide, topotecan, uracil mustard, vinorelbine, vinblastine, vincristine and vinca alkaloids.

Toxins of use may include ricin, abrin, alpha toxin, saporin, ribonuclease (RNase), e.g., onconase, DNase I, *Staphylococcal* enterotoxin-A, pokeweed antiviral protein, gelonin, diphtheria toxin, *Pseudomonas* exotoxin, and *Pseudomonas* endotoxin.

Chemokines of use may include RANTES, MCAF, MIP1-alpha, MIP1-Beta and IP-10.

In certain embodiments of the invention as defined in the claims, anti-angiogenic agents, such as angiostatin, baculostatin, canstatin, maspin, anti-VEGF antibodies, anti-PlGF peptides and antibodies, anti-vascular growth factor antibodies, anti-Flk-1 antibodies, anti-Flt-1 antibodies and peptides, anti-Kras antibodies, anti-cMET antibodies, anti-MIF (macrophage migration-inhibitory factor) antibodies, laminin peptides, fibronectin peptides, plasminogen activator inhibitors, tissue metalloproteinase inhibitors, interferons, interleukin-12, IP-10, Gro-β, thrombospondin, 2-methoxyoestradiol, proliferin-related protein, carboxiamidotriazole, CM101, Marimastat, pentosan poly sulphate, angiopoietin-2, interferon-alpha, herbimycin A, PNU145156E, 16K prolactin fragment, Linomide (roquinimex), thalidomide, pentoxifylline, genistein, TNP-470, endostatin, paclitaxel, accutin, angiostatin, cidofovir, vincristine, bleomycin, AGM-1470, platelet factor 4 or minocycline may be of use.

Immunomodulators of use may be selected from a cytokine, a stem cell growth factor, a lymphotoxin, an hematopoietic factor, a colony stimulating factor (CSF), an interferon (IFN), erythropoietin, thrombopoietin and a combination thereof. Specifically useful are lymphotoxins such as tumor necrosis factor (TNF), hematopoietic factors, such as interleukin (IL), colony stimulating factor, such as granulocyte-colony stimulating factor (G-CSF) or granulocyte macrophage-colony stimulating factor (GM-CSF), interferon, such as interferons-α, -β or -γ, and stem cell growth factor, such as that designated "S1 factor". Included among the cytokines are growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; prostaglandin, fibroblast growth factor; prolactin; placental lactogen, OB protein; tumor necrosis factor-α and -β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-I and -II; erythropoietin (EPO); osteoinductive factors; interferons such as interferon-α, -β, and -γ; colony stimulating factors (CSFs) such as macrophage-CSF (M-CSF); interleukins (ILs) such as IL-1, IL-1α IL-2, IL-3, IL-4, IL-5, IL-6, IL-7, IL-8, IL-9, IL-10, IL-11, IL-12; IL-13, IL-14, IL-15, IL-16, IL-17, IL-18, IL-21, IL-25, LIF, kit-ligand or FLT-3, angiostatin, thrombospondin, endostatin, tumor necrosis factor and LT.

Radionuclides of use include, but are not limited to- ¹¹¹In, ¹⁷⁷Lu, ²¹²Bi, ²¹³Bi, ²¹¹At, ⁶²Cu, ⁶⁷Cu, ⁹⁰Y, ¹²⁵I, ¹³¹I, ³²P, ³³P, ⁴⁷Sc, ¹¹¹Ag, ⁶⁷Ga, ¹⁴²Pr, ¹⁵³Sm, ¹⁶¹Tb, ¹⁶⁶Dy, ¹⁶⁶Ho, ¹⁸⁶Re, ¹⁸⁸Re, ¹⁸⁹Re, ²¹²Pb, ²²³Ra, ²²⁵Ac, ⁵⁹Fe, ⁷⁵Se, ⁷⁷As, ⁸⁹Sr, ⁹⁹Mo, ¹⁰⁵Rh, ¹⁰⁹Pd, ¹⁴³Pr, ¹⁴⁹Pm, ¹⁶⁹Er, ¹⁹⁴Ir, ¹⁹⁸Au, ¹⁹⁹Au, and ²¹¹Pb. The therapeutic radionuclide preferably has a decay-energy in the range of 20 to 6,000 keV, preferably in the ranges 60 to 200 keV for an Auger emitter, 100-2,500 keV for a beta emitter, and 4,000-6,000 keV for an alpha emitter. Maximum decay energies of useful beta-particle-emitting nuclides are preferably 20-5,000 keV, more preferably 100-4,000 keV, and most preferably 500-2,500 keV. Also preferred are radionuclides that substantially decay with Auger-emitting particles. For example, Co-58, Ga-67, Br-80m, Tc-99m, Rh-103m, Pt-109, In-Ill, Sb-119, 1-125, Ho-161, Os-189m and Ir-192. Decay energies of useful beta-particle-emitting nuclides are preferably <1,000 keV, more preferably <100 keV, and most preferably <70 keV. Also preferred are radionuclides that substantially decay with generation of alpha-particles. Such radionuclides include, but are not limited to: Dy-152, At-211, Bi-212, Ra-223, Rn-219, Po-215, Bi-211, Ac-225, Fr-221, At-217, Bi-213 and Fm-255. Decay energies of useful alpha-particle-emitting radionuclides are preferably 2,000-10,000 keV, more preferably 3,000-8,000 keV, and most preferably 4,000-7,000 keV. Additional potential radioisotopes of use include ¹¹C, ¹³N, ¹⁵O, ⁷⁵Br, ¹⁹⁸Au, ²²⁴Ac, ¹²⁶I, ¹³³I, ⁷⁷Br, ¹¹³In, ⁹⁵Ru, ⁹⁷Ru, ¹⁰³Ru, ¹⁰⁵Ru, ¹⁰⁷Hg, ²⁰³Hg, ¹²¹Te, ^{122m}Te, ^{125m}Te, ¹⁶⁵Tm, ¹⁶⁷Tm, ¹⁶⁸Tm, ¹⁹⁷Pt, ¹⁰⁹Pd, ¹⁰⁵Rh, ¹⁴²Pr, ¹⁴³Pr, ¹⁶¹Tb, ¹⁶⁶Ho, ¹⁹⁹Au , ⁵⁷Co, ⁵⁸Co, ⁵¹Cr, ⁵⁹Fe, ⁷⁵Se, ²⁰¹Tl, ²²⁵Ac, ⁷⁶Br, ¹⁶⁹Yb, and the like. Some useful diagnostic nuclides may include ¹⁸F, ⁵²Fe, ⁶²Cu, ⁶⁴Cu, ⁶⁷Cu, ⁶⁷Ga, ⁶⁸Ga, ⁸⁶Y, ⁸⁹Zr, ⁹⁴Tc, ^{94m}Tc, ^{99m}Tc, or ¹¹¹In.

Therapeutic agents may include a photoactive agent or dye. Fluorescent compositions, such as fluorochrome, and other chromogens, or dyes, such as porphyrins sensitive to visible light, have been used to detect and to treat lesions by directing the suitable light to the lesion. In therapy, this has been termed photoradiation, phototherapy, or photodynamic therapy. See Jori et al. (eds.), PHOTODYNAMIC THERAPY OF TUMORS AND OTHER DISEASES (Libreria Progetto 1985); van den Bergh, Chem. Britain (1986), 22:430. Moreover, monoclonal antibodies have been coupled with photoactivated dyes for achieving phototherapy. See Mew et al., J. Immunol. (1983),130:1473; idem., Cancer Res. (1985), 45:4380; Oseroff et al., Proc. Natl. Acad. Sci. USA (1986), 83:8744; idem., Photochem. Photobiol. (1987), 46:83; Hasan et al., Prog. Clin. Biol. Res. (1989), 288:471; Tatsuta et al., Lasers Surg. Med. (1989), 9:422; Pelegrin et al., Cancer (1991), 67:2529.

Other useful therapeutic agents may comprise oligonucleotides, especially antisense oligonucleotides that preferably are directed against oncogenes and oncogene products, such as bcl-2 or p53. A preferred form of therapeutic oligonucleotide is siRNA.

### Diagnostic Agents

Diagnostic agents are preferably selected from the group consisting of a radionuclide, a radiological contrast agent, a paramagnetic ion, a metal, a fluorescent label, a chemiluminescent label, an ultrasound contrast agent and a photoactive agent. Such diagnostic agents are well known and any such known diagnostic agent may be used. Nonlimiting examples of diagnostic agents may include a radionuclide such as ¹¹⁰In, ¹¹¹In, ¹⁷⁷Lu, ¹⁸F, ⁵²Fe, ⁶²Cu, ⁶⁴Cu, ⁶⁷Cu, ⁶⁷Ga, ⁶⁸Ga, ⁸⁶Y, ⁹⁰Y, ⁸⁹Zr, ^{94m}Tc, ⁹⁴Tc, ^{99m}Tc, ¹²⁰I, ¹²³I, ¹²⁴I, ¹²⁵I, ¹³¹I, ¹⁵⁴⁻¹⁵⁸Gd, ³²P, ¹¹C, ¹³N, ¹⁵O, ¹⁸⁶Re, ¹⁸⁸Re, ⁵¹Mn, ^{52m}Mn, ⁵⁵Co, ⁷²As, ⁷⁵Br, ⁷⁶Br, ^{82m}Rb, ⁸³Sr, or other gamma-, beta-, or positron-emitters. Paramagnetic ions of use may include chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III) or erbium (III). Metal contrast agents may include lanthanum (III), gold (III), lead (II) or bismuth (III). Ultrasound contrast agents may comprise liposomes, such as gas filled liposomes. Radiopaque diagnostic agents may be selected from compounds, barium compounds, gallium compounds, and thallium compounds. A wide variety of fluorescent labels are known in the art, including but not limited to fluorescein isothiocyanate, rhodamine, phycoerytherin, phycocyanin, allophycocyanin, o-phthaldehyde and fluorescamine. Chemiluminescent labels of use may include luminol, isoluminol, an aromatic acridinium ester, an imidazole, an acridinium salt or an oxalate ester.

### Methods of Therapeutic Treatment

According to the invention as defined in the claims, treatment of a cancer in a subject, such as a mammal, including humans, domestic or companion pets, such as dogs and cats, comprising administering to the subject a therapeutically effective amount of a multivalent complex comprising an anti-IGF-1R antibody or fragment.

It is disclosed that immunological diseases which may be treated with the subject anti-IGF-1R complexes may include, for example, joint diseases such as ankylosing spondylitis, juvenile rheumatoid arthritis, rheumatoid arthritis; neurological disease such as multiple sclerosis and myasthenia gravis; pancreatic disease such as diabetes, especially juvenile onset diabetes; gastrointestinal tract disease such as chronic active hepatitis, celiac disease, ulcerative colitis, Crohn's disease, pernicious anemia; skin diseases such as psoriasis or scleroderma; allergic diseases such as asthma and in transplantation related conditions such as graft versus host disease and allograft rejection.

The administration of the anti-IGF-1R complexes can be supplemented by administering concurrently or sequentially a therapeutically effective amount of another antibody that binds to or is reactive with another antigen on the surface of the target cell. Preferred additional antibodies comprise at least one humanized, chimeric or human antibody selected from the group consisting of an antibody reactive with CD4, CD5, CD8, CD14, CD15, CD16, CD19, IGF-1R, CD20, CD21, CD22, CD23, CD25, CD30, CD32b, CD33, CD37, CD38, CD40, CD40L, CD45, CD46, CD52, CD54, CD70, CD74, CD79a, CD80, CD95, CD126, CD133, CD138, CD154, CEACAM5, CEACAM6, B7, AFP, PSMA, EGP-1, EGP-2, carbonic anhydrase IX, PAM4 antigen, MUC1, MUC2, MUC3, MUC4, MUC5ac, la, MIF, HM1.24, HLA-DR, tenascin, Flt-3, VEGFR, P1GF, ILGF, IL-6, IL-25, tenascin, TRAIL-R1, TRAIL-R2, complement factor C5, or a combination thereof. Various antibodies of use, such as anti-CD 19, anti-CD20, and anti-CD22 antibodies, are known to those of skill in the art. See, for example, Ghetie et al., Cancer Res. 48:2610 (1988); Hekman et al., Cancer Immunol. Immunother. 32:364 (1991); Longo, Curr. Opin. Oncol. 8:353 (1996), U.S. Patent Nos. 5,798,554; 6,187,287; 6,306,393; 6,676,924; 7,109,304; 7,151,164; 7,230,084; 7,230,085; 7,238,785; 7,238,786; 7,282,567; 7,300,655; 7,312,318; 7,501,498; 7,612,180; 7,670,804; and U.S. Patent Application Publ. Nos. 20080131363; 20070172920; 20060193865; and 20080138333.

The therapy can be further supplemented with the administration, either concurrently or sequentially, of at least one therapeutic agent. For example, "CVB" (1.5 g/m² cyclophosphamide, 200-400 mg/m² etoposide, and 150-200 mg/m² carmustine) is a regimen used to treat non-Hodgkin's lymphoma. Patti et al., Eur. J. Haematol. 51: 18 (1993). Other suitable combination chemotherapeutic regimens are well-known to those of skill in the art. See, for example, Freedman et al., "Non-Hodgkin's Lymphomas," in CANCER MEDICINE, VOLUME 2, 3rd Edition, Holland et al. (eds.), pages 2028-2068 (Lea & Febiger 1993). As an illustration, first generation chemotherapeutic regimens for treatment of intermediate-grade non-Hodgkin's lymphoma (NHL) include C-MOPP (cyclophosphamide, vincristine, procarbazine and prednisone) and CHOP (cyclophosphamide, doxorubicin, vincristine, and prednisone). A useful second generation chemotherapeutic regimen is m-BACOD (methotrexate, bleomycin, doxorubicin, cyclophosphamide, vincristine, dexamethasone and leucovorin), while a suitable third generation regimen is MACOP-B (methotrexate, doxorubicin, cyclophosphamide, vincristine, prednisone, bleomycin and leucovorin). Additional useful drugs include phenyl butyrate, bendamustine, and bryostatin-1.

The subject anti-IGF-1R complexes can be formulated according to known methods to prepare pharmaceutically useful compositions, whereby the anti-IGF-1R complex is combined in a mixture with a pharmaceutically suitable excipient. Sterile phosphate-buffered saline is one example of a pharmaceutically suitable excipient. Other suitable excipients are well-known to those in the art. See, for example, Ansel et al., PHARMACEUTICAL DOSAGE FORMS AND DRUG DELIVERY SYSTEMS, 5th Edition (Lea & Febiger 1990), and Gennaro (ed.), REMINGTON'S PHARMACEUTICAL SCIENCES, 18th Edition (Mack Publishing Company 1990), and revised editions thereof.

The subject anti-IGF-1R complexes can be formulated for intravenous administration via, for example, bolus injection or continuous infusion. Preferably, the complex is infused over a period of less than about 4 hours, and more preferably, over a period of less than about 3 hours. For example, the first 25-50 mg could be infused within 30 minutes, preferably even 15 min, and the remainder infused over the next 2-3 hrs. Formulations for injection can be presented in unit dosage form, e.g., in ampoules or in multi-dose containers, with an added preservative. The compositions can take such forms as suspensions, solutions or emulsions in oily or aqueous vehicles, and can contain formulatory agents such as suspending, stabilizing and/or dispersing agents. Alternatively, the active ingredient can be in powder form for constitution with a suitable vehicle, e.g., sterile pyrogen-free water, before use.

Additional pharmaceutical methods may be employed to control the duration of action of the anti-IGF-1R complexes. Control release preparations can be prepared through the use of polymers to complex or adsorb the anti-IGF-1R complexes. For example, biocompatible polymers include matrices of poly(ethylene-co-vinyl acetate) and matrices of a polyanhydride copolymer of a stearic acid dimer and sebacic acid. Sherwood et al., Bio/Technology 10: 1446 (1992). The rate of release from such a matrix depends upon the molecular weight of the anti-IGF-1R complex, the amount of complex within the matrix, and the size of dispersed particles. Saltzman et al., Biophys. J. 55: 163 (1989); Sherwood *et al., supra.* Other solid dosage forms are described in Ansel et al., PHARMACEUTICAL DOSAGE FORMS AND DRUG DELIVERY SYSTEMS, 5th Edition (Lea & Febiger 1990), and Gennaro (ed.), REMINGTON'S PHARMACEUTICAL SCIENCES, 18th Edition (Mack Publishing Company 1990), and revised editions thereof.

The anti-IGF-1R complex may also be for administration to a mammal subcutaneously or even by other parenteral routes. Moreover, the administration may be by continuous infusion or by single or multiple boluses. Preferably, the complex is infused over a period of less than about 4 hours, and more preferably, over a period of less than about 3 hours.

More generally, the dosage of an administered anti-IGF-1R complex for humans will vary depending upon such factors as the patient's age, weight, height, sex, general medical condition and previous medical history. It may be desirable to provide the recipient with a dosage of anti-IGF-1R complex that is in the range of from about 1 mg/kg to 25 mg/kg as a single intravenous infusion, although a lower or higher dosage also may be administered as circumstances dictate. A dosage of 1-20 mg/kg for a 70 kg patient, for example, is 70-1,400 mg, or 41-824 mg/m² for a 1.7-m patient. The dosage may be repeated as needed, for example, once per week for 4-10 weeks, once per week for 8 weeks, or once per week for 4 weeks. It may also be given less frequently, such as every other week for several months, or monthly or quarterly for many months, as needed in a maintenance therapy.

Alternatively, an anti-IGF-1R complex may be for administration as one dosage every 2 or 3 weeks, repeated for a total of at least 3 dosages. Or, the construct may be administered twice per week for 4-6 weeks. If the dosage is lowered to approximately 200-300 mg/m² (340 mg per dosage for a 1.7-m patient, or 4.9 mg/kg for a 70 kg patient), it may be administered once or even twice weekly for 4 to 10 weeks. Alternatively, the dosage schedule may be decreased, namely every 2 or 3 weeks for 2-3 months. It has been determined, however, that even higher doses, such as 20 mg/kg once weekly or once every 2-3 weeks can be administered by slow i.v. infusion, for repeated dosing cycles. The dosing schedule can optionally be repeated at other intervals and dosage may be given through various parenteral routes, with appropriate adjustment of the dose and schedule.

According to the invention as defined in the claims, the anti-IGF-1R complexes are of use for therapy of cancer. Examples of cancers include, but are not limited to, carcinoma, lymphoma, glioblastoma, melanoma, sarcoma, and leukemia, myeloma, or lymphoid malignancies. More particular examples of such cancers are noted below and include: squamous cell cancer (e.g., epithelial squamous cell cancer), Ewing sarcoma, Wilms tumor, astrocytomas, lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma multiforme, cervical cancer, ovarian cancer, liver cancer, bladder cancer, hepatoma, hepatocellular carcinoma, neuroendocrine tumors, medullary thyroid cancer, differentiated thyroid carcinoma, breast cancer, ovarian cancer, colon cancer, rectal cancer, endometrial cancer or uterine carcinoma, salivary gland carcinoma, kidney or renal cancer, prostate cancer, vulvar cancer, anal carcinoma, penile carcinoma, as well as head-and-neck cancer. The term "cancer" includes primary malignant cells or tumors (e.g., those whose cells have not migrated to sites in the subject's body other than the site of the original malignancy or tumor) and secondary malignant cells or tumors (e.g., those arising from metastasis, the migration of malignant cells or tumor cells to secondary sites that are different from the site of the original tumor). Cancers conducive to treatment methods of the present disclosure involves cells which express, over-express, or abnormally express IGF-1R.

Other examples of cancers or malignancies include, but are not limited to: Acute Childhood Lymphoblastic Leukemia, Acute Lymphoblastic Leukemia, Acute Lymphocytic Leukemia, Acute Myeloid Leukemia, Adrenocortical Carcinoma, Adult (Primary) Hepatocellular Cancer, Adult (Primary) Liver Cancer, Adult Acute Lymphocytic Leukemia, Adult Acute Myeloid Leukemia, Adult Hodgkin's Lymphoma, Adult Lymphocytic Leukemia, Adult Non-Hodgkin's Lymphoma, Adult Primary Liver Cancer, Adult Soft Tissue Sarcoma, AIDS-Related Lymphoma, AIDS-Related Malignancies, Anal Cancer, Astrocytoma, Bile Duct Cancer, Bladder Cancer, Bone Cancer, Brain Stem Glioma, Brain Tumors, Breast Cancer, Cancer of the Renal Pelvis and Ureter, Central Nervous System (Primary) Lymphoma, Central Nervous System Lymphoma, Cerebellar Astrocytoma, Cerebral Astrocytoma, Cervical Cancer, Childhood (Primary) Hepatocellular Cancer, Childhood (Primary) Liver Cancer, Childhood Acute Lymphoblastic Leukemia, Childhood Acute Myeloid Leukemia, Childhood Brain Stem Glioma, Childhood Cerebellar Astrocytoma, Childhood Cerebral Astrocytoma, Childhood Extracranial Germ Cell Tumors, Childhood Hodgkin's Disease, Childhood Hodgkin's Lymphoma, Childhood Hypothalamic and Visual Pathway Glioma, Childhood Lymphoblastic Leukemia, Childhood Medulloblastoma, Childhood Non-Hodgkin's Lymphoma, Childhood Pineal and Supratentorial Primitive Neuroectodermal Tumors, Childhood Primary Liver Cancer, Childhood Rhabdomyosarcoma, Childhood Soft Tissue Sarcoma, Childhood Visual Pathway and Hypothalamic Glioma, Chronic Lymphocytic Leukemia, Chronic Myelogenous Leukemia, Colon Cancer, Cutaneous T-Cell Lymphoma, Endocrine Pancreas Islet Cell Carcinoma, Endometrial Cancer, Ependymoma, Epithelial Cancer, Esophageal Cancer, Ewing's Sarcoma and Related Tumors, Exocrine Pancreatic Cancer, Extracranial Germ Cell Tumor, Extragonadal Germ Cell Tumor, Extrahepatic Bile Duct Cancer, Eye Cancer, Female Breast Cancer, Gaucher's Disease, Gallbladder Cancer, Gastric Cancer, Gastrointestinal Carcinoid Tumor, Gastrointestinal Tumors, Germ Cell Tumors, Gestational Trophoblastic Tumor, Hairy Cell Leukemia, Head and Neck Cancer, Hepatocellular Cancer, Hodgkin's Lymphoma, Hypergammaglobulinemia, Hypopharyngeal Cancer, Intestinal Cancers, Intraocular Melanoma, Islet Cell Carcinoma, Islet Cell Pancreatic Cancer, Kaposi's Sarcoma, Kidney Cancer, Laryngeal Cancer, Lip and Oral Cavity Cancer, Liver Cancer, Lung Cancer, Lymphoproliferative Disorders, Macroglobulinemia, Male Breast Cancer, Malignant Mesothelioma, Malignant Thymoma, Medulloblastoma, Melanoma, Mesothelioma, Metastatic Occult Primary Squamous Neck Cancer, Metastatic Primary Squamous Neck Cancer, Metastatic Squamous Neck Cancer, Multiple Myeloma, Multiple Myeloma/Plasma Cell Neoplasm, Myelodysplastic Syndrome, Myelogenous Leukemia, Myeloid Leukemia, Myeloproliferative Disorders, Nasal Cavity and Paranasal Sinus Cancer, Nasopharyngeal Cancer, Neuroblastoma, Non-Hodgkin's Lymphoma, Nonmelanoma Skin Cancer, Non-Small Cell Lung Cancer, Occult Primary Metastatic Squamous Neck Cancer, Oropharyngeal Cancer, Osteo-/Malignant Fibrous Sarcoma, Osteosarcoma/Malignant Fibrous Histiocytoma, Osteosarcoma/Malignant Fibrous Histiocytoma of Bone, Ovarian Epithelial Cancer, Ovarian Germ Cell Tumor, Ovarian Low Malignant Potential Tumor, Pancreatic Cancer, Paraproteinemias, Polycythemia vera, Parathyroid Cancer, Penile Cancer, Pheochromocytoma, Pituitary Tumor, Primary Central Nervous System Lymphoma, Primary Liver Cancer, Prostate Cancer, Rectal Cancer, Renal Cell Cancer, Renal Pelvis and Ureter Cancer, Retinoblastoma, Rhabdomyosarcoma, Salivary Gland Cancer, Sarcoidosis Sarcomas, Sezary Syndrome, Skin Cancer, Small Cell Lung Cancer, Small Intestine Cancer, Soft Tissue Sarcoma, Squamous Neck Cancer, Stomach Cancer, Supratentorial Primitive Neuroectodermal and Pineal Tumors, T-Cell Lymphoma, Testicular Cancer, Thymoma, Thyroid Cancer, Transitional Cell Cancer of the Renal Pelvis and Ureter, Transitional Renal Pelvis and Ureter Cancer, Trophoblastic Tumors, Ureter and Renal Pelvis Cell Cancer, Urethral Cancer, Uterine Cancer, Uterine Sarcoma, Vaginal Cancer, Visual Pathway and Hypothalamic Glioma, Vulvar Cancer, Waldenstrom's Macroglobulinemia, Wilms'Tumor, and any other hyperproliferative disease, besides neoplasia, located in an organ system listed above.

The invention as defined in the claims is for use in a method for treating malignant or premalignant conditions and to prevent progression to a neoplastic or malignant state, including but not limited to those disorders described above. Such uses are indicated in conditions known or suspected of preceding progression to neoplasia or cancer, in particular, where non-neoplastic cell growth consisting of hyperplasia, metaplasia, or most particularly, dysplasia has occurred (for review of such abnormal growth conditions, see Robbins and Angell, Basic Pathology, 2d Ed., W. B. Saunders Co., Philadelphia, pp. 68-79 (1976)).

Dysplasia is frequently a forerunner of cancer, and is found mainly in the epithelia. It is the most disorderly form of non-neoplastic cell growth, involving a loss in individual cell uniformity and in the architectural orientation of cells. Dysplasia characteristically occurs where there exists chronic irritation or inflammation. Dysplastic disorders which can be treated include, but are not limited to, anhidrotic ectodermal dysplasia, anterofacial dysplasia, asphyxiating thoracic dysplasia, atriodigital dysplasia, bronchopulmonary dysplasia, cerebral dysplasia, cervical dysplasia, chondroectodermal dysplasia, cleidocranial dysplasia, congenital ectodermal dysplasia, craniodiaphysial dysplasia, craniocarpotarsal dysplasia, craniometaphysial dysplasia, dentin dysplasia, diaphysial dysplasia, ectodermal dysplasia, enamel dysplasia, encephalo-ophthalmic dysplasia, dysplasia epiphysialis hemimelia, dysplasia epiphysialis multiplex, dysplasia epiphysialis punctata, epithelial dysplasia, faciodigitogenital dysplasia, familial fibrous dysplasia of jaws, familial white folded dysplasia, fibromuscular dysplasia, fibrous dysplasia of bone, florid osseous dysplasia, hereditary renal-retinal dysplasia, hidrotic ectodermal dysplasia, hypohidrotic ectodermal dysplasia, lymphopenic thymic dysplasia, mammary dysplasia, mandibulofacial dysplasia, metaphysial dysplasia, Mondini dysplasia, monostotic fibrous dysplasia, mucoepithelial dysplasia, multiple epiphysial dysplasia, oculoauriculovertebral dysplasia, oculodentodigital dysplasia, oculovertebral dysplasia, odontogenic dysplasia, opthalmomandibulomelic dysplasia, periapical cemental dysplasia, polyostotic fibrous dysplasia, pseudoachondroplastic spondyloepiphysial dysplasia, retinal dysplasia, septo-optic dysplasia, spondyloepiphysial dysplasia, and ventriculoradial dysplasia.

Additional pre-neoplastic disorders which can be treated include, but are not limited to, benign dysproliferative disorders (e.g., benign tumors, fibrocystic conditions, tissue hypertrophy, intestinal polyps or adenomas, and esophageal dysplasia), leukoplakia, keratoses, Bowen's disease, Farmer's Skin, solar cheilitis, and solar keratosis.

In preferred embodiments, the invention as defined in the claims is used to inhibit growth, progression, and/or metastasis of cancers, in particular those listed above.

Additional hyperproliferative diseases, disorders, and/or conditions include, but are not limited to, progression, and/or metastases of malignancies and related disorders such as leukemia (including acute leukemias (e.g., acute lymphocytic leukemia, acute myelocytic leukemia (including myeloblastic, promyelocytic, myelomonocytic, monocytic, and erythroleukemia)) and chronic leukemias (e.g., chronic myelocytic (granulocytic) leukemia and chronic lymphocytic leukemia)), polycythemia vera, lymphomas (e.g., Hodgkin's disease and non-Hodgkin's disease), multiple myeloma, Waldenstrom's macroglobulinemia, heavy chain disease, and solid tumors including, but not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, emangioblastoma, acoustic neuroma, oligodendroglioma, meningioma, melanoma, neuroblastoma, and retinoblastoma.

Exemplary autoimmune diseases include acute idiopathic thrombocytopenic purpura, chronic immune thrombocytopenia, dermatomyositis, Sydenham's chorea, myasthenia gravis, systemic lupus erythematosus, lupus nephritis, rheumatic fever, polyglandular syndromes, bullous pemphigoid, pemphigus vulgaris, juvenile diabetes mellitus, Henoch-Schonlein purpura, post-streptococcal nephritis, erythema nodosum, Takayasu's arteritis, Addison's disease, rheumatoid arthritis, multiple sclerosis, sarcoidosis, ulcerative colitis, erythema multiforme, IgA nephropathy, polyarteritis nodosa, ankylosing spondylitis, Goodpasture's syndrome, thromboangitis obliterans, Sjögren's syndrome, primary biliary cirrhosis, Hashimoto's thyroiditis, thyrotoxicosis, scleroderma, chronic active hepatitis, polymyositis/dermatomyositis, polychondritis, pemphigus vulgaris, Wegener's granulomatosis, membranous nephropathy, amyotrophic lateral sclerosis, tabes dorsalis, giant cell arteritis/polymyalgia, pernicious anemia, rapidly progressive glomerulonephritis, psoriasis and fibrosing alveolitis.

### Expression Vectors

The disclosure also concerns DNA sequences comprising a nucleic acid encoding an antibody, antibody fragment, cytokine or constituent fusion protein of an anti-IGF-1R complex, such as a DNL construct. Fusion proteins may comprise an antibody or fragment or toxin attached to, for example, an AD or DDD moiety.

relate to expression vectors comprising the coding DNA sequences. The vectors may contain sequences encoding the light and heavy chain constant regions and the hinge region of a human immunoglobulin to which may be attached chimeric, humanized or human variable region sequences. The vectors may additionally contain promoters that express the encoded protein(s) in a selected host cell, enhancers and signal or leader sequences. Vectors that are particularly useful are pdHL2 or GS. More preferably, the light and heavy chain constant regions and hinge region may be from a human EU myeloma immunoglobulin, where optionally at least one of the amino acid in the allotype positions is changed to that found in a different IgG1 allotype, and wherein optionally amino acid 253 of the heavy chain of EU based on the EU number system may be replaced with alanine. See Edelman et al., Proc. Natl. Acad. Sci USA 63: 78-85 (1969). In other examples, an IgG1 sequence may be converted to an IgG4 sequence.

The skilled artisan will realize that methods of genetically engineering expression constructs and insertion into host cells to express engineered proteins are well known in the art and a matter of routine experimentation. Host cells and methods of expression of cloned antibodies or fragments have been described, for example, in U.S. Patent Nos. 7,531,327 and 7,537,930.

### Kits

The disclosure also concerns kits containing components suitable for treating or diagnosing diseased tissue in a patient. Exemplary kits may contain one or more anti-IGF-1R complexes as described herein. If the composition containing components for administration is not formulated for delivery via the alimentary canal, such as by oral delivery, a device capable of delivering the kit components through some other route may be included. One type of device, for applications such as parenteral delivery, is a syringe that is used to inject the composition into the body of a subject. Inhalation devices may also be used. In certain embodiments, a therapeutic agent may be provided in the form of a prefilled syringe or autoinjection pen containing a sterile, liquid formulation or lyophilized preparation.

The kit components may be packaged together or separated into two or more containers. In some embodiments, the containers may be vials that contain sterile, lyophilized formulations of a composition that are suitable for reconstitution. A kit may also contain one or more buffers suitable for reconstitution and/or dilution of other reagents. Other containers that may be used include, but are not limited to, a pouch, tray, box, tube, or the like. Kit components may be packaged and maintained sterilely within the containers. Another component that can be included is instructions to a person using a kit for its use.

### General techniques for construction of anti-IGF-1R antibodies

The Vκ (variable light chain) and V_{H} (variable heavy chain) sequences for anti-IGF-1R antibodies may be obtained by a variety of molecular cloning procedures, such as RT-PCR, 5'-RACE, and cDNA library screening. The V genes of an anti-IGF-1R MAb from a cell that expresses a murine anti-IGF-1R MAb can be cloned by PCR amplification and sequenced. To confirm their authenticity, the cloned V_{L} and V_{H} genes can be expressed in cell culture as a chimeric Ab as described by Orlandi et al., (Proc. Natl. Acad. Sci., USA, 86: 3833 (1989)). Based on the V gene sequences, a humanized anti-IGF-1R MAb can then be designed and constructed as described by Leung et al. (Mol. Immunol., 32: 1413 (1995)).

cDNA can be prepared from any known hybridoma line or transfected cell line producing a murine anti-IGF-1R MAb by general molecular cloning techniques (Sambrook et al., Molecular Cloning, A laboratory manual, 2nd Ed (1989)). The Vκ sequence for the MAb may be amplified using the primers VK1BACK and VK1FOR (Orlandi *et al.,* 1989) or the extended primer set described by Leung et al. (BioTechniques, 15: 286 (1993)). The V_{H} sequences can be amplified using the primer pair VH1BACK/VH1FOR (Orlandi *et al.,* 1989) or the primers annealing to the constant region of murine IgG described by Leung et al. (Hybridoma, 13:469 (1994)).

PCR reaction mixtures containing 10 µl of the first strand cDNA product, 10 µl of 10X PCR buffer [500 mM KCl, 100 mM Tris-HCl (pH 8.3), 15 mM MgCl₂, and 0.01% (w/v) gelatin] (Perkin Elmer Cetus, Norwalk, CT), 250 µM of each dNTP, 200 nM of the primers, and 5 units of Taq DNA polymerase (Perkin Elmer Cetus) can be subjected to 30 cycles of PCR. Each PCR cycle preferably consists of denaturation at 94°C for 1 min, annealing at 50°C for 1.5 min, and polymerization at 72°C for 1.5 min. Amplified Vκ and VH fragments can be purified on 2% agarose (BioRad, Richmond, CA). The humanized V genes can be constructed by a combination of long oligonucleotide template syntheses and PCR amplification as described by Leung et al. (Mol. Immunol., 32: 1413 (1995)).

PCR products for Vκ can be subcloned into a staging vector, such as a pBR327-based staging vector, VKpBR, that contains an Ig promoter, a signal peptide sequence and convenient restriction sites to facilitate in-frame ligation of the Vκ PCR products. PCR products for V_{H} can be subcloned into a similar staging vector, such as the pBluescript-based VHpBS. Individual clones containing the respective PCR products may be sequenced by, for example, the method of Sanger et al. (Proc. Natl. Acad. Sci., USA, 74: 5463 (1977)).

Expression cassettes containing the Vκ and V_{H} sequences, together with the promoter and signal peptide sequences, can be excised from VKpBR and VHpBS, respectively, by double restriction digestion as HindIII-BamHI fragments. The Vκ and V_{H} expression cassettes can be ligated into appropriate expression vectors, such as pKh and pG1g, respectively (Leung et al., Hybridoma, 13:469 (1994)). The expression vectors can be co-transfected into an appropriate cell, e.g., myeloma Sp2/0-Ag14 (ATCC, VA), colonies selected for hygromycin resistance, and supernatant fluids monitored for production of a chimeric, humanized or human anti-IGF-1R MAb by, for example, an ELISA assay. Alternatively, the Vκ and V_{H} expression cassettes can be assembled in the modified staging vectors, VKpBR2 and VHpBS2, excised as XbaI/BamHI and XhoI/BamHI fragments, respectively, and subcloned into a single expression vector, such as pdHL2, as described by Gillies et al. (J. Immunol. Methods 125:191 (1989) and also shown in Losman et al., Cancer, 80:2660 (1997)). Another vector that is useful is the GS vector, as described in Barnes et al., Cytotechnology 32:109-123 (2000). Other appropriate mammalian expression systems are described in Werner et al., Arzneim.-Forsch./Drug Res. 48(II), Nr. 8, 870-880 (1998).

Co-transfection and assay for antibody secreting clones by ELISA, can be carried out as follows. About 10 µg of VKpKh (light chain expression vector) and 20 µg of VHpG1g (heavy chain expression vector) can be used for the transfection of 5 X 10⁶ SP2/0 myeloma cells by electroporation (BioRad, Richmond, CA) according to Co et al., J. Immunol., 148: 1149 (1992). Following transfection, cells may be grown in 96-well microtiter plates in complete HSFM medium (Life Technologies, Inc., Grand Island, NY) at 37°C, 5% CO₂. The selection process can be initiated after two days by the addition of hygromycin selection medium (Calbiochem, San Diego, CA) at a final concentration of 500 units/ml of hygromycin. Colonies typically emerge 2-3 weeks post-electroporation. The cultures can then be expanded for further analysis. Transfectoma clones that are positive for the secretion of chimeric, humanized or human heavy chain can be identified by ELISA assay.

Antibodies can be isolated from cell culture media as follows. Transfectoma cultures are adapted to serum-free medium. For production of chimeric antibody, cells are grown as a 500 ml culture in roller bottles using HSFM. Cultures are centrifuged and the supernatant filtered through a 0.2 membrane. The filtered medium is passed through a protein A column (1 x 3 cm) at a flow rate of 1 ml/min. The resin is then washed with about 10 column volumes of PBS and protein A-bound antibody is eluted from the column with 0.1 M citrate buffer (pH 3.5) containing 10 mM EDTA. Fractions of 1.0 ml are collected in tubes containing 10 1 of 3 M Tris (pH 8.6), and protein concentrations determined from the absorbance at 280/260 nm. Peak fractions are pooled, dialyzed against PBS, and the antibody concentrated, for example, with the Centricon 30 (Amicon, Beverly, MA). The antibody concentration is determined by absorbance at 280 nm and its concentration adjusted to about 1 mg/ml using PBS. Sodium azide, 0.01% (w/v), is conveniently added to the sample as preservative.

### EXAMPLES

### Example 1. Generation and initial characterization of anti-IGF-1R antibodies: R1, cR1, and hR1

Three BALB/c mice were each immunized i.p. with 15 µg of recombinant human IGF-1R (R&D Systems, Catalog # 391-GR), comprising a mixture of both processed and unprocessed extracellular domain of human IGF-1R, in complete Freund's adjuvant. Additional immunizations in incomplete Freund's adjuvant were done 14,21, and 28 days after the initial immunization. Spleen cells from the immunized mice were fused with P3X63Ag8.653 cells to generate hybridomas according to standard protocols. One clone (C-11) expressing anti-IGF-1R but not anti-IR (insulin receptor) activity was isolated and expanded in cultures to obtain the mouse antibody designated ML04R1 or R1, which was shown to be an IgG1/*k* with the ability to inhibit the binding of radioiodinated IGF-1 to the IGF-1R expressing human breast cancer cell line MCF-7L (a subline of MCF-7) comparable to a commercially available mouse anti-IGF-1R monoclonal antibody (mAb) MAB391 **(Table 6).**

**Table 6. Binding of ¹²⁵I-IGF-1 to MCF-7L in the presence of MAB391 or R1**

| [Ab] | MAB391^{a} | R1 |
|---|---|---|
| 1000 ng/mL | 38% | 58 % |
| 100 ng/mL | 54% | 71 % |
| 10 ng/mL | 95% | 97% |
| 0 ng/mL | 100 % | 100 % |

| | | |
|---|---|---|
| ^{a}R&D clone 33255.111 | | |

To obtain cR1, the mouse-human chimeric mAb of R1, the V_{H} and V_{K} genes of R1 were cloned by 5'-RACE. The authenticity of the cloned V_{H} and V_{K} genes was confirmed by N-terminal protein sequencing that showed an exact match of the first 15 N-terminal amino acids with the corresponding amino acids deduced from DNA sequences (not shown). The cloned V_{H} and V_{K} genes were inserted into the *pdHL2* vector to generate *cR1pdHL2* (not shown), the expression vector for cR1.

cR1-producing clones were generated using SpE-26 (e.g., U.S. Patent No. 7,531,327), a variant of Sp2/0-Ag14 that shows improved growth properties, as host cells. Briefly, approximately 30 g of *cR1pdHL2* was linearized by digestion with SalI restriction endonuclease and transfected into SpE-26 cells by electroporation. The transfectants were selected with 0.075 µM methotrexate (MTX), and screened by ELISA for human Fc binding activities. The higher producing clones were further expanded to pick the two best clones (709.2D2 and 710.2G2), from which cR1 was produced in batch cultures, purified by Protein A, and each confirmed by ELISA to bind specifically to immobilized rhIGF-1R, but not to immobilized rhIR (not shown), with the same high affinity (K_{D} ∼0.1 nM) for immobilized rhIGF-1R (not shown). Surprisingly, cR1 appeared to have a higher affinity than R1 for rhIGF-1R immobilized onto polystyrene beads as shown by a competition assay in which the binding of R1 tagged with a fluorescent probe was measured by flow cytometry in the presence of varying concentrations of cR1 or R1 (not shown).

Successful humanization of cR1 to hR1 was achieved by grafting the CDRs onto the human framework regions of hMN-14 (U.S. Patent Nos. 5,874,540 and 6,676,924,) in which certain human framework residues were replaced with murine counterparts of R1 at corresponding positions. Other selected residues were substituted for cloning purposes, resulting in the amino acid sequences of hR1 V_{H} and hR1 V_{K} as shown in SEQ ID NO:94 and SEQ ID NO:95, respectively. Genes encoding hR1 V_{H} and hR1 Vk were then synthesized and engineered into *pdHL2* to obtain *hR1pdHL2,* the expression vector for hR1. Subsequent efforts to secure the production clone (711.3C11) for hR1 were similar to those describe above for cR1. Positive clones were selected for binding activity to rhIGF-1R. hR1 displayed virtually the same binding affinity as cR1 for rhIGF-1R immobilized on polystyrene beads (not shown).
hR1 VH
hR1 VK

To determine whether cR1 can block the binding of IGF-1 or IGF-2 to IGF-1R, we used polystyrene beads immobilized with rhIGF-1R as surrogates of cells expressing IGF-1R and performed the beads-competition assays as follows. Briefly, varying concentrations (0 to 670 nM) of cR1, IGF-1, or IGF-2 were mixed with a constant amount of ¹²⁵I-IGF-1 or ¹²⁵I-IGF-2. The rhIGF-1-coated beads were then added, incubated at room temperature for 1 h with gentle rocking, washed, and counted for radioactivity. The results indicated that cR1 failed to block the binding of either IGF-1 or IGF-2 to such immobilized rhIGF-1R under these conditions (not shown). The results of a similar experiment also indicated that binding of ¹²⁵I-IGF-1 to the bead-immobilized IGF-1R was effectively blocked by IGF-1 or MAB391, but not by hR1 or R1 (not shown). These findings suggest IGF-1 and MAB391 bind to the same epitope, or have overlapping epitopes of IGF-1R, and hR1 targets a different region of IGF-1R from MAB391 or IGF-1. As the primary binding site of IGF-1R for IGF-1 was reported to be located in the cysteine-rich (CR) domain between amino acids (aa) 223 and 274, and the same region (aa 223-274) has been assigned as the epitope to αIR-3, which like MAB391, competes for IGF-1 binding (Gustafson TA, Rutter WJ. J Biol Chem 1990; 265:18663-7), it appeared that MAB391 also binds to the same region or interacts with sites in close proximity.

### Example 2. Epitope mapping studies of R1, cR1, and hR1

To further locate the region of IGF-1R to which hR1 binds, a panel of commercially available anti-IGF-1R mAbs that have their epitopes to IGF-1R mapped, were evaluated for their ability to cross-block each other from binding to the IGF-1R-coated beads. The results showed that binding of R1 tagged with a fluorescent probe (PE) was not affected by MAB391 even at 100 µg/mL, and the binding of MAB391 tagged with PE was only partially inhibited (50 to 60%) by R1 at 100 µg/mL (not shown). Additional mapping studies indicate that the epitope of R1 is located in the CR domain between aa 151 and 282 and can be further located to the first half of the CR domain between aa 151 and 222 (not shown).

### Example 3. Additional characterization of R1, cR1, and hR1

Whereas IGF-1 stimulates proliferation of MCF-7 cells grown in serum-free medium, achieving a maximal effect of 50% increase in viable cell counts at 100 ng/mL when compared to the untreated control at 48 h, hR1 does not (not shown). Thus hR1 is not agonistic upon binding to IGF-1R. Internalization of hR1 into MCF-7 was observed at 37 °C but not at 4 °C (not shown).

### Example 4. Construction of expression vectors for hR1-IgG4(S228P) variant

B13-24 cells containing an IgG4 gene are purchased from ATCC (ATCC Number CRL-11397) and genomic DNA is isolated. Briefly, cells are washed with PBS, resuspended in digestion buffer (100 mM NaCl, 10 mM Tris-HCl pH8.0, 25 mM EDTA pH8.0, 0.5% SDS, 0.1 mg/ml proteinase K) and incubated at 50°C for 18 h. The sample is extracted with an equal volume of phenol/chloroform/isoamylalcohol and precipitated with 7.5 M NH.sub.4Ac/100% EtOH. Genomic DNA is recovered by centrifugation and dissolved in TE buffer. Using genomic DNA as template, the IgG4 gene is amplified by PCR using the following primers.
Primer-SacII
   CCGCGGTCACATGGCACCACCTCTCTTGCAGCTTCCACCAAGGGCCC (SEQ ID NO:96)
Primer-EagI:
   CCGGCCGTCGCACTCATTTACCCAGAGACAGGG (SEQ ID NO:97)

Amplified PCR product is cloned into a TOPO-TA sequencing vector (Invitrogen) and confirmed by DNA sequencing. The SacII-EagI fragment containing the heavy chain constant region of IgG1 in *hR1pdHL2* is replaced with SacII-EagI of the TOPO-TA-IgG4 plasmid to produce the hR1-pdHL2-IgG4 (*hR1pdHL2*-γ4) vector.

### IgG4-Proline mutation

A Ser228Pro mutation is introduced in the hinge region of IgG4 to avoid formation of half-molecules. A mutated hinge region 56 bp fragment (PstI-StuI) is synthesized
GAGTCCAAATATGGTCCCCCATGCCCACCGTGCCCAGGTAAGCCAACCCAGG (SEQ ID NO:98); annealed and replaced with the PstI-StuI fragment of IgG4. This construction results in a final vector *hR1pdHL2-*γ4P.

### Example 5. Generation of Multivalent hR1-based Antibodies by DNL

The DNL technique may be used to make multivalent, hR1-based antibodies in various formats that are either monospecific or bispecific. For certain preferred embodiments, Fab antibody fragments may be produced as fusion proteins containing either a DDD or AD sequence. Bispecific antibodies may be formed by combining a Fab-DDD fusion protein of a first antibody with a Fab-AD fusion protein of a second antibody. Alternatively, an IgG-AD module may be produced as a fusion protein and combined with a Fab-DDD module of the same or different specificity. Another alternative is a DDD-cytokine fusion, such as a DDD-interferon-a2b construct, combined with an anti-IGF-1R IgG-AD or Fab-AD construct. Additional types of constructs may be made that combine the targeting capabilities of an antibody with the effector function of any other protein or peptide.

Independent transgenic cell lines are developed for each DDD- or AD-fusion protein. Once produced, the modules can be purified if desired or maintained in the cell culture supernatant fluid. Following production, any Fab-AD or IgG-AD module can be combined with any DDD-module, or any Fab-DDD module may be combined with any AD-module. DDD- or AD-modules may be produced synthetically such as linkinging an AD-sequence to polyethylene glycol or a DDD-sequence to an oligonucleotide. For different types of constructs, different AD or DDD sequences may be utilized.
DDD1: SHIQIPPGLTELLQGYTVEVLRQQPPDLVEFAVEYFTRLREARA (SEQ ID NO:1)
DDD2: CGHIQIPPGLTELLQGYTVEVLRQQPPDLVEFAVEYFTRLREARA (SEQ ID NO:2)
AD1: QIEYLAKQIVDNAIQQA (SEQ ID NO:3)
AD2: CGQIEYLAKQIVDNAIQQAGC (SEQ ID NO:4)

The plasmid vector pdHL2 has been used to produce a number of antibodies and antibody-based constructs. See, Gillies et al., J Immunol Methods (1989), 125:191-202; Losman et al., Cancer (Phila) (1997), 80:2660-6. The di-cistronic mammalian expression vector directs the synthesis of the heavy and light chains of IgG. The vector sequences are mostly identical for many different IgG-pdHL2 constructs, with the only differences existing in the variable domain (V_{H} and V_{L}) sequences. Using molecular biology tools known to those skilled in the art, these IgG expression vectors can be converted into Fab-DDD, Fab-AD, or IgG-AD expression vectors, as described in detail below for Fab-DDD1 and Fab-AD1. To generate the expression vector for Fab-DDD1, the coding sequences for the hinge, C_{H}2 and C_{H}3 domains of the heavy chain are replaced with a sequence encoding the first 4 residues of the hinge, a 14 residue Gly-Ser linker and DDD1 (the first 44 residues of human RIIα). To generate the expression vector for Fab-AD1, the sequences for the hinge, C_{H}2 and C_{H}3 domains of IgG are replaced with a sequence encoding the first 4 residues of the hinge, a 15 residue Gly-Ser linker and AD1 (a 17 residue synthetic AD called AKAP-*IS*, which was generated using bioinformatics and peptide array technology and shown to bind RIIα dimers with a very high affinity (0.4 nM). See Alto, et al. Proc. Natl. Acad. Sci., U.S.A (2003), 100:4445-50).

To facilitate the conversion of IgG-pdHL2 vectors to either Fab-DDD 1 or Fab-AD1 expression vectors, two shuttle vectors were designed and constructed as follows.

### Preparation of C_{H}1

The C_{H}1 domain was amplified by PCR using the pdHL2 plasmid vector as a template. The left PCR primer consists of the upstream (5') end of the C_{H}1 domain and a SacII restriction endonuclease site, which is 5' of the C_{H}1 coding sequence. The right primer consists of the sequence coding for the first 4 residues of the hinge followed by four glycines and a serine (SEQ ID NO: 122), with the final two codons comprising a Bam HI restriction site.
*5' of C_{H}1 Left Primer*
   5'GAACCTCGCGGACAGTTAAG-3' (SEQ ID NO: 100)
*C_{H}1*+*G₄S-Bam Right* ("G₄S" disclosed as SEQ ID NO: 122)
   5'GGATCCTCCGCCGCCGCAGCTCTTAGGTTTCTTGTCCACCTTGGTGTTGCTGG-3' (SEQ ID NO:101)

The 410 bp PCR amplimer was cloned into the pGemT PCR cloning vector (Promega, Inc.) and clones were screened for inserts in the T7 (5') orientation.
**Construction of (G₄S)₂DDD1** ("(G₄S)₂" disclosed as SEQ ID NO: 123)

A duplex oligonucleotide, designated (G₄S)₂DDD1 ("(G₄S)₂" disclosed as SEQ ID NO: 123), was synthesized by Sigma Genosys (Haverhill, UK) to code for the amino acid sequence of DDD1 preceded by 11 residues of the linker peptide, with the first two codons comprising a BamHI restriction site. A stop codon and an EagI restriction site are appended to the 3'end. The encoded polypeptide sequence is shown below.
GSGGGGSGGGGSHIQIPPGLTELLQGYTVEVLRQQPPDLVEFAVEYFTRLREARA (SEQ ID NO:102)

The two oligonucleotides, designated RIIA1-44 top and RIIA1-44 bottom, that overlap by 30 base pairs on their 3' ends, were synthesized (Sigma Genosys) and combined to comprise the central 154 base pairs of the 174 bp DDD1 sequence. The oligonucleotides were annealed and subjected to a primer extension reaction with Taq polymerase.
*RIIA1-44 top*
*RIIA1-44 bottom*

Following primer extension, the duplex was amplified by PCR using the following primers:
*G4S Bam-Left* ("G4S" disclosed as SEQ ID NO: 122)
   5'-GGATCCGGAGGTGGCGGGTCTGGCGGAGGT-3' (SEQ ID NO:105)
*1-44 stop Eag Right*
   5'-CGGCCGTCAAGCGCGAGCTTCTCTCAGGCG-3' (SEQ ID NO:106)

This amplimer was cloned into pGemT and screened for inserts in the T7 (5') orientation.
**Construction of (G₄S)₂-AD1** ("(G₄S)₂" disclosed as SEQ ID NO: 123)

A duplex oligonucleotide, designated (G₄S)₂-AD1 ("(G₄S)₂" disclosed as SEQ ID NO: 123), was synthesized (Sigma Genosys) to code for the amino acid sequence of AD1 preceded by 11 residues of the linker peptide with the first two codons comprising a BamHI restriction site. A stop codon and an EagI restriction site are appended to the 3'end. The encoded polypeptide sequence is shown below.
GSGGGGSGGGGSQIEYLAKQIVDNAIQQA (SEQ ID NO:107)

Two complimentary overlapping oligonucleotides, designated AKAP-IS Top and AKAP-IS Bottom, were synthesized.
*AKAP-IS Top*
*AKAP-IS Bottom*

The duplex was amplified by PCR using the following primers:
*G4S Bam-Left* ("G4S" disclosed as SEQ ID NO: 122)
   5'-GGATCCGGAGGTGGCGGGTCTGGCGGAGGT-3' (SEQ ID NO:110)
*AKAP-IS stop Eag Right*
   5'-CGGCCGTCAGGCCTGCTGGATG-3' (SEQ ID NO:111)

This amplimer was cloned into the pGemT vector and screened for inserts in the T7 (5') orientation.

### Ligating DDD1 with CH1

A 190 bp fragment encoding the DDD1 sequence was excised from pGemT with BamHI and NotI restriction enzymes and then ligated into the same sites in C_{H}1-pGemT to generate the shuttle vector C_{H}1-DDD1-pGemT.

### Ligating AD1 with CH1

A 110 bp fragment containing the AD1 sequence was excised from pGemT with BamHI and NotI and then ligated into the same sites in CH1-pGemT to generate the shuttle vector CH1-AD1-pGemT.

### Cloning CH1-DDD1 or CH1-AD1 into pdHL2-based vectors

With this modular design either C_{H}1-DDD1 or CH1-AD1 can be incorporated into any IgG- pdHL2 vector. The entire heavy chain constant domain is replaced with one of the above constructs by removing the SacII/EagI restriction fragment (C_{H}1-C_{H}3) from pdHL2 and replacing it with the SacII/EagI fragment of C_{H}1-DDD1 or C_{H}1-AD1, which is excised from the respective pGemT shuttle vector.

### CH1-DDD2-Fab-hR1-pdHL2

C_{H}1-DDD2-Fab-hR1-pdHL2 is an expression vector for production of C_{H}1-DDD2-Fab-hR1, which possesses a dimerization and docking domain sequence of DDD2 appended to the carboxyl terminus of the Fd via a 14 amino acid residue Gly/Ser peptide linker.

The expression vector was engineered as follows. Two overlapping, complimentary oligonucleotides, which comprise the coding sequence for part of the linker peptide (GGGGSGGGCG, SEQ ID NO:112) and residues 1 - 13 of DDD2, were made synthetically. The oligonucleotides were annealed and phosphorylated with T4 PNK, resulting in overhangs on the 5' and 3' ends that are compatible for ligation with DNA digested with the restriction endonucleases BamHI and PstI, respectively.
*G4S-DDD2 top* ("G4S" disclosed as SEQ ID NO: 122)
*G4S-DDD2 bottom* ("G4S" disclosed as SEQ ID NO: 122)

The duplex DNA was ligated with the shuttle vector CH1-DDD1-pGemT, which was prepared by digestion with BamHI and PstI, to generate the shuttle vector CH1-DDD2-pGemT. A 507 bp fragment was excised from CH1-DDD2-pGemT with SacII and EagI and ligated with the IgG expression vector *hR1pdHL2,* which was prepared by digestion with SacII and EagI. The final expression construct is C_{H}1-DDD2-Fab-hR1-pdHL2.

### Generation of C_{H}1-AD2-Fab-h679-pdHL2

C_{H}1-AD2-Fab-h679-pdHL2 is an expression vector for the production of C_{H1}-AD2-Fab-h679 and is useful as a template for the DNA sequence encoding AD2. The expression vector is engineered as follows. Two overlapping, complimentary oligonucleotides (AD2 Top and AD2 Bottom), which comprise the coding sequence for AD2 and part of the linker sequence, are made synthetically. The oligonucleotides are annealed and phosphorylated with T4 polynucleotide kinase, resulting in overhangs on the 5' and 3' ends that are compatible for ligation with DNA digested with the restriction endonucleases BamHI and SpeI, respectively.
*AD2 Top*
*AD2 Bottom*

The duplex DNA is ligated into the shuttle vector C_{H}1-AD1-pGemT, which is prepared by digestion with BamHI and SpeI, to generate the shuttle vector CH1-AD2-pGemT. A 429 base pair fragment containing C_{H}1 and AD2 coding sequences is excised from the shuttle vector with SacII and EagI restriction enzymes and ligated into h679-pdHL2 vector that is prepared by digestion with those same enzymes, resulting in C_{H}1-AD2-Fab-h679-pdHL2.

### Generation of C_{H}3-AD2-IgG-pdHL2 for expressing C_{H3}-AD2-IgG

C_{H}3-AD2-IgG modules have an AD2 peptide fused to the carboxyl terminus of the heavy chain of IgG via a 9 amino acid residue peptide linker. The DNA coding sequences for the linker peptide (GSGGGGSGG, SEQ ID NO:117) followed by the AD2 peptide are coupled to the 3' end of the C_{H}3 (heavy chain constant domain 3) coding sequence by standard recombinant DNA methodologies, resulting in a contiguous open reading frame. When the heavy chain-AD2 polypeptide is co-expressed with a light chain polypeptide, an IgG molecule is formed possessing two AD2 peptides, which can therefore bind two Fab-DDD2 dimers. The C_{H}3-AD2-IgG module can be combined with any C_{H}1-DDD2-Fab module to generate a wide variety of hexavalent structures composed of an Fc fragment and six Fab fragments. If the C_{H}3-AD2-IgG module and the C_{H}1-DDD2-Fab module are derived from the same parental monoclonal antibody (MAb) the resulting complex is monospecific with 6 binding arms to the same antigen. If the modules are instead derived from two different MAbs then the resulting complexes are bispecific, with two binding arms for the specificity of the C_{H}3-AD2-IgG module and 4 binding arms for the specificity of the C_{H}1-DDD2-Fab module.

A plasmid shuttle vector was produced to facilitate the conversion of any IgG-pdHL2 vector into a C_{H}3-AD2-IgG-pdHL2 vector. The gene for the Fc (C_{H}2 and C_{H}3 domains) was amplified using the pdHL2 vector as a template and the oligonucleotides *Fc BglII Left* and *Fc Bam-EcoRI Right* as primers.
*Fc BglII Left*
   5'-AGATCTGGCGCACCTGAACTCCTG-3' (SEQ ID NO:118)
*Fc Bam-EcoRI Right*
   5'-GAATTCGGATCCTTTACCCGGAGACAGGGAGAG-3' (SEQ ID NO:119)

The amplimer was cloned in the pGemT PCR cloning vector. The Fc insert fragment was excised from pGemT with XbaI and BamHI restriction enzymes and ligated with AD2-pdHL2 vector that was prepared by digestion of C_{H}1-AD2-Fab-h679-pdHL2 with Xbal and BamHI, to generate the shuttle vector Fc-AD2-pdHL2.

To convert any IgG-pdHL2 expression vector to a C_{H}3-AD2-IgG-pdHL2 expression vector, an 861 bp BsrGI / NdeI restriction fragment is excised from the former and replaced with a 952 bp BsrGI / NdeI restriction fragment excised from the Fc-AD2-pdHL2 vector. BsrGI cuts in the C_{H}3 domain and NdeI cuts downstream (3') of the expression cassette.

### Example 6. Generation of Hex-hRl

The DNL method was used to create Hex-hRl, a monospecific anti-IGF-1R with one Fc and six Fabs, by combining C_{H3}-AD2-IgG-hR1 with C_{H}1-DDD2-Fab-hR1. Hex-hRl was made in four steps.

*Step1, Combination:* C_{H}1-DDD2-Fab-hR1 was mixed with C_{H}3-AD2-IgG-hR1 in phosphate buffered saline, pH 7.4 (PBS) with 1 mM EDTA, at a molar ratio of 4.2 such that there are two C_{H}1-DDD2-Fab-hR1 for each AD2 on C_{H}3-AD2-IgG-hR1, allowing some excess of C_{H}1-DDD2-Fab-hR1 to ensure that the coupling reaction was complete.

*Step 2, Mild Reduction*: Reduced glutathione (GSH) was added to a final concentration of 1 mM and the solution held at room temperature (16 - 25°C) for 1 to 24 hours.

*Step 3, Mild Oxidation*: Following reduction, oxidized glutathione (GSSH) was added directly to the reaction mixture to a final concentration of 2 mM and the solution was held at room temperature for 1 to 24 hours.

*Step 4, Isolation of the DNL product:* Following oxidation, the reaction mixture was loaded directly onto a Protein-A affinity chromatography column. The column was washed with PBS and the Hex-hRl eluted with 0.1 M Glycine, pH 2.5. The unreacted C_{H}1-DDD2-Fab-hR1 was removed from the desired product in the unbound fraction. Other hexavalent DNL constructs can be prepared similarly by mixing a selected pair of C_{H}3-AD2-IgG and C_{H}1-DDD2-Fab.

A list of such DNL constructs and structural controls related to the present invention is provided in **Table 7.** Each of these constructs was shown to retain the binding activities of the constitutive antibodies.

**Table 7. hR1-containing DNL constructs and structural controls**

| **DNL code** | **IgG-AD2** | **Fab-DDD2** | **Valency** | | |
|---|---|---|---|---|---|
| | | | 2 | 4 | 6 |
| Hex-hRl | hR1 | hR1 | - | - | IGF-1R |
| Hex-hRS7 | hRS7 | hRS7 | - | - | EGP-1 |
| Hex-hPAM4 | hPAM4 | hPAM4 | - | - | mucin |
| Hex-hMN-14 | hMN-14 | hMN14 | - | - | CEACAM5 |
| Hex-hLL1 | hLL1 | hLL1 | - | - | CD74 |
| Hex-hL243 | hL243 | hL243 | - | - | HLA-DR |
| 1R-E1 | hR1 | hRS7 | IGF-1R | EGP-1 | - |
| 1R-14 | hR1 | hMN-14 | IGF-1R | CEACAM5 | - |
| 1R-15 | hR1 | hMN-15 | IGF-1R | CEACAM6 | - |
| 1R-31 | hR1 | hAFP | IGF-1R | AFP | - |
| 1R-74 | hR1 | hLL1 | IGF-1R | CD74 | - |
| 1R-C2 | hR1 | hL243 | IGF-1R | HLA-DR | - |
| 1R-M1 | hR1 | hPAM4 | IGF-1R | mucin | |
| E1-1R | hRS7 | hR1 | EGP-1 | IGF-1R | - |
| M1-1R | hPAM4 | hR1 | mucin | IGF-1R | - |
| 14-1R | hMN-14 | hR1 | CEACAM5 | IGF-1R | - |
| 74-1R | hLL1 | hR1 | CD74 | IGF-1R | - |
| C2-1R | hL243 | hR1 | HLA-DR | IGF-1R | - |
| 22-20 | hLL2 | hA20 | CD22 | CD20 | - |

### Example 7. Production of AD- and DDD-linked Fab and IgG Fusion Proteins From Multiple Antibodies

The following IgG or Fab fusion proteins were constructed and incorporated into DNL constructs, retaining the antigen-binding characteristics of the parent antibodies.

**Table 8. Fusion proteins comprising IgG or Fab Moieties**

| **Fusion Protein** | **Binding Specificity** |
|---|---|
| **C-AD1-Fab-h679** | **HSG** |
| **C-AD2-Fab-h679** | **HSG** |
| **C-(AD)₂-Fab-h679** | **HSG** |
| **C-AD2-Fab-h734** | **Indium-DTPA** |
| **C-AD2-Fab-hA20** | **CD20** |
| **C-AD2-Fab-hA20L** | **CD20** |
| **C-AD2-Fab-hL243** | **HLA-DR** |
| **C-AD2-Fab-hLL2** | **CD22** |
| **N-AD2-Fab-hLL2** | **CD22** |
| **C-AD2-IgG-hMN-14** | **CEACAM5** |
| **C-AD2-IgG-hR1** | **IGF-1R** |
| **C-AD2-IgG-hRS7** | **EGP-1** |
| **C-AD2-IgG-hPAM4** | **MUC** |
| **C-AD2-IgG-hLL1** | **CD74** |
| | |
| **C-DDD1-Fab-hMN-14** | **CEACAM5** |
| **C-DDD2-Fab-hMN-14** | **CEACAM5** |
| **C-DDD2-Fab-h679** | **HSG** |
| **C-DDD2-Fab-hA19** | **CD19** |
| **C-DDD2-Fab-hA20** | **CD20** |
| **C-DDD2-Fab-hAFP** | **AFP** |
| **C-DDD2-Fab-hL243** | **HLA-DR** |
| **C-DDD2-Fab-hLL1** | **CD74** |
| **C-DDD2-Fab-hLL2** | **CD22** |
| **C-DDD2-Fab-hMN-3** | **CEACAM6** |
| **C-DDD2-Fab-hMN-15** | **CEACAM6** |
| **C-DDD2-Fab-hPAM4** | **MUC** |
| **C-DDD2-Fab-hR1** | **IGF-1R** |
| **C-DDD2-Fab-hRS7** | **EGP-1** |
| **N-DDD2-Fab-hMN-14** | **CEACAM5** |

### Example 8. IGF-1R expression in cancer cell lines

Zenon-labeled various parental antibodies as well as multivalent antibodies derived from these antibodies were used to assess the expression levels of cognate antigens in several cancer cell lines by flow cytometry performed on Guava instrument. Expression of IGF-1R was confirmed by the binding of hR1 to MCF-7 (breast cancer), CaPan1 (pancreatic cancer), and DU-145 (prostate cancer) (not shown). The dual expression of IGF-1R and AFP in HepG2 (liver cancer) was also shown by the binding of humanized anti-AFP IgG and TF18 (made by combining C_{H}1-DDD2-Fab-hAFP with C_{H1}-AD2-Fab-h679 to contain two Fab fragments of hAFP), as well as by the enhanced binding of hR1-IgG-AD2 (the dimer of C_{H}3-AD2-IgG-hR1) and 1R-31, suggesting a higher affinity of these multivalent DNL constructs (not shown). The expression of CEACAM6 in Hep G2 was evidenced by the enhanced binding of 1R-15. Additional studies performed with MCF-7, DU-145, and ME-180 (cervical cancer) on FACSCAN™ are summarized in **Table 9,** which shows that the multivalent DNL constructs exhibit enhanced binding to target cell lines compared to their parental antibodies.

| **Table 9. Flow cytometry data obtained from FACS can** | | | | | | | | |
|---|---|---|---|---|---|---|---|---|
| **MCF-7** | | | **DU 145** | | | **ME-180** | | |
| *Antibody* | *MFI* | % *Positive* | *Antibody* | *MFI* | *% Positive* | *Antibody* | *MFI* | % *Positive* |
| | / | / | - | 2.4 | 1.96 | - | 1.86 | 2 |
| human IgG | 1.84 | 2.34 | human IgG | 2.21 | 1.44 | human IgG | 1.8 | 1.37 |
| 22-20 | 2.11 | 3.1 | DNLI | 2.74 | 7.88 | DNL1 | 1.98 | 12.6 |
| hR1 | 9.93 | 89.15 | hR1 | 5.33 | 30.39 | hR1 | 3.65 | 10.74 |
| hRS7 | 21.42 | 99.15 | hRS7 | 10.58 | 82.82 | hRS7 | 35.54 | 99.96 |
| Hex-hR1 | 14.08 | 98.58 | Hex-hR1 | 7.83 | 72.56 | Hex-hR1 | 6.36 | 33.02 |
| Hex-hRS7 | 35.73 | 99.86 | Hex-hRS7 | 17.03 | 93.74 | Hex-hRS7 | 59.58 | 99.95 |
| 1R-E1 | 47.85 | 99.92 | 1R-E1 | 22.10 | 99.53 | 1R-E1 | 76.29 | 99.94 |
| E1-1R | 109.19 | 98.77 | E1-1R | 53.96 | 99.9 | E1-1R | 254.8 | 99.89 |

### Example 9. Neutralizing activity of Hex-hRl and 1R-E1

The following experiments were performed to determine the effect of Hex-hRl or 1RE1 on neutralizing the growth stimulating activity of IGF-1 in DU-145 and ME-180, both of which express IGF-1R and EGP-1. Target cells were seeded at 2000/well onto 96-well plates and grown overnight in complete medium. Cells were washed twice with serum free medium and exposed to a selected multivalent antibody at 0.8, 4, 20, and 100 µg/mL in serum free medium for 2 h, followed by the addition of IGF-1 to a final concentration of 10 ng/ml. Cells were incubated for 72 hours and then subjected to MTS assay. Under these conditions, Hex-hR1 suppressed the proliferation of DU-145 and ME-180 in a dose-dependent manner (not shown) with statistical significance. Similar results were obtained with 1R-E1 in ME-180 (not shown).

### Example 10. Downregulation of IGF-1R

One major mechanism of anti-tumor actions induced by an anti-IGF-1R antibody, despite its being an agonist or antagonist, is to downregulate IGF-1R via endocytosis leading to subsequent degradation in endosomal vesicles. Efficient downregulation of IGF-1R in MCF-7 or HT-29 (colorectal cancer) was clearly demonstrated with hR1 at 100 nM as well as the two commercially available anti-IGF-1R antibodies (MAB391 and 24-60) serving as positive controls, but not with the anti-CD22 antibody, hLL2 (epratuzumab), which serves as a negative control (not shown). Further studies revealed that Hex-hRl and 1R-E1 were capable of substantially reducing the level of IGF-1R at a concentration as low as 0.1 nM in MCF-7, DU-145, and LnCap (androgen-dependent prostate cancer) (not shown).

### Example 11. Anti-Tumor Effects of Multivalent Anti-IGF-1R Complexes Are Enhanced In Renal Cell Carcinoma and Synergistic With an mTOR Inhibitor

Among kidney cancer types, approximately 90% are renal cell carcinomas (RCC). Advanced or metastatic RCC, which presents in about one third of the patients, has a poor prognosis, because it is resistant to conventional chemotherapy or radiotherapy. Treatments with human interferon-α2b (IFN-α2b) alone or in combination with mTOR inhibitors such as rapamycin have led to only modest improvements in outcome. One observation made with mTOR inhibitors is that cancer cells can overcome the effects of the inhibitor by activating the insulin-like growth factor-I (IGF-I) signaling pathways. Clinically, there is an association of IGF-I receptor (IGF-IR) expression in RCC and poor long-term patient survival, particularly among patients with high-grade tumors.

A humanized anti-IGF-IR monoclonal antibody, hR1, binds to multiple tumor types, including RCC, resulting in effective down-regulation of IGF-IR and moderate inhibition of cell proliferation *in vitro.* To enhance the anti-tumor activity of hR1, we generated the DOCK-AND-LOCK™ (DNL™) complex 1R-2b, comprising a conjugate of hR1 IgG with two dimers of interferon-α2b, and Hex-hRl, comprising 6 Fab fragments of hR1 tethered onto a common Fc. To make Hex-R1, a dimerization and docking domain (DDD) was fused to hR1 Fab to produc a self-associating dimeric Fab-DDD2. An anchor domain (AD) was fused to the two CH3 domains of hR1 IgG to produce a CH3-AD2-IgG molecule with two AD peptides. Final assembly was readily obtained by mixing the Fab-DDD2 with the CH3-AD2-IgG under mild redox conditions, to produce a DNL™ complex comprising four Fab moieties attached to an IgG moiety. To produce 1R-2b, DDD2 was fused to human IFN-a2b and the DDD2- IFN-a2b moiety was mixed with a CH3-AD2-IgG molecule under mild redox conditions.

There was no loss in cell binding for either 1R-2b or Hex-hRl when compared to parental hR1 as determined by flow cytometry (not shown). The IFN-α specific activity was measured at 3750 U/pmole for 1R-2b *versus* 180 U/pmole and 3255 U/pmole for two different forms of peginterferon alpha-2 (60 and 31 kDa), respectively with a luciferase reporter gene fused to a promoter containing the interferon-stimulated response element (iLite kit).

An *in vitro* cytotoxicity assay with 1R-2b demonstrated growth inhibition of two different RCC cell lines, 786-0 and ACHN, with EC50-values of 0.049 and 0.062 pmole/mL, respectively **(****FIG. 1****).** Hex-hRl induced the down-regulation of IGF-IR at 10-fold lower concentrations compared to the parental hR1 IgG **(****FIG. 1****).** In soft-agar growth assays, all three agents (hR1, Hex-hRl and 1R-2b) significantly inhibited colony formation of 786-0 and ACHN (P<0.038 and P<0.0022, respectively) **(****FIG. 1****). Table 10** summarizes the data on growth inhibition by different forms of anti-IGF-1R for Caki-2 cells, ACHN cells and 786-0 cells.

**Table 10. Maximum growth inhibition by Anti-IGF-1R under serum-free conditions.**

| Data were obtained from the experiments shown in FIG. 1. Concentration at maximum inhibition in parentheses. | | | |
|---|---|---|---|
| **Antibody** | **Caki-2 Cells** (8 nM) | **ACHN Cells** (200 nM) | **786-O Cells** (8 nM) |
| **hR1** | 33 ± 0.6% | 3 ± 4.4% | 26 ± 5.4% |
| **Hex-hRl** | *43 ± 0.3% | *48 ± 1.9% | 35 ± 4.8% |
| **MAB 391** | *50 ± 4.7% | *48 ± 13.2% | 34 ± 2.0% |

| | | | |
|---|---|---|---|
| *Significantly different from hR1 (*P*<0.001) | | | |

The activity of the 1R-2b DNL™ complex, comprising four copies of IFN-α2b attached to hR1 IgG, was examined. Based on a luciferase reporter gene assay (iLite kit), 1R-2b yielded a specific activity of 15x10⁶ U/mg or 3750 U/pmole versus 180 and 3255 U/pmole for two different pegylated-IFN molecules **(****FIG. 2A****).** In growth inhibition assays of 786-O **(****FIG. 2B****)** and ACHN **(****FIG. 2C****),** 1R-2b had EC50 values of 49 and 62 pM, respectively. Confirmation of units of activity was further demonstrated in IFN-mediated phosphorylation of STAT1. Cells were plated in 6-well plates overnight. On the following day IFN, either in the form of rhIFN-a2a or 1R-2b, was added at 100, 10 and 1 U/ml of IFN. After 30 min, cell lysates were prepared and resolved by SDS-PAGE, transferred to nitrocellulose membranes and probed with antibodies to phospho-STATl. ACHN cells showed similar levels of pSTAT1 for 1R-2b and rhIFN-a2a at each of the three different concentrations added to the plates (not shown). The ratios of pSTAT1 relative to untreated (β-actin control) for were respectively 13.1 (rhIFN-α at 100 U/ml IFN), 2.3 (rMFN-α at 10 U/ml IFN), 1.3 (rhIFN-α at 1 U/ml IFN), 13.7 (1R-2b at 100 U/ml IFN), 2.4 (1R-2b at 10 U/ml IFN), 1.1 (1R-2b at 1 U/ml IFN) and 1.0 (control). 1R-2b and rhIFN-a2a had similar effects in 786-O cells (not shown).

The effect of hR1 constructs on growth inhibition under anchorage-independent conditions was examined **(****FIG. 3****).** A 1% base agar was mixed 1:1 with 2x growth media (10% FBS final concentration) and added to wells of a 24-well plate. Cells in 2x growth media were mixed 1:1 with 0.7% agarose and added (1250 cells per well) to the base agar. Cells were fed by weekly replacement of growth media on the top of the agarose layer. Treated wells contained the test articles in the agarose/cell layer at the beginning and in subsequent feedings. Once colonies were clearly visible by microscopy in untreated control wells, the medium was removed and the colonies stained with crystal violet. Colonies were counted under a microscope and the average number was determined from five different fields of view within the well. Each of the hR1 constructs induced significant growth inhibition of ACHN cells under anchorage-independent conditions **(****FIG. 3****).** Both Hex-hR1 and 1R-2b showed significantly greater growth inhibition compared to unconjugated hR1, with the greatest effect shown by the IFN-α2b DNL™ construct **(****FIG. 3****).** hR1 IgG and 1R-2b, but not Hex-hRl, signicantly inhibited anchorage-independent growth of 786-0 cells, with the greatest effect again observed with the IFN-α2b DNL™ construct **(****FIG. 3****).**

When combined with temsirolimus, an mTOR inhibitor, *in vitro* cytotoxicity assays demonstrated a synergistic interaction with hR1, Hex-hRl, and 1R-2b. This synergy occurred at concentrations as low as 10 nM for hR1, 1 nM for Hex-hR1, and 2.6 nM for 1R-2b **(****FIG. 4****).** ACHN cells were harvested, washed in PBS several times to remove FBS, and plated in 96-wells plates overnight in SFM. On the following day, various doses (1 mM to 0.06 nM) of the mTOR inhibitor temsirolimus was added to the plates with and without hR1 or Hex-hR1 (100, 10, and 1 nM constant amounts) or 1R-2b (26, 2.6, or 0.26 nM; NOTE: 26 nM 1R-2b -100,000 Units/mL of IFN). IGF-1 was added at 100 ng/mL. Plates were incubated for 96-h before MTS substrate was added to all the wells and the plates read at 492nm. Data was graphed as Percent Growth Inhibition vs. [temsirolimus]. IC50-values for temsirolimus were determined for each condition and Combinatorial Index (CI) was calculated based on changes in these values when co-incubated with hR1, Hex-hRl, or 1R-2b (CI<1 for synergy). **FIG. 4A** shows the effect of the combination of temsirolimus with hR1 (CI = 0.64). The IC₅₀ values for temsirolimus concentration needed to mediate 50% inhibition of cell growth were 7.76 nM for Tem alone (R² 0.94); 1.45 nM with 100 nM hR1 (R² 0.88); 0.56 nM with 10 nM hR1 (R² 0.84); and 2.86 nM with 1 nM hR1 (R² 0.93). **FIG. 4B** shows the effect of the combination of temsirolimus with Hex-hR1 (CI = 0.43). The IC₅₀ values were 7.76 nM for Tem alone (R² 0.94); 3.15 nM with 1 nM Hex-hRl (R² 0.63); 0.06 nM with 10 nM Hex-hRl (R² 0.66); and <0.06 nM with 100 nM HexhRl (R² 0.63). **FIG. 4C** shows the effect of the combination of temsirolimus with 1R-2b (CI = 0.02). The IC₅₀ values were 7.76 nM for Tem alone (R² 0.94); <0.06 nM with 26 nM 1R-2b (R² 0.32); <0.06 nM with 2.6 nM 1R-2b (R² 0.34); and 12.7 nM with 0.26 nM 1R-2b (R² 0.81).

In conclusion, two novel anti-IGF-1R DNL™ complexes were created for the treatment of RCC. Both Hex-hRl and 1R-2b retained anti-IGF-1R binding capacity to target cells, similar to that of the parental hR1. Hex-hRl mediated complete receptor down-regulation at concentrations as low as 1 nM *versus* 10 nM for hR1. 1R-2b demonstrated signaling activity similar to rhIFN-a2a in two different RCC cell lines. Both Hex-hRl and 1R-2b inhibit the growth of RCC tumor lines *in vitro* and under anchorage-independent growth in soft-agar. Synergistic interactions were observed with these anti-IGF-1R molecules when combined with the mTOR inhibitor, temsirolimus. These data suggest that while each agent shows activity when used alone, a greater benefit might be achieved when combined with an mTOR inhibitor.

### Example 12. Bispecific, Hexavalent Antibodies Targeting IGF-1R and Trop-2 or CEACAM6 Inhibit Anchorage-Independent Growth and Invasion of Breast and Pancreatic Cancer Cell Lines

Combination therapy using two distinct monoclonal antibodies to achieve improved efficacy without increased toxicity is being pursued in various preclinical and clinical studies. Preferably, this may be accomplished with a single bispecific antibody to avoid the need for administering two antibodies sequentially, which is time consuming, expensive and inconvenient. Based on the elevated expression of the type I insulin-like growth factor receptor (IGF-1R), the trophoblast cell-surface marker (Trop-2), and the carcinoembryonic antigen related cell adhesion molecule 6 (CEACAM6) in diverse epithelial cancer cell lines, we explored the potential of 1R-(E1)-(E1) and 1R-(15)-(15), two novel bispecific HexAbs targeting IGF-1R/Trop-2 and IGF-1R/CEACAM6, respectively, for treating breast and pancreatic cancers.

1R-(E1)-(E1), comprising the IgG of hR1 (humanized anti-IGF-1R) and four Fabs of hRS7 (humanized anti-Trop-2) was generated as a DNL™ complex by reacting the IgG-AD2 module of hR1 with the Fab-DDD2 module of hRS7 under mild redox conditions, followed by purification on Protein A. 1R-(15)-(15) was generated in a similar fashion using the Fab-DDD2 module of hMN-15 (humanized anti-CEACAM6).

The *in vitro* effects of 1R-(E1)-(E1) and 1R-(15)-(15) on three triple-negative breast cancer lines of varying invasive activities (MCF-7, low; MDA-MB-468, moderate; MDA-MB-231, high) and two pancreatic cancer lines (Capan-1 and BxPC-3) were determined, which included cell binding by flow cytometry, anchorage-independent growth by soft agar assay, and invasiveness by BD matrigel chambers. Statistical differences (*P* values) between two populations were determined by Student's t-test.

All five cell lines were found to express IGF-1R, Trop-2, and CEACAM6 of sufficient levels, which are higher in MCF-7, MDA-MB-468, and BxPC-3, respectively (not shown). When tested at 100 µg/mL, 1R-(E1)-(E1) reduced the invasion of MDA-MB-468 to less than 10% of the untreated control (not shown), whereas under the same conditions, MDA-MB-231 appeared to be resistant and the parental antibodies showed no effect (not shown). 1R-(15)-(15) at 100 µg/mL potently reduced the invasion of Capan-1, but had little effect on MDA-MB-468 (not shown). The ability of 1R-(E1)-(E1) to inhibit anchorage-independent growth was demonstrated at 200 nM in MDA-MB-231 with statistically significant difference (*P*< 0.041) when compared with samples treated with parental antibodies at the same concentrations (not shown). Cells treated with 1R-(E1)-(E1) produced few and much smaller colonies, the largest size of which was less than 1/10 of the untreated cells (not shown). The parental hR1 alone, but not hRS7, had some effect on inhibiting the growth of MDA-MB-231 in soft agar, presumably resulting from the downregulation of IGF-1R (not shown). These results evidence the potential of bispecific HexAbs for targeted therapy of solid cancers.

The skilled artisan will realize that the disclosed methods and compositions are not limited to the specific anti-IGF-1R antibodies, DNL constructs and/or mTOR inhibitors described above, but rather may comprise other known anti-IGF-1R antibodies or antigen-binding fragments thereof, other known mTOR inhibitors and alternative constructs.

All of the COMPOSITIONS and METHODS disclosed and claimed herein can be made and used without undue experimentation in light of the present disclosure. While the compositions and methods have been described in terms of preferred embodiments, it is apparent to those of skill in the art that variations maybe applied to the COMPOSITIONS and METHODS and in the steps or in the sequence of steps of the METHODS described herein without departing from the scope of the invention.

## Claims

1. A complex comprising
(i) an anti-IGF-1R IgG antibody attached to one or more anchor domain (AD) moieties from an AKAP protein, and
(ii) two or more copies of an effector attached to a dimerization and docking domain (DDD) moiety from a human protein kinase A (PKA) regulatory subunit RIα, RIβ, RIIα or RIIβ, wherein the effector is a second antibody fragment, a second antibody or a cytokine,
wherein two copies of the DDD moiety form a dimer that binds to the AD moiety to form the complex,
for use in a method of treating a cancer that expresses IGF-1R, wherein the method comprises:
a) administering the complex to an individual with the cancer; and
b) administering an mTOR inhibitor to the individual, wherein the mTOR inhibitor is selected from the group consisting of temsirolimus, rapamycin, ridaforolimus and everolimus;
wherein the combination of anti-IGF-1R antibody or antigen-binding fragment and mTOR inhibitor is more effective than either alone.

2. An mTOR inhibitor selected from the group consisting of temsirolimus, rapamycin, ridaforolimus and everolimus, for use in a method of treating a cancer that expresses IGF-1R, wherein the method comprises:
a) administering to an individual with the cancer a complex comprising
(i) an anti-IGF-1R IgG antibody attached to one or more anchor domain (AD) moieties from an AKAP protein, and
(ii) two or more copies of an effector attached to a dimerization and docking domain (DDD) moiety from a human protein kinase A (PKA) regulatory subunit RIα, RIβ, RIIα or RIIβ, wherein the effector is a second antibody fragment, a second antibody or a cytokine,
wherein two copies of the DDD moiety form a dimer that binds to the AD moiety to form the complex; and
b) administering the mTOR inhibitor to the individual;
wherein the combination of anti-IGF-1R antibody or antigen-binding fragment and mTOR inhibitor is more effective than either alone.

3. The complex for use according to claim 1 and the mTOR inhibitor for use according to claim 2, wherein the second antibody fragment is an anti-IGF-1R antibody fragment, an anti-TROP2 antibody fragment or an anti-CEACAM6 antibody fragment and wherein the second antibody is an anti-IGF-1R antibody, an anti-TROP2 antibody or an anti-CEACAM6 antibody.

4. The complex for use according to claim 1 and the mTOR inhibitor for use according to claim 2, wherein the second antibody fragment is an anti-IGF-1R antibody fragment and the second antibody is an anti-IGF-1R antibody.

5. The complex for use according to claim 1 and the mTOR inhibitor for use according to claim 2, wherein the second antibody fragment binds to a tumor-associated antigen selected from the group consisting of carbonic anhydrase IX, CCCL19, CCCL21 , CSAp, CD1, CDla, CD2, CD3, CD4, CD5, CD8, CD11A, CD14, CD15, CD16, CD18, CD19, CD20, IGF-1R, CD21, CD22, CD23, CD25, CD29, CD30, CD32b, CD33, CD37, CD38, CD40, CD40L, CD45, CD46, CD52, CD54, CD55, CD59, CD64, CD66a-e, CD67, CD70, CD74, CD79a, CD80, CD83, CD95, CD126, CD133, CD138, CD147, CD154, CEACAM5, CEACAM6, B7, ED-B fibronectin, Factor H, FHL-1 , Flt-3, folate receptor, GROB, HMGB-1, hypoxia inducible factor (HIF), HM1.24, insulin-like growth factor-1 (ILGF-1), IFN-γ, IFN-α, IFN-β, IL-2, IL-4R, IL-6R, IL-13R, IL-15R, IL-17R, IL-18R, IL-6, IL-8, IL-12, IL-15, IL-17, IL-18, IL-25, IP-10, MAGE, mCRP, MCP-1, MIP-1A, MIP-1B, MIF, MUC1, MUC2, MUC3, MUC4, MUC5ac, PAM4 antigen, NCA-95, NCA-90, PSMA, EGP-1, EGP-2, AFP, la, HM1.24, HLA-DR, tenascin, Le(y), RANTES, T101, TAC, Tn antigen, Thomson-Friedenreich antigens, tumor necrosis antigens, TNF-α, TRAIL receptor (R1 and R2), VEGFR, EGFR, P1GF, complement factors C3, C3a, C3b, C5a, C5.

6. The complex for use according to claim 1 and the mTOR inhibitor for use according to claim 2, wherein the cytokine is interferon-α2b.

7. The complex for use according to claim 1 and the mTOR inhibitor for use according to claim 2, wherein the cytokine is selected from the group consisting of human growth hormone, N-methionyl human growth hormone, parathyroid hormone, thyroxine, insulin, proinsulin, relaxin, prorelaxin, follicle stimulating hormone (FSH), thyroid stimulating hormone (TSH), luteinizing hormone (LH), hepatic growth factor, prostaglandin, fibroblast growth factor, prolactin, placental lactogen, tumor necrosis factor-α, tumor necrosis factor-β, mullerian-inhibiting substance, mouse gonadotropin-associated peptide, inhibin, activin, vascular endothelial growth factor, integrin, thrombopoietin (TPO), NGF-β, platelet-growth factor, TGF-α, TGF- β, insulin-like growth factor-I, insulin-like growth factor-II, erythropoietin (EPO), interferon-α, interferon-β, interferon-γ, macrophage-CSF (M-CSF), interleukin-1 (IL-1), IL-1α, IL-2, IL-3, IL-4, IL-5, IL-6, IL-7, IL-8, IL-9, IL-10, IL-11, IL-12, IL-13, IL-14, IL-15, IL-16, IL-17, IL-18, IL-21, IL-25, LIF, FLT-3, angiostatin, thrombospondin, endostatin, tumor necrosis factor and lymphotoxin.

8. The complex for use according to any of claims 1 and 3 to 7 and the mTOR inhibitor for use according to any of claims 2 to 7, wherein the anti-IGF- 1R antibody or fragment thereof binds to the first half of the cysteine-rich domain of IGF-1R, between amino acid residues 151 and 222 of human IGF-1R.

9. The complex for use according to any of claims 1 and 3 to 8 and the mTOR inhibitor for use according to any of claims 2 to 8, wherein the anti-IGF-1R antibody or fragment thereof comprises the heavy chain CDR sequences CDR1 (DYYMY, SEQ ID NO:85), CDR2 (YITNYGGSTYYPDTVKG, SEQ ID NO:86) and CDR3 (QSNYDYDGWFAY, SEQ ID NO:87) and the light chain CDR sequences CDR1 (KASQEVGTAVA, SEQ ID NO:88), CDR2 (WASTRHT, SEQ ID NO:89) and CDR3 (QQYSNYPLT, SEQ ID NO:90).

10. The complex for use according to any of claims 1 and 3 to 9 and the mTOR inhibitor for use according to any of claims 2 to 9, wherein the cancer is selected from the group consisting of Wilms' tumor, Ewing sarcoma, a neuroendocrine tumor, a glioblastoma, a neuroblastoma, a melanoma, skin cancer, breast cancer, colon cancer, rectal cancer, prostate cancer, liver cancer, renal cancer, pancreatic cancer, lung cancer, biliary cancer, cervical cancer, endometrial cancer, esophageal cancer, gastric cancer, head and neck cancer, medullary thyroid carcinoma, ovarian cancer, glioma, lymphoma, leukemia, myeloma, acute lymphoblastic leukemia, acute myelogenous leukemia, chronic lymphocytic leukemia, chronic myelogenous leukemia, Hodgkin's lymphoma, non-Hodgkin's lymphoma, and urinary bladder cancer.

11. The complex for use according to any of claims 1 and 3 to 10 and the mTOR inhibitor for use according to any of claims 2 to 10, wherein the mTOR inhibitor is temsirolimus.

12. The complex for use according to any of claims 1 and 3 to 11 and the mTOR inhibitor for use according to any of claims 2 to 11, wherein the anti-IGF-1R antibody is a naked antibody or is conjugated to at least one therapeutic agent.

13. The complex and the mTOR inhibitor for use according to claim 12, wherein said therapeutic agent is selected from the group consisting of a radionuclide, an immunomodulator, an anti-angiogenic agent, a cytokine, a chemokine, a growth factor, a hormone, a drug, a prodrug, an enzyme, an oligonucleotide, a pro-apoptotic agent, an interference RNA, a photoactive therapeutic agent, a cytotoxic agent, a chemotherapeutic agent and a toxin.

14. The complex and the mTOR inhibitor for use according to claim 13, wherein the drug is selected from the group consisting of 5-fluorouracil, aplidin, azaribine, anastrozole, anthracyclines, bendamustine, bleomycin, bortezomib, bryostatin-1, busulfan, calicheamycin, camptothecin, carboplatin, 10- hydroxycamptothecin, carmustine, celebrex, chlorambucil, cisplatin (CDDP), Cox-2 inhibitors, irinotecan (CPT-11), SN-38, carboplatin, cladribine, camptothecans, cyclophosphamide, cytarabine, dacarbazine, docetaxel, dactinomycin, daunorubicin, doxorubicin, 2-pyrrolinodoxorubicine (2P-DOX), cyano-morpholino doxorubicin, doxorubicin glucuronide, epirubicin glucuronide, estramustine, epipodophyllotoxin, estrogen receptor binding agents, etoposide (VP16), etoposide glucuronide, etoposide phosphate, floxuridine (FUdR), 3',5'-0-dioleoyl-FudR (FUdR-dO), fludarabine, flutamide, farnesyl-protein transferase inhibitors, gemcitabine, hydroxyurea, idarubicin, ifosfamide, L-asparaginase, lenolidamide, leucovorin, lomustine, mechlorethamine, melphalan, mercaptopurine, 6-mercaptopurine, methotrexate, mitoxantrone, mithramycin, mitomycin, mitotane, navelbine, nitrosourea, plicomycin, procarbazine, paclitaxel, pentostatin, PSI-341, raloxifene, semustine, streptozocin, tamoxifen, taxol, temazolomide (an aqueous form of DTIC), transplatinum, thalidomide, thioguanine, thiotepa, teniposide, topotecan, uracil mustard, vinorelbine, vinblastine, vincristine and vinca alkaloids.

15. The complex and the mTOR inhibitor for use according to claim 13, wherein the radioisotope is selected from the group consisting of ¹¹¹In, ¹⁷⁷Lu, ²¹²Bi, ²¹³Bi, ²¹¹At, ⁶²Cu, ⁶⁷Cu, ⁹⁰Y, ¹²⁵I, ¹³¹I, ³²P, ³³P, ⁴⁷Sc, ¹¹¹Ag, ⁶⁷Ga, ¹⁴²Pr, ¹⁵³Sm, ¹⁶¹Tb, ¹⁶⁶Dy, ¹⁶⁶Ho, ¹⁸⁶Re, ¹⁸⁸Re, ¹⁸⁹Re, ²¹²Pb, ²²³Ra, ²²⁵Ac, ⁵⁹Fe, ⁷⁵Se, ⁷⁷As, ⁸⁹Sr, ⁹⁹Mo, ¹⁰⁵Rh, ¹⁰⁹Pd, ¹⁴³Pr, ¹⁴⁹Pm, ¹⁶⁹Er, ¹⁹⁴Ir, ¹⁹⁸Au, ¹⁹⁹Au, and ²¹¹Pb.

## Patentansprüche

1. Komplex umfassend
(i) einen anti-IGF-1R-IgG-Antikörper, der an einen oder mehrere Ankerdomänen (AD) - Teil(e) von einem AKAP-Protein gebunden ist, und
(ii) zwei oder mehr Kopien eines Effektors, die an einen Dimerisierungs- und Andockdomänen (DDD) - Teil von einer regulatorischen Untereinheit RIα, RIβ, RIIα oder RIIβ von menschlicher Proteinkinase A (PKA) gebunden sind, wobei der Effektor ein zweites Antikörperfragment, ein zweiter Antikörper oder ein Cytokin ist,
wobei zwei Kopien des DDD-Teils ein Dimer bilden, das an den AD-Teil bindet, um den Komplex auszubilden,
zur Verwendung in einem Verfahren zum Behandeln von einem IGF-1R exprimierenden Krebs, wobei das Verfahren umfasst:
a) Verabreichen des Komplexes an ein Individuum, das an dem Krebs leidet; und
b) Verabreichen eines mTOR-Inhibitors an das Individuum, wobei der mTOR-Inhibitor ausgewählt ist aus der Gruppe bestehend aus Temsirolimus, Rapamycin, Ridaforolimus und Everolimus;
wobei die Kombination aus anti-IGF-1R-Antikörper oder Antigen-bindendem Fragment und mTOR-Inhibitor wirksamer ist als ein jeder alleine.

2. mTOR-Inhibitor ausgewählt aus der Gruppe bestehend aus Temsirolimus, Rapamycin, Ridaforolimus und Everolimus, zur Verwendung in einem Verfahren zum Behandeln von einem IGF-1R exprimierenden Krebs, wobei das Verfahren umfasst:
a) Verabreichen an ein Individuum, das an dem Krebs leidet, eines Komplexes umfassend
(i) einen anti-IGF-1R-IgG-Antikörper, der an einen oder mehrere Ankerdomänen (AD) - Teil(e) von einem AKAP-Protein gebunden ist, und
(ii) zwei oder mehr Kopien eines Effektors, die an einen Dimerisierungs- und Andockdomänen (DDD) - Teil von einer regulatorischen Untereinheit RIα, RIβ, RIIα oder RIIβ von menschlicher Proteinkinase A (PKA) gebunden sind, wobei der Effektor ein zweites Antikörperfragment, ein zweiter Antikörper oder ein Cytokin ist,
wobei zwei Kopien des DDD-Teils ein Dimer bilden, das an den AD-Teil bindet, um den Komplex auszubilden; und
b) Verabreichen des mTOR-Inhibitors an das Individuum;
wobei die Kombination aus anti-IGF-1R-Antikörper oder Antigen-bindendem Fragment und mTOR-Inhibitor wirksamer ist als ein jeder alleine.

3. Komplex zur Verwendung nach Anspruch 1 und mTOR-Inhibitor zur Verwendung nach Anspruch 2, wobei das zweite Antikörperfragment ein anti-IGF-1R-Antikörperfragment, ein anti-TROP2-Antikörperfragment oder ein anti-CEACAM6-Antikörperfragment ist und wobei der zweite Antikörper ein anti-IGF-1R-Antikörper, ein anti-TROP2-Antikörper oder ein anti-CEACAM6-Antikörper ist.

4. Komplex zur Verwendung nach Anspruch 1 und mTOR-Inhibitor zur Verwendung nach Anspruch 2, wobei das zweite Antikörperfragment ein anti-IGF-1R-Antikörperfragment und der zweite Antikörper ein anti-IGF-1R-Antikörper ist.

5. Komplex zur Verwendung nach Anspruch 1 und mTOR-Inhibitor zur Verwendung nach Anspruch 2, wobei das zweite Antikörperfragment an ein Tumor-assoziiertes Antigen bindet, das ausgewählt ist aus der Gruppe bestehend aus Kohlensäureanhydrase IX, CCCL19, CCCL21, CSAp, CD1, CD1a, CD2, CD3, CD4, CD5, CD8, CD11A, CD14, CD15, CD16, CD18, CD19, CD20, IGF-1R, CD21, CD22, CD23, CD25, CD29, CD30, CD32b, CD33, CD37, CD38, CD40, CD40L, CD45, CD46, CD52, CD54, CD55, CD59, CD64, CD66a-e, CD67, CD70, CD74, CD79a, CD80, CD83, CD95, CD126, CD133, CD138, CD147, CD154, CEACAM5, CEACAM6, B7, ED-B-Fibronectin, Factor H, FHL-1 , Flt-3, Folat-Rezeptor, GROB, HMGB-1, Hypoxie-induzierbarem Faktor (HIF), HM1.24, Insulin-ähnlichem Wachstumsfaktor-1 (ILGF-1), IFN-γ, IFN-α, IFN-β, IL-2, IL-4R, IL-6R, IL-13R, IL-15R, IL-17R, IL-18R, IL-6, IL-8, IL-12, IL-15, IL-17, IL-18, IL-25, IP-10, MAGE, mCRP, MCP-1, MIP-1A, MIP-1B, MIF, MUC1, MUC2, MUC3, MUC4, MUC5ac, PAM4 antigen, NCA-95, NCA-90, PSMA, EGP-1, EGP-2, AFP, la, HM1.24, HLA-DR, Tenascin, Le(y), RANTES, T101, TAC, Tn-Antigen, Thomson-Friedenreich-Antigenen, Tumornekroseantigenen, TNF-α, TRAIL-Rezeptor (R1 und R2), VEGFR, EGFR, P1GF, Komplementfaktoren C3, C3a, C3b, C5a und C5.

6. Komplex zur Verwendung nach Anspruch 1 und mTOR-Inhibitor zur Verwendung nach Anspruch 2, wobei das Cytokin interferon-α2b ist.

7. Komplex zur Verwendung nach Anspruch 1 und mTOR-Inhibitor zur Verwendung nach Anspruch 2, wobei das Cytokin ausgewählt ist aus der Gruppe bestehend aus menschlichem Wachstumshormon, N-Methionyl menschliche, Wachstumshormon, Parathormon, Thyroxin, Insulin, Proinsulin, Relaxin, Prorelaxin, follikelstimulierendem Hormon (FSH), schilddrüsenstimulierendem Hormon (TSH), luteinisierendem Hormon (LH), Leberwachstumsfaktor, Prostaglandin, Fibroblastenwachstumsfaktor, Prolactin, Plazentalaktogen, Tumornekrosefaktor-α, Tumornekrosefaktor-β, Müller-inhibierender Substanz, Gonadotropin-assoziiertem Peptid der Maus, Inhibin, Activin, Vaskularendothelwachstumsfaktor, Integrin, Thrombopoietin (TPO), NGF-β, Plättchenwachstumsfaktor, TGF-α, TGF-β, Insulin-ähnlichem Wachstumsfaktor-I, Insulin-ähnlichem Wachstumsfaktor-II, Erythropoietin (EPO), Interferon-α, Interferon-β, Interferon-γ, Makrophagen-CSF (M-CSF), Interleukin-1 (IL-1), IL-1 α, IL-2, IL-3, IL-4, IL-5, IL-6, IL-7, IL-8. IL-9, IL-10, IL-11, IL-12, IL-13, IL-14, IL-15, IL-16, IL-17, IL-18, IL-21, IL-25, LIF, FLT-3, Angiostatin, Thrombospondin, Endostatin, Tumornekrosefaktor und Lymphotyoxin.

8. Komplex zur Verwendung nach einem der Ansprüche 1 und 3 bis 7 und mTOR-Inhibitor zur Verwendung nach einem der Ansprüche 2 bis 7, wobei der anti-IGF-1R-Antikörper oder Fragment davon an die erste Hälfte der Cystein-reichen Domäne von IGF-1R zwischen den Aminosäureresten 151 und 222 von menschlichem IGF-1R bindet.

9. Komplex zur Verwendung nach einem der Ansprüche 1 und 3 bis 8 und mTOR-Inhibitor zur Verwendung nach einem der Ansprüche 2 bis 8, wobei der anti-IGF-1R-Antikörper oder Fragment davon die CDR-Sequenzen der schweren Kette CDR1 (DYYMY, SEQ ID NO:85), CDR2 (YITNYGGSTYYPDTVKG, SEQ ID NO:86) und CDR3 (QSNYDYDGWFAY, SEQ ID NO:87) und die CDR-Sequenzen der leichten Kette CDR1 (KASQEVGTAVA, SEQ ID NO:88), CDR2 (WASTRHT, SEQ ID NO:89) und CDR3 (QQYSNYPLT, SEQ ID NO:90) umfasst.

10. Komplex zur Verwendung nach einem der Ansprüche 1 und 3 bis 9 und mTOR-Inhibitor zur Verwendung nach einem der Ansprüche 2 bis 9, wobei der Krebs ausgewählt ist aus der Gruppe bestehend aus Wilms-Tumor, Ewing-Sarkoma, einem neuroendokrinen Tumor, einem Glioblastom, einem Neuroblastom, einem Melanom, Hautkrebs, Brustkrebs, Dickdarmkarzinom, Rektumkarzinom, Prostatakrebs, Leberkrebs, Nierenkrebs, Pankreaskarzinom, Lungenkrebs, Gallenkrebs, Zervixkarzinom, Endometriumkarzinom, Ösophaguskarzinom, Magenkarzinom, Karzinom des Kopfes und des Halses, medulärem Schilddrüsenkarzinom, Ovarialkarzinom, Gliom, Lymphom, Leukämie, Myelom, akuter lymphatischer Leukämie, akuter myeloischer Leukämie, chronischer lymphatischer Leukämie, chronischer myeloischer Leukämie, Hodgkin Lymphom, non-Hodgkin Lymphom und Harnblasenkrebs.

11. Komplex zur Verwendung nach einem der Ansprüche 1 und 3 bis 10 und mTOR-Inhibitor zur Verwendung nach einem der Ansprüche 2 bis 10, wobei der mTOR-Inhibitor Temsirolimus ist.

12. Komplex zur Verwendung nach einem der Ansprüche 1 und 3 bis 11 und mTOR-Inhibitor zur Verwendung nach einem der Ansprüche 2 bis 11, wobei der anti-IGF-1R-Antikörper ein nackter Antikörper oder mit wenigstens einem therapeutischen Agens konjugiert ist.

13. Komplex und mTOR-Inhibitor zur Verwendung nach Anspruch 12, wobei das therapeutische Agens ausgewählt ist aus der Gruppe bestehend aus einem Radionuklid, einem Immunmodulator, einem anti-angiogenem Agens, einem Cytokin, einem Chemokin, einem Wachstumsfaktor, einem Hormon, einem Wirkstoff, einem Prodrug, einem Enzym, einem Oligonucletoide, einem pro-apoptotischen Agens, einer interferierenden RNA, einem photoaktiven therapeutischen Agens, einem cytotoxischen Agens, einem chemotherapeutischen Agens und einem Toxin.

14. Komplex und mTOR-Inhibitor zur Verwendung nach Anspruch 13, wobei der Wirkstoff ausgewählt ist aus der Gruppe bestehend aus 5-Fluoruracil, Aplidin, Azaribin, Anastrozol, Anthracyclinen, Bendamustine, Bleomycin, Bortezomib, Bryostatin-1, Busulfan, Calicheamycin, Camptothecin, Carboplatin, 10-Hydroxycamptothecin, Carmustin, Celebrex, Chlorambucil, cis-Platin (CDDP), Cox-2-Inhibitoren, Irinotecan (CPT-11), SN-38, Carboplatin, Cladribin, Camptothecanrn, Cyclophosphamid, Cytarabin, Dacarbazin, Docetaxel, Dactinomycin, Daunorubicin, Doxorubicin, 2-Pyrrolinodoxorubicin (2P-DOX), Cyano-Morpholino-Doxorubicin, Doxorubicin-Glucuronid, Epirubicin-Glucuronid, Estramustin, Epipodophyllotoxin, Estrogenrezeptor-bindenden Agenzien, Etoposid (VP16), Etoposid-Glucuronid, Etoposidphosphat, Floxuridin (FUdR), 3',5'-0-Dioleoyl-FudR (FUdR-dO), Fludarabin, Flutamid, Farnesyl-protein-Transferase-Inhibitoren, Gemcitabin, Hydroxyharnstoff, Idarubicin, Ifosfamide, L-Asparaginase, Lenolidamid, Leucovorin, Lomustin, Mechlorethamin, Melphalan, Mercaptopurin, 6-Mercaptopurin, Methotrexat, Mitoxantron, Mithramycin, Mitomycin, Mitotan, Navelbin, Nitrosoharnstoff, Plicomycin, Procarbazin, Paclitaxel, Pentostatin, PSI-341, Raloxifen, Semustin, Streptozocin, Tamoxifen, Taxol, Temazolomid (einer wässrigen Form von DTIC), Transplatin, Thalidomid, Thioguanin, Thiotepa, Teniposid, Topotecan, Uracil-Senfgas, Vinorelbin, Vinblastin, Vincristin und Vinca-Alkaloiden.

15. Komplex und mTOR-Inhibitor zur Verwendung nach Anspruch 13, wobei das Radioisotop ausgewählt ist aus der Gruppe bestehend aus ¹¹¹In, ¹⁷⁷Lu, ²¹²Bi, ²¹³Bi, ²¹¹At, ⁶²Cu, ⁶⁷Cu, ⁹⁰Y, ¹²⁵I, ¹³¹I, ³²P, ³³P, ⁴⁷Sc, ¹¹¹Ag, ⁶⁷Ga, ¹⁴²Pr, ¹⁵³Sm, ¹⁶¹Tb, ¹⁶⁶Dy, ¹⁶⁶Ho, ¹⁸⁶Re, ¹⁸⁸Re, ¹⁸⁹Re, ²¹²Pb, ²²³Ra, ²²⁵Ac, ⁵⁹Fe, ⁷⁵Se, ⁷⁷As, ⁸⁹Sr, ⁹⁹Mo, ¹⁰⁵Rh, ¹⁰⁹Pd, ¹⁴³Pr, ¹⁴⁹Pm, ¹⁶⁹Er, ¹⁹⁴Ir, ¹⁹⁸Au, ¹⁹⁹Au und ²¹¹Pb.

## Revendications

1. Complexe comprenant
(i) un anticorps IgG anti-IGF-1R lié à un ou plusieurs fragments du domaine d'ancrage (AD) d'une protéine AKAP, et
(ii) deux copies ou plus d'un effecteur lié à un fragment du domaine de dimérisation et d'amarrage (DDD) d'une sous-unité régulatrice RIα, PIβ, RIIα ou RIIβ de protéine kinase A (PKA) humaine, où l'effecteur est un second fragment d'anticorps, un second anticorps ou une cytokine,
dans lequel deux copies du fragment DDD forment un dimère qui se lie au fragment AD pour former le complexe,
pour une utilisation dans une méthode de traitement d'un cancer qui exprime l'IGF-1R, où la méthode comprend :
a) l'administration du complexe à un individu atteint de cancer ; et
b) l'administration d'un inhibiteur de mTOR à l'individu, où l'inhibiteur de mTOR est choisi dans le groupe constitué par le temsirolimus, la rapamycine, le ridaforolimus et l'évérolimus ;
où la combinaison de l'anticorps anti-IGF-1R ou d'un fragment se liant à l'antigène et de l'inhibiteur mTOR est plus efficace que l'un ou l'autre utilisé seul.

2. Inhibiteur de mTOR choisi dans le groupe constitué par le temsirolimus, la rapamycine, le ridaforolimus et l'évérolimus, pour une utilisation dans une méthode de traitement d'un cancer qui exprime l'IGF-1R, où la méthode comprend :
a) l'administration à un individu atteint de cancer d'un complexe comprenant
(i) un anticorps IgG anti-IGF-1R lié à un ou plusieurs fragments du domaine d'ancrage (AD) d'une protéine AKAP, et
(ii) deux copies ou plus d'un effecteur lié à un fragment du domaine de dimérisation et d'amarrage (DDD) d'une sous-unité régulatrice RIα, PIβ, RIIα ou RIIβ de protéine kinase A (PKA) humaine, où l'effecteur est un second fragment d'anticorps, un second anticorps ou une cytokine,
dans lequel deux copies du fragment DDD forment un dimère qui se lie au fragment AD pour former le complexe ; et
b) l'administration de l'inhibiteur de mTOR à l'individu ;
où la combinaison de l'anticorps anti-IGF-1R ou d'un fragment se liant à l'antigène et de l'inhibiteur de mTOR et plus efficace que l'un ou l'autre utilisé seul.

3. Complexe pour une utilisation selon la revendication 1 et inhibiteur de mTOR pour une utilisation selon la revendication 2, dans lequel le second fragment d'anticorps est un fragment d'anticorps anti-IGF-1R, un fragment d'anticorps anti-TROP2 ou un fragment d'anticorps anti-CEACAM6 et le second anticorps est un anticorps anti-IGF-1R, un anticorps anti-TROP2 ou un anticorps anti-CEACAM6.

4. Complexe pour une utilisation selon la revendication 1 et inhibiteur de mTOR pour une utilisation selon la revendication 2, dans lequel le second fragment d'anticorps est un fragment d'anticorps anti-IGF-1R et le second anticorps est un anticorps anti-IGF-1R.

5. Complexe pour une utilisation selon la revendication 1 et inhibiteur de mTOR pour une utilisation selon la revendication 2, dans lequel le second fragment d'anticorps se lie à un antigène associé à la tumeur choisi dans le groupe constitué par : anhydrase carbonique IX, CCCL19, CCCL21, CSAp, CD1, CD1a, CD2, CD3, CD4, CD5, CD8, CD11A, CD14, CD15, CD16, CD18, CD19, CD20, IGF-1R, CD21, CD22, CD23, CD25, CD29, CD30, CD32b, CD33, CD37, CD38, CD40, CD40L, CD45, CD46, CD52, CD54, CD55, CD59, CD64, CD66a-e, CD67, CD70, CD74, CD79a, CD80, CD83, CD95, CD126, CD133, CD138, CD147, CD154, CEACAM5, CEACAM6, B7, domaine ED-B de fibronectine, facteur H, FHL-1, Flt-3, récepteur de folate, GROB, HMGB-1, facteur inductible par hypoxie (HIF), HM1.24, facteur de croissance analogue de l'insuline-1 (ILGF-1), IFN-γ, IFN-α, IFN-β, IL-2, IL-4R, IL-6R, IL-13R, IL-15R, IL-17R, IL-18R, IL-6, IL-8, IL-12, IL-15, IL-17, IL-18, IL-25, IP-10, MAGE, mCRP, MCP-1, MIP-1A, MIP-1B, MIF, MUC1, MUC2, MUC3, MUC4, MUC5ac, antigène PAM4, NCA-95, NCA-90, PSMA, EGP-1, EGP-2, AFP, la, HM1.24, HLA-DR, ténascine, Le(y), RANTES, T101, TAC, antigène Tn, antigènes de Thomson-Friedenreich, antigènes de nécrose tumorale, TNF-α, récepteur TRAIL (R1 et R2), VEGFR, EGFR, P1GF, facteurs C3, C3a, C3b, C5a et C5 du complément.

6. Complexe pour une utilisation selon la revendication 1 et inhibiteur de mTOR pour une utilisation selon la revendication 2, dans lequel la cytokine est l'interféron-α2b.

7. Complexe pour une utilisation selon la revendication 1 et inhibiteur de mTOR pour une utilisation selon la revendication 2, dans lequel la cytokine est choisie dans le groupe constitué par : hormone de croissance humaine, hormone de croissance humaine N-méthionylée, parathormone, thyroxine, insuline, proinsuline, relaxine, prorelaxine, hormone folliculostimulante (FSH), hormone thyréostimulante (TSH), hormone lutéinisante (LH), facteur de croissance hépatique, prostaglandine, facteur de croissance fibroblastique, prolactine, lactogène placentaire, facteur de nécrose tumorale α, facteur de nécrose tumorale β, substance anti-müllerienne, peptide murin associé à la gonadotropine, inhibine, activine, facteur de croissance endothétiale vasculaire, intégrine, thrombopoïétine (TPO), NGF-β, facteur de croissance plaquettaire, TGF-α, TGF-β, facteur de croissance analogue à l'insuline-I, facteur de croissance analogue à l'insuline-II, érythropoïétine (EPO), interféron-a, interféron-β, interféron-y, macrophage-CSF (M-CSF), interleukine-1 (IL-1), IL-la, IL-2, IL-3, IL-4, IL-5, IL-6, IL-7, IL-8, IL-9, IL-10, IL-11, IL-12, IL-13, IL-14, IL-15, IL-16, IL-17, IL-18, IL-21, IL-25, LIF, FLT-3, angiostatine, thrombospondine, endostatine, facteur de nécrose tumorale et lymphotoxine.

8. Complexe pour une utilisation selon l'une quelconque des revendications 1 et 3 à 7 et inhibiteur de mTOR pour une utilisation selon l'une quelconque des revendications 2 à 7, dans lequel l'anticorps anti-IGF-1R ou fragment de celui-ci se lie à la première moitié du domaine riche en cystéine de l'IGF-1R, entre les résidus acides aminés 151 et 222 de l'IGF-1R humain.

9. Complexe pour une utilisation selon l'une quelconque des revendications 1 et 3 à 8 et inhibiteur de mTOR pour une utilisation selon l'une quelconque des revendications 2 à 8, dans lequel l'anticorps anti-IGF-1R ou fragment de celui-ci comprend les séquences CDR de chaîne lourde CDR1 (DYYMY, SEQ ID No. : 85), CDR2 (YITNYGGSTYYPDTVKG, SEQ ID No. : 86) et CDR3 (QSNYDYDGWFAY, SEQ ID No. : 87) et les séquences CDR de chaîne légère CDR1 (KASQEVGTAVA, SEQ ID No. : 88), CDR2 (WASTRHT, SEQ ID No. : 89) et CDR3 (QQYSNYPLT, SEQ ID No. : 90).

10. Complexe pour une utilisation selon l'une quelconque des revendications 1 et 3 à 9 et inhibiteur de mTOR pour une utilisation selon l'une quelconque des revendications 2 à 9, dans lequel le cancer est choisi dans le groupe constitué par : tumeur de Wilm, sarcome d'Ewing, tumeur neuroendocrine, glioblastome, neuroblastome, mélanome, cancer de la peau, cancer du sein, cancer du côlon, cancer rectal, cancer de la prostate, cancer du foie, cancer rénal, cancer pancréatique, cancer du poumon, cancer biliaire, cancer du col de l'utérus, cancer de l'endomètre, cancer de l'oesophage, cancer gastrique, cancer de la tête et du cou, cancer médullaire de la thyroïde, cancer de l'ovaire, gliome, lymphome, leucémie, myélome, leucémie aiguë lymphoblastique, leucémie aiguë myélogène, leucémie chronique lymphocytaire, leucémie chronique myélogène, lymphome hodgkinien, lymphome non hodgkinien et cancer de la vessie.

11. Complexe pour une utilisation selon l'une quelconque des revendications 1 et 3 à 10 et inhibiteur de mTOR pour une utilisation selon l'une quelconque des revendications 2 à 10, dans lequel l'inhibiteur de mTOR est le temsirolimus.

12. Complexe pour une utilisation selon l'une quelconque des revendications 1 et 3 à 11 et inhibiteur de mTOR pour une utilisation selon l'une quelconque des revendications 2 à 11, dans lequel l'anticorps anti-IGF-1R est un anticorps nu ou conjugué à au moins un agent thérapeutique.

13. Complexe et inhibiteur de mTOR pour une utilisation selon la revendication 12, dans lequel ledit agent thérapeutique est choisi dans le groupe constitué par : radionucléide, immunomodulateur, agent anti-angiogénique, cytokine, chimiokine, facteur de croissance, hormone, médicament, promédicament, enzyme, oligonucléotide, agent pro-apoptique, ARN d'interférence, agent thérapeutique photoactif, agent cytotoxique, agent chimiothérapique et toxine.

14. Complexe et inhibiteur de mTOR pour une utilisation selon la revendication 13, dans lequel le médicament est choisi dans le groupe constitué par : 5-fluorouracil, aplidine, azaribine, anastrozole, anthracyclines, bendamustine, bléomycine, bortézomib, bryostatine-1, busulfan, calichéamycine, camptothécine, carboplatine, 10-hydroxycamptothécine, carmustine, célébrex, chlorambucil, cisplatine (CDDP), inhibiteurs de Cox-2, irinotécan (CPT-11), SN-38, carboplatine, cladribine, camptothécans, cyclophosphamide, cytarabine, dacarbazine, docétaxel, dactinomycine, daunorubicine, doxorubicine, 2-pyrrolinodoxorubicine (2P-DOX), cyano-morpholino-doxorubicine, doxorubicine glucuronide, épirubicine glucuronide, estramustine, épipodophyllotoxine, agents de liaison des récepteurs d'oestrogènes, étoposide (VP16), étoposide glucuronide, phosphate d'étoposide, floxuridine (FUdR), 3',5'-O-dioléoyl-FudR (FUdR-dO), fludarabine, flutamide, inhibiteurs de farnésyl-protéine transférase, gemcitabine, hydroxyurée, idarubicine, ifosfamide, L-asparaginase, lénolidamide, leucovorine, lomustine, méchloréthamine, melphalan, mercaptopurine, 6-mercaptopurine, méthotrexate, mitoxantrone, mithramycine, mitomycine, mitotane, navelbine, nitrosurée, plicomycine, procarbazine, paclitaxel, pentostatine, PSI-341, raloxifène, sémustine, streptozocine, tamoxifène, taxol, témazolomide (forme aqueuse de DTIC), transplatine, thalidomide, thioguanine, thiotépa, téniposide, topotécan, moutarde uracil, vinorelbine, vinblastine, vincristine et alcaloïdes vinca.

15. Complexe et inhibiteur de mTOR pour une utilisation selon la revendication 13, dans lequel le radioisotope est choisi dans le groupe constitué par : ¹¹¹In, ¹⁷⁷Lu, ²¹²Bi, ²¹³Bi, ²¹¹At, ⁶²Cu, ⁶⁷Cu, ⁹⁰Y, ¹²⁵I, ¹³¹I, ³²P, ³³P, ⁴⁷Sc, ¹¹¹Ag, ⁶⁷Ga, ¹⁴²Pr, ¹⁵³Sm, ¹⁶¹Tb, ¹⁶⁶Dy, ¹⁶⁶Ho, ¹⁸⁶Re, ¹⁸⁸Re, ¹⁸⁹Re, ²¹²Pb, ²²³Ra, ²²⁵Ac, ⁵⁹Fe, ⁷⁵Se, ⁷⁷As, ⁸⁹Sr, ⁹⁹Mo, ¹⁰⁵Rh, ¹⁰⁹Pd, ¹⁴³Pr, ¹⁴⁹Pm, ¹⁶⁹Er, ¹⁹⁴Ir, ¹⁹⁸Au et ²¹¹Pb.
